# EUROPEAN PATENT SPECIFICATION

(11) **EP 2 956 484 B1**
(45) Date of publication and mention of the grant of the patent: **14.11.2018**
(21) Application number: 14732006.3
(22) Date of filing: 13.02.2014
(51) Int. Cl.: C07K 16/28, A01K 67/027, A61K 39/395, A61K 31/4745, A61K 31/513, A61K 31/522, A61K 39/00

(54) **CETUXIMAB WITH MODIFIED GLYCOSYLATION AND USES THEREOF**
CETUXIMAB MIT MODIFIZIERTER GLYKOSILIERUNG UND VERWENDUNGEN DAVON
CÉTUXIMAB AVEC GLYCOSYLATION MODIFIÉE ET UTILISATIONS ASSOCIÉES

(30) Priority: 13.02.2013 US 201361764446 P
(43) Date of publication of application: 23.12.2015
(73) Proprietor: Laboratoire Français du Fractionnement et des Biotechnologies, 91940 Les Ulis (FR)
(72) Inventor: MEADE, Harry, M., Newton, MA 02458 (US)
(74) Representative: Cabinet Becker et Associés
(86) International application number: PCT/IB2014/000867
(87) International publication number: WO 2014/125382

(56) References cited:
- WO-A2-2008/101177
- CHRISTINE H. CHUNG ET AL: "Cetuximab-Induced Anaphylaxis and IgE Specific for Galactose-[alpha]-1,3-Galactose", NEW ENGLAND JOURNAL OF MEDICINE, vol. 358, no. 11, 13 March 2008 (2008-03-13), pages 1109-1117, XP055077473, ISSN: 0028-4793, DOI: 10.1056/NEJMoa074943 cited in the application
- QIAN ET AL: "Structural characterization of N-linked oligosaccharides on monoclonal antibody cetuximab by the combination of orthogonal matrix-assisted laser desorption/ionization hybrid quadrupole-quadrupole time-of-flight tandem mass spectrometry and sequential enzymatic digestion", ANALYTICAL BIOCHEMISTRY, ACADEMIC PRESS INC, NEW YORK, vol. 364, no. 1, 31 March 2007 (2007-03-31), pages 8-18, XP022005960, ISSN: 0003-2697, DOI: 10.1016/J.AB.2007.01.023 cited in the application
- MAGDELAINE-BEUZELIN ET AL: "Structure-function relationships of the variable domains of monoclonal antibodies approved for cancer treatment", CRITICAL REVIEWS IN ONCOLOGY / HEMATOLOGY, ELSEVIER SCIENCE IRELAND LTD., LIMERICK, IE, vol. 64, no. 3, 14 November 2007 (2007-11-14), pages 210-225, XP022344458, ISSN: 1040-8428, DOI: 10.1016/J.CRITREVONC.2007.04.011 cited in the application
- JACQUENET S ET AL: "Mammalian meat-induced anaphylaxis: Clinical relevance of anti-galactose-alpha-1,3-galactose IgE confirmed by means of skin tests to cetuximab", JOURNAL OF ALLERGY AND CLINICAL IMMUNOLOGY, ELSEVIER, AMSTERDAM, NL, vol. 124, no. 3, 1 September 2009 (2009-09-01), pages 603-605, XP026557575, ISSN: 0091-6749, DOI: 10.1016/J.JACI.2009.06.014 [retrieved on 2009-09-03]
- GRONLUND H ET AL: "The carbohydrate galactose-alpha-1,3-galactose is a major IgE-binding epitope on cat IgA", JOURNAL OF ALLERGY AND CLINICAL IMMUNOLOGY, ELSEVIER, AMSTERDAM, NL, vol. 123, no. 5, 1 May 2009 (2009-05-01), pages 1189-1191, XP026083389, ISSN: 0091-6749, DOI: 10.1016/J.JACI.2009.03.011 [retrieved on 2009-04-10]
- COMMINS S P ET AL: "Delayed anaphylaxis, angioedema, or urticaria after consumption of red meat in patients with IgE antibodies specific for galactose-alpha-1,3-galactose", JOURNAL OF ALLERGY AND CLINICAL IMMUNOLOGY, ELSEVIER, AMSTERDAM, NL, vol. 123, no. 2, 1 February 2009 (2009-02-01), pages 426-433.e2, XP025907162, ISSN: 0091-6749, DOI: 10.1016/J.JACI.2008.10.052 [retrieved on 2008-12-13]
- MORISSET M ET AL: "Anaphylaxis to pork kidney is related to IgE antibodies specific for galactose-alpha-1,3-galactose.", ALLERGY MAY 2012, vol. 67, no. 5, May 2012 (2012-05), pages 699-704, XP002727255, ISSN: 1398-9995

## Description

### FIELD OF THE INVENTION

The present invention relates in part to antibodies with altered glycosylation patterns, methods of their production, and methods of use.

### BACKGROUND OF THE INVENTION

Erbitux® (cetuximab) is a recombinant chimeric monoclonal antibody that binds to the extracellular domain of the human epidermal growth factor receptor (EGFR) and is used in the clinic to treat certain forms of cancer. Unfortunately, some patients have experienced an allergic response, in some cases anaphylactic shock and death, when treated with Erbitux®. This allergic response has been shown to be caused by IgE that recognizes the galactose-alpha-1,3-galactose glycosylation molecule present on the commercial form of cetuximab, Erbitux® (C.Chung, et al. Cetuximab-induced anaphylaxis and IgE specific for galactose-alpha-1,3-galactose. N Engl J Med (2008);358:1109-17). Addition of galactose-alpha-1,3-galactose is thought to be a result of the mammalian (murine myeloma) cell culture system used to commercially produce Erbitux®. Production of proteins in Chinese hamster ovary cells can also result in galactose-alpha-1,3-galactose moieties on the proteins (Bosques et al., Nat. Biotechn. 28: 1153-1156, 2010).

### SUMMARY OF INVENTION

It has been found that antibodies produced in mammalian mammary epithelial cells, such as in the mammalian mammary epithelial cells of a transgenic animal engineered to secrete antibodies in its milk, have no detectable galactose-alpha-1,3-galatose moieties. Accordingly, in aspect, the disclosure provides antibodies and compositions thereof that have no detectable galactose-alpha-1,3-galatose moieties. Also provided are methods of producing such antibodies as well as methods of their use.

In one aspect, the invention provides a glycosylated antibody comprising
(a) a heavy chain comprising SEQ ID NO:1; and
(b) a light chain comprising SEQ ID NO:2;
wherein the glycosylated antibody does not have detectable galactose-alpha-1,3-galactose moieties and wherein the glycosylated antibody is produced in mammary gland epithelial cells of a non-human transgenic mammal.

In some of the embodiments of the glycosylated antibodies provided herein, the glycosylated antibody lacks galactose-alpha-1,3-galactose moieties. In some embodiments, the non-human mammal is a goat, sheep, bison, camel, cow, pig, rabbit, buffalo, horse, rat, mouse or llama. In some embodiments, the non-human mammal is a goat. In some of the embodiments of the glycosylated antibodies provided herein, the antibody is chimeric, humanized or a fully human antibody. In some of the embodiments of the glycosylated antibodies provided herein, the antibody is cetuximab.

In one aspect, the invention provides a composition comprising any of the glycosylated antibodies provided herein, and a pharmaceutically-acceptable carrier.

In one aspect, the invention provides a method comprising producing a population of glycosylated antibodies, in mammary gland epithelial cells such that the population of glycosylated antibodies produced does not have detectable galactose-alpha-1,3-galactose moieties, wherein the glycosylated antibodies comprise:
(a) a heavy chain comprising SEQ ID NO:1; and
(b) a light chain comprising SEQ ID NO:2.

In some of the embodiments of the compositions provided herein, the glycosylated antibodies lack galactose-alpha-1,3-galactose moietiesIn some of the embodiments of the compositions provided herein, the population of glycosylated antibodies is produced in the mammary gland epithelial cells of a non-human transgenic mammal. In some embodiments, the non-human mammal is a goat, sheep, bison, camel, cow, pig, rabbit, buffalo, horse, rat, mouse or llama. In some embodiments, the non-human mammal is a goat.

In some of the embodiments of the compositions provided herein, the glycosylated antibodies in the population are chimeric, humanized or fully human antibodies. In some of the embodiments of the compositions provided herein, the glycosylated antibodies in the population are cetuximab antibodies. In some of the embodiments of the compositions provided herein, the composition further comprises milk. In some of the embodiments of the compositions provided herein, the composition further comprises a pharmaceutically acceptable carrier.

In one aspect, the disclosure provides a composition comprising monoclonal antibodies, wherein the monoclonal antibodies comprise:
(a) a heavy chain comprising SEQ ID NO:1;
(b) a light chain comprising SEQ ID NO:2; and
wherein the composition is produced in a mammary gland of a transgenic goat.

In one aspect, the disclosure provides a composition comprising monoclonal antibodies, wherein the monoclonal antibodies comprise:
(a) a heavy chain comprising SEQ ID NO:1;
(b) a light chain comprising SEQ ID NO:2; and
wherein the monoclonal antibodies lack galactose-alpha-1,3-galactose.

In some of the embodiments of the methods provided herein, the population of glycosylated antibodies lacks galactose-alpha-1,3-galactose. In some of the embodiments of the methods provided herein, the method further comprises collecting the population of glycosylated antibodies. In some of the embodiments of the methods provided herein, the producing occurs *in vitro.* In some of the embodiments of the methods provided herein, the producing occurs *in vivo.* In some of the embodiments of the methods provided herein, the producing occurs in a non-human transgenic mammal. In some embodiments, the non-human mammal is a goat, sheep, bison, camel, cow, pig, rabbit, buffalo, horse, rat, mouse or llama. In some embodiments, the non-human mammal is a goat.

In some of the embodiments of the methods provided herein, the method further comprising purifying the population of glycosylated antibodies. In some of the embodiments of the methods provided herein, the method further comprises comparing the number of galactose-alpha-1,3-galactose moieties present in the population of glycosylated antibodies to the number of galactose-alpha-1,3-galactose moieties present in a population of glycosylated antibodies produced in non-mammary cell culture.

In one aspect, the disclosure provides a population of glycosylated antibodies produced by any of the methods provided herein.

In one aspect, the disclosure provides mammary gland epithelial cells that produce any of the antibodies provided herein. In some embodiments of the mammary gland epithelial cells provided herein, the mammary gland epithelial cells comprise a nucleic acid comprising SEQ ID NO:3 and a nucleic acid comprising SEQ ID NO:4. In some embodiments of the mammary gland epithelial cells provided herein, the nucleic acid comprising SEQ ID NO:3 and the nucleic acid comprising SEQ ID NO:4 are connected.

In one aspect, the disclosure provides a transgenic non-human mammal comprising any of the mammary gland epithelial cells disclosed herein. In some embodiments, the non-human mammal is a goat.

In one aspect, the disclosure provides mammary gland epithelial cells that produce any of the population of antibodies disclosed herein. In some embodiments of the mammary gland epithelial cells provided herein, the mammary gland epithelial cells comprise a nucleic acid comprising SEQ ID NO: 3 and a nucleic acid comprising SEQ ID NO: 4. In some embodiments of the mammary gland epithelial cells provided herein, the nucleic acid comprising SEQ ID NO: 3 and the nucleic acid comprising SEQ ID NO: 4 are connected.

In one aspect, the disclosure provides a transgenic non-human mammal comprising any of the mammary gland epithelial cells disclosed herein.

In one aspect, the disclosure provides a method comprising administering an effective amount of any of the antibodies provided herein to a subject in need thereof.

In one aspect, the disclosure provides a method comprising administering an effective amount of any of the compositions provided herein to a subject in need thereof.

In some embodiments, the subject has cancer. In some embodiments, the cancer is head and neck cancer or colorectal cancer. In some embodiments of the methods provided herein, the method further comprises administering at least one additional therapeutic agent. In some embodiments, the at least one additional therapeutic agent is irinotecan, leucovorin calcium, fluorouracil, or a combination thereof.

### BRIEF DESCRIPTION OF THE DRAWINGS

The drawings are first described. It is to be understood that the drawings are exemplary.
**Fig. 1** shows the amino acid sequence of the heavy chain (HC) of cetuximab (SEQ ID NO:1).

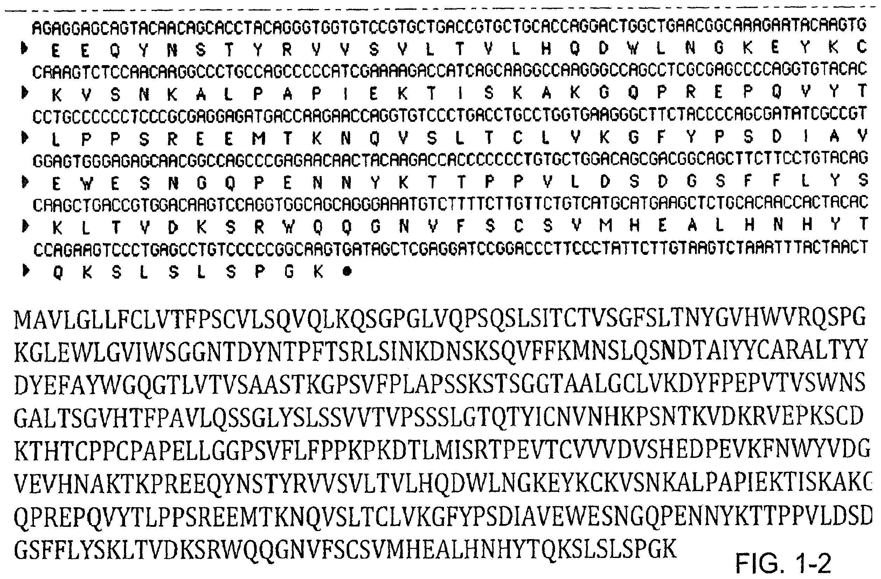

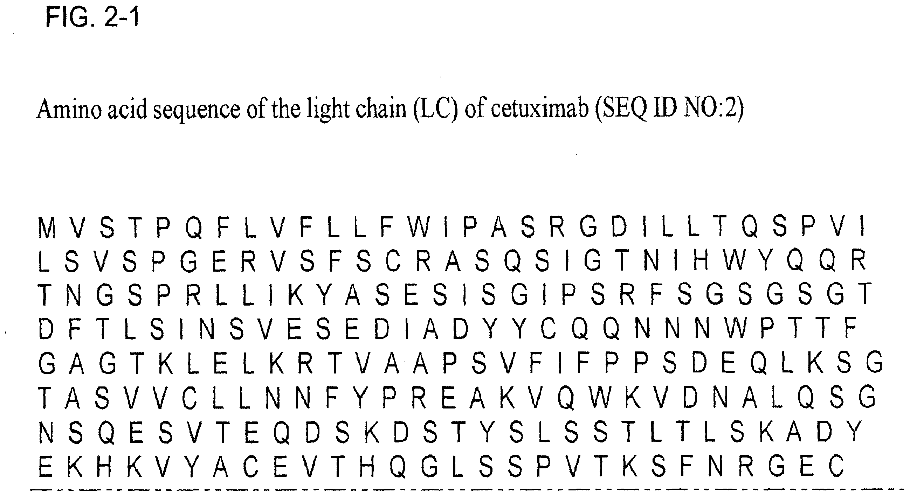

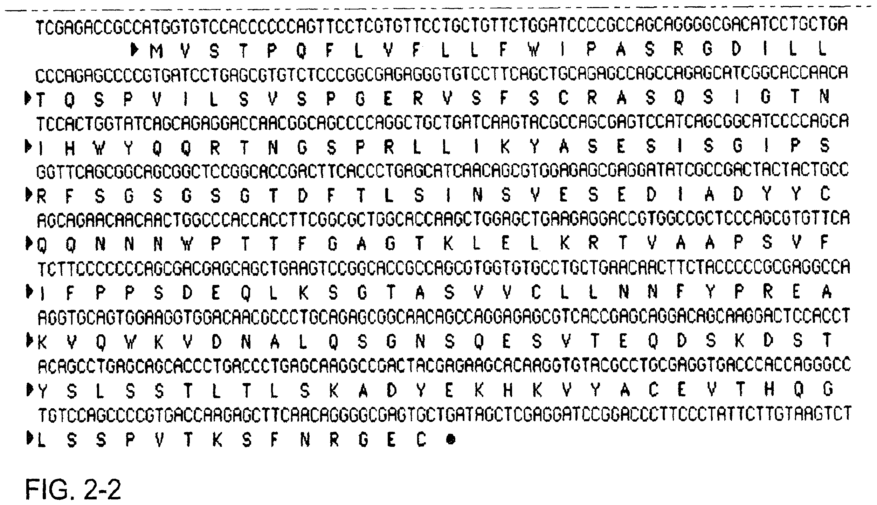

**Fig. 2** shows the amino acid sequence of the light chain (LC) of cetuximab (SEQ ID NO:2).

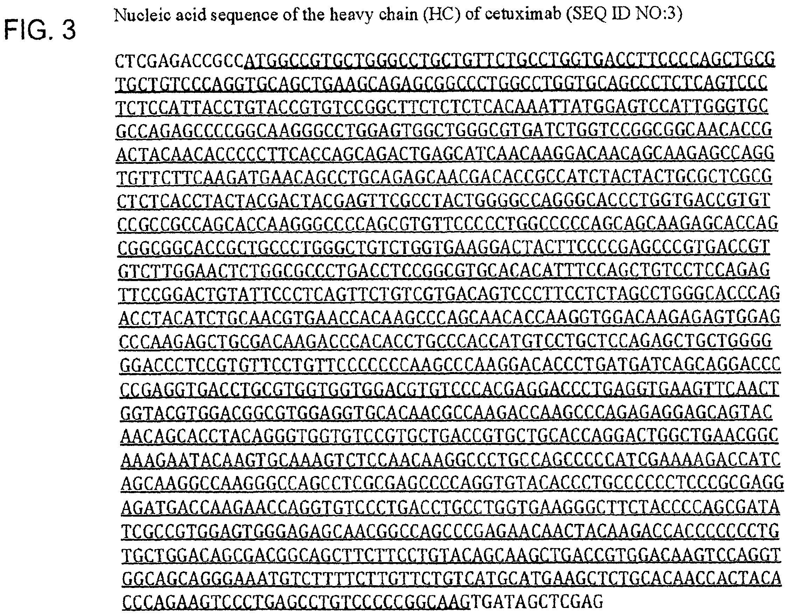

**Fig. 3** shows the nucleic acid sequence of the heavy chain (HC) of cetuximab (SEQ ID NO:3).

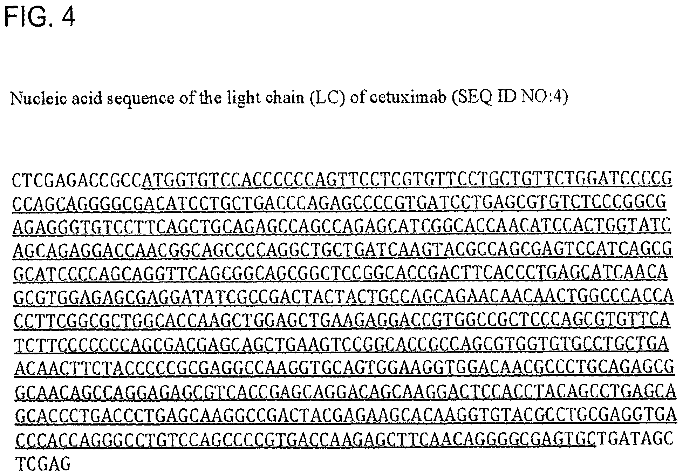

**Fig. 4** shows the nucleic acid sequence of the light chain (LC) of cetuximab (SEQ ID NO:4).
**Fig. 5** shows the plasmid BC 2553 LC cetuximab (light chain) (**A**) and the nucleic acid sequence of the plasmid (SEQ ID NO:5) (**B**).
**Fig. 6** shows the plasmid BC 2554 HC cetuximab (heavy chain) (**A**) and the nucleic acid sequence of the plasmid (SEQ ID NO:6) (**B**).
**Fig. 7** shows the plasmid BC 2584 puromycin beta casein vector carrying heavy chain (**A**) and the nucleic acid sequence of the plasmid (SEQ ID NO:7) (**B**).
**Fig. 8** shows a Western Blot with the levels of expression of cetuximab in the milk of two mouse lines (#15 and #27).
**Fig. 9** shows a Western Blot used to detect levels of alpha-1,3-gal in milk-produced cetuximab and commercially produced cetuximab (Erbitux®).
**Fig. 10** shows a Western Blot with of cetuximab produced in the milk of goats #1 and #2.
**Fig. 11** shows the purification of cetuximab produced in the milk of goats.
**Fig. 12** is a western blot depicting anti-alpha 1,3 galactose monoclonal antibody staining on cetuximab produced in goats (lane 2, goat #1, and lane 3, goat #2), and the commercially product Erbitux®.
**Fig. 13** is a mass spectrum (LC/MS) of purified Erbitux®, purified cetuximab produced in the milk of goat #1, and purified cetuximab produced in the milk of goat #2.

### DETAILED DESCRIPTION OF INVENTION

Erbitux® (cetuximab) is a human/mouse chimeric recombinant antibody that binds to the extracellular domain of the human epidermal growth factor receptor (EGFR) and is used in the clinic to treat cancer. A study of the structure of Erbitux® showed that one N-linked glycosylation site present in the framework 3 region of the heavy chain is occupied by galactose-alpha-1,3-galactose (also referred to herein as "gal-alpha-1,3-gal" or "alpha-1,3-gal"), see Jun Qian, et al.: Structural characterization of N-linked oligosaccharides on monoclonal antibody cetuximab by the combination of orthogonal matrix-assisted laser desorption/ionization hybrid quadrupole-quadrupole time-of-flight tandem mass spectrometry and sequential enzymatic digestion. (Analytical Biochemistry 364 (2007) 8-18). The presence of this gal-alpha-1,3-gal is thought to be due to the fact that commercial Erbitux® is produced in mouse myeloma cells, which are known to add gal-alpha-1,3-gal on N-linked glycosylation sites.

This gal-alpha-1,3-gal glycosylation has been shown to give rise to an allergic response in some patients treated with Erbitux® (Chung, et al. Cetuximab-induced anaphylaxis and IgE specific for galactose-alpha-1,3-galactose. N Engl J Med (2008);358:1109-17). These patients were shown to have pre-existing IgE antibodies to gal-alpha-1,3-gal, which gave rise to a hypersensitive reaction upon treatment with the antibody.

Additionally, galactose-alpha-1,3-galactose has been shown to cause allergic responses in other contexts. For instance, gal-alpha-1,3-gal is present on red meat and people who experience an allergic reaction (e.g., anaphylaxis) after eating red meat were found to have IgE antibodies specific to gal-alpha-1,3-gal (Commins et al., Delayed anaphylaxis, angioedema, or urticaria after consumption of red meat in patients with IgE antibodies specific for galactose-alpha-1,3-galactose. J Allergy Clin Immunol. 2009 Feb;123(2):426-33; and Commins and Platts-Mills, Anaphylaxis syndromes related to a new mammalian cross-reactive carbohydrate determinant. J Allergy Clin Immunol. 2009 October; 124(4): 652-657.). In another instance, it was shown that gal-alpha-1,3-gal present on cat IgA, another allergen, can be bound by IgE in humans (Gronlund et al. The carbohydrate galactose-alpha-1,3-galactose is a major IgE-binding epitope on cat IgA. J Allergy Clin Immunol. 2009 May;123(5):1189-91). Hence, the presence of galactose-alpha-1,3-galactose poses potential problems in the development of antibodies and other glycoproteins for therapeutic use in humans.

Production of recombinant antibodies is often performed in cell culture using well-established cell lines like NSo and Sp2/0. These cell lines have been shown to add galactose to recombinant antibodies in an alpha(1-3) linkage to form gal-alpha-1,3-gal (Chung, et al. Cetuximab-induced anaphylaxis and IgE specific for galactose-alpha-1,3-galactose. N Engl J Med (2008);358:1109-17). In humans and higher primates the gene that produces gal-alpha-1,3-gal through addition of galactose in an alpha(1-3) linkage (alpha-1,3-galactosyltransferase) is non-functional, and humans and higher primates therefore generally do not produce gal-alpha-1,3-gal. As a result, gal-alpha-1,3-gal negative animals, such as humans, have the potential to recognize gal-alpha-1,3-gal as "non-self" and can generate IgE and IgG antibodies specific for this oligosaccharide, potentially giving rise to the allergic reactions described above. Thus, a need exists for methods to produce recombinant therapeutic antibodies that lack gal-alpha-1,3-gal moieties.

The ability to modify animal genomes through transgenic technology offers alternatives for the manufacture of recombinant antibodies with modified glycosylation patterns. The production of human recombinant pharmaceuticals in the milk of transgenic farm animals solves many of the problems associated with microbial bioreactors (e.g., lack of proper post-translational modifications, improper protein folding, high purification costs) or animal cell bioreactors (e.g., high capital costs, expensive culture media, low yields).

Surprisingly, as disclosed herein, cetuximab produced in the milk of mice and goats through expression of the heavy and light chains of cetuximab in mammary epithelial cells was found to have no detectable gal-alpha-1,3-gal (as determined by LC/MS and Western blot analysis). This is surprising, at least, because commercially produced cetuximab (Erbitux®), which is produced in mouse myeloma cells contains gal-alpha-1,3-gal moieties. In addition, goat serum proteins also show gal-alpha-1,3-gal moieties.

Accordingly, aspects of the disclosure relate to glycosylated antibodies or populations of antibodies that have altered glycosylation patterns, specifically fewer gal-alpha-1,3-gal moieties compared to glycosylated antibodies produced in cell culture. In some embodiments, the antibody is cetuximab. Other aspects of the disclosure provide methods for producing glycosylated antibody or populations of antibodies that comprise fewer gal-alpha-1,3-gal moieties than glycosylated antibodies produced in cell culture. Yet another aspect of the disclosure relates to mammalian mammary epithelial cells or transgenic animals capable of producing populations of antibodies that have altered glycosylation patterns, specifically fewer gal-alpha-1,3-gal moieties compared to glycosylated antibodies produced in cell culture. According to the invention, the glycosylated antibodies provided herein do not have any detectable level of gal-alpha-1,3-gal moieties.

### Antibodies and Antigen-binding fragments thereof

As used herein, the term "antibody" can refer to a glycoprotein comprising at least two heavy (H) chains and two light (L) chains. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. In some embodiments, the antigen is the extracellular domain of the human epidermal growth factor receptor (EGFR). The constant regions of the antibodies may mediate the binding of the immunoglobulin to host tissues or factors, including various cells of the immune system (*e.g*., effector cells) and the first component (C1q) of the classical complement system. Formation of a mature functional antibody molecule can be accomplished when two proteins are expressed in stoichiometric quantities and self-assemble with the proper configuration.

The term "antibodies" is also meant to encompass antigen-binding fragments thereof. Methods for making antibodies and antigen-binding fragments are well known in the art (see, e.g. Sambrook et al, "Molecular Cloning: A Laboratory Manual" (2nd Ed.), Cold Spring Harbor Laboratory Press (1989); Lewin, "Genes IV", Oxford University Press, New York, (1990), and Roitt et al., "Immunology" (2nd Ed.), Gower Medical Publishing, London, New York (1989), WO2006/040153, WO2006/122786, and WO2003/002609). As used herein, an "antigen-binding fragment" of an antibody refers to one or more portions of an antibody that retain the ability to specifically bind to an antigen, *e.g.* the extracellular domain of EGFR. It has been shown that the antigen-binding function of an antibody can be performed by fragments of a full-length antibody. Examples of binding fragments encompassed within the term "antigen-binding fragment" of an antibody include (i) a Fab fragment, a monovalent fragment consisting of the VL, VH, CL and CH1 domains; (ii) a F(ab')2 fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546) which consists of a VH domain; and (vi) an isolated complementarity determining region (CDR). Furthermore, although the two domains of the Fv fragment, VL and VH, are encoded by separate genes, they can be joined, using recombinant methods, by a synthetic linker that enables them to be made as a single protein chain in which the VL and VH regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. These antibody fragments are obtained using conventional procedures, such as proteolytic fragmentation procedures, as described in J. Goding, Monoclonal Antibodies: Principles and Practice, pp 98-118 (N.Y. Academic Press 1983), as well as by other techniques known to those with skill in the art. The fragments are screened for utility in the same manner as are intact antibodies.

In some embodiments, the antibodies are of the isotype IgG, IgA or IgD. In further embodiments, the antibodies are selected from the group consisting of IgG1, IgG2, IgG3, IgG4, IgM, IgA1, IgA2, IgAsec, IgD, IgE or has immunoglobulin constant and/or variable domain of IgG1, IgG2, IgG3, IgG4, IgM, IgA1, IgA2, IgAsec, IgD or IgE. In other embodiments, the antibodies are bispecific or multispecific antibodies. According to an alternative embodiment, the antibodies of the present disclosure can be modified to be in the form of a bispecific antibody, or a multispecific antibody. The term "bispecific antibody" is intended to include any agent, *e.g.,* a protein, peptide, or protein or peptide complex, which has two different binding specificities which bind to, or interact with (a) a cell surface antigen and (b) an Fc receptor on the surface of an effector cell. The term "multispecific antibody" is intended to include any agent, *e.g.,* a protein, peptide, or protein or peptide complex, which has more than two different binding specificities which bind to, or interact with (a) a cell surface antigen, (b) an Fc receptor on the surface of an effector cell, and (c) at least one other component. Accordingly, the disclosure includes, but is not limited to, bispecific, trispecific, tetraspecific, and other multispecific antibodies which are directed to cell surface antigens, and to Fc receptors on effector cells. The term "bispecific antibodies" further includes diabodies. Diabodies are bivalent, bispecific antibodies in which the VH and VL domains are expressed on a single polypeptide chain, but using a linker that is too short to allow for pairing between the two domains on the same chain, thereby forcing the domains to pair with complementary domains of another chain and creating two antigen-binding sites (see e.g., Holliger, P., et al. (1993) Proc. Natl. Acad. Sci. USA 90:6444-6448; Poijak, R.J., et al. (1994) Structure 2:1121-1123).

The term "antibodies" also encompasses different types of antibodies, e.g., recombinant antibodies, monoclonal antibodies, humanized antibodies or chimeric antibodies, or a mixture of these.

In some embodiments, the antibodies are recombinant antibodies. The term "recombinant antibody", as used herein, is intended to include antibodies that are prepared, expressed, created or isolated by recombinant means, such as antibodies isolated from an animal that is transgenic for another species' immunoglobulin genes, antibodies expressed using a recombinant expression vector transfected into a host cell, antibodies isolated from a recombinant, combinatorial antibody library, or antibodies prepared, expressed, created or isolated by any other means that involves splicing of immunoglobulin gene sequences to other DNA sequences.

In yet other embodiments, the antibodies can be chimeric or humanized antibodies. As used herein, the term "chimeric antibody" refers to an antibody that combines parts of a non-human (e.g., mouse, rat, rabbit) antibody with parts of a human antibody. As used herein, the term "humanized antibody" refers to an antibody that retains only the antigen-binding CDRs from the parent antibody in association with human framework regions (see, Waldmann, 1991, Science 252:1657). Such chimeric or humanized antibodies retaining binding specificity of the murine antibody are expected to have reduced immunogenicity when administered in vivo for diagnostic, prophylactic or therapeutic applications according to the disclosure. In some embodiments, the antibody is cetuximab.

In certain embodiments, the antibodies are human antibodies. The term "human antibody", as used herein, is intended to include antibodies having variable and constant regions derived from human germline immunoglobulin sequences. The human antibodies of the disclosure may include amino acid residues not encoded by human germline immunoglobulin sequences (e.g., mutations introduced by random or site-specific mutagenesis in vitro or by somatic mutation *in vivo*). However, the term "human antibody", as used herein, is not intended to include antibodies in which CDR sequences derived from the germline of another mammalian species, such as a mouse, have been grafted onto human framework sequences (referred to herein as "humanized antibodies"). Human antibodies are generated using transgenic mice carrying parts of the human immune system rather than the mouse system. Fully human monoclonal antibodies also can be prepared by immunizing mice transgenic for large portions of human immunoglobulin heavy and light chain loci. See, *e.g.,* U.S. patents 5,591,669, 5,598,369, 5,545,806, 5,545,807, 6,150,584, and references cited therein. These animals have been genetically modified such that there is a functional deletion in the production of endogenous (e.g., murine) antibodies. The animals are further modified to contain all or a portion of the human germ-line immunoglobulin gene locus such that immunization of these animals results in the production of fully human antibodies to the antigen of interest. Following immunization of these mice (e.g., XenoMouse (Abgenix), HuMAb mice (Medarex/GenPharm)), monoclonal antibodies are prepared according to standard hybridoma technology. These monoclonal antibodies have human immunoglobulin amino acid sequences and therefore will not provoke human anti-mouse antibody (HAMA) responses when administered to humans. The human antibodies, like any of the antibodies provided herein can be monoclonal antibodies.

In some embodiments, the antibody is a full-length antibody. In some embodiments the full-length antibody comprises a heavy chain and a light chain. In some embodiments, the heavy chain comprises SEQ ID NO:1 and the light chain comprises SEQ ID NO:2. In some embodiments, the heavy chain consists of SEQ ID NO:1 and the light chain consists of SEQ ID NO:2. In some embodiments, the antibody is cetuximab.

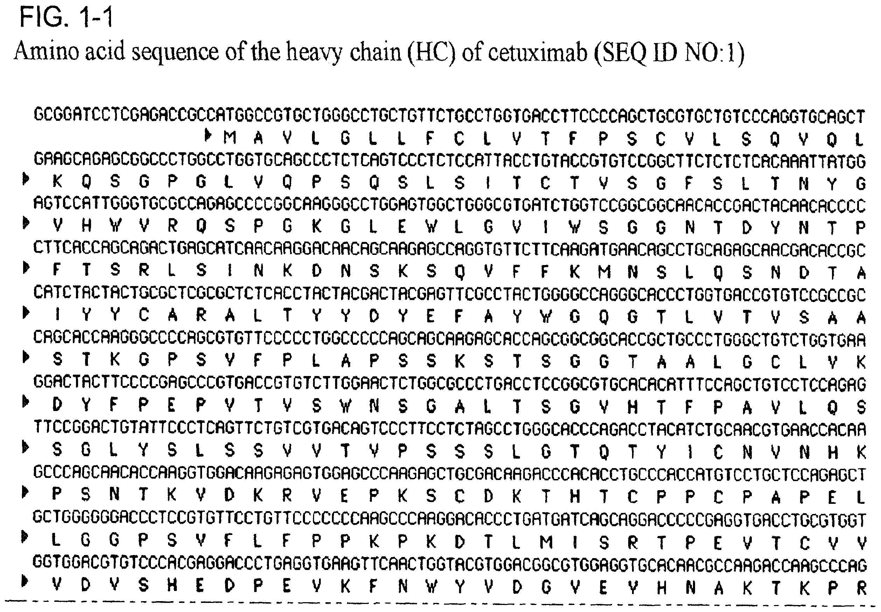

In some embodiments, the antibody is a glycosylated antibody. Glycosylated antibodies are described in greater detail herein. In some embodiments, the glycosylated antibody comprises a heavy chain comprising SEQ ID NO:1 and a light chain comprising SEQ ID NO:2. In some embodiments, the glycosylated antibody comprises a heavy chain that consists of SEQ ID NO:1 and a light chain that consists of SEQ ID NO:2. In some embodiments, the glycosylated antibody has fewer galactose-alpha-1,3-galactose moieties than the glycosylated antibody produced in non-mammary cell culture. In some embodiments, the fewer galactose-alpha-1,3-galactose moieties are located in the variable region of the heavy chain of the glycosylated antibody. In one embodiment, the fewer galactose-alpha-1,3-galactose moieties are located at position 107 in the heavy chains as shown in SEQ ID NO:1 (Highlighted in Figure 1).

### Glycosylation

Glycosylation is important for the correct folding, targeting, bioactivity and clearance of therapeutic glycoproteins. Glycosylation is a post-translational modification that can produce a variety of final protein forms in the natural state. Glycosylation patterns differ between different animals, e.g. between mice and humans. Certain alterations in carbohydrate structure also can affect antibody function, tolerability, or therapeutic effectiveness. For example, as described above, certain sugar moieties, such as gal-alpha-1,3-gal, can cause allergic reactions in humans.

Most animals, aside from humans and higher primates incorporate alpha-1,3-gal on N-linked sugars. For instance, CHO (Chinese Hamster Ovary) cell line used for the production of therapeutic proteins puts low level of alpha-1,3-gal on some sites. Furthermore, the mouse myeloma cell line used for the production of the therapeutic antibody cetuximab also incorporates alpha-1,3-gal moieties.

Surprisingly, as disclosed herein, recombinant cetuximab produced in the mammary glands of both transgenic mice and goats did not have detectable gal-alpha-1,3-gal. As also noted above, the result is surprising, at least because cetuximab that is produced in mouse myeloma cells (Erbitux®) is known to contain gal-alpha-1,3-gal sugars (Jun Qian, et al. Structural characterization of N-linked oligosaccharides on monoclonal antibody cetuximab by the combination of orthogonal matrix-assisted laser desorption/ionization hybrid quadrupole-quadrupole time-of-flight tandem mass spectrometry and sequential enzymatic digestion. Analytical Biochemistry 364 (2007) 8-18).

Thus, in some aspects, the disclosure provides a glycosylated antibody or populations of glycosylated antibodies with altered glycosylation patterns. In some embodiments, the disclosure provides a glycosylated antibody or populations of glycosylated antibodies that comprise fewer gal-alpha-1,3-gal moieties than a glycosylated antibody or populations of glycosylated antibodies produced in cell culture. In some embodiments, the cell culture resulting in the production of higher levels gal-alpha-1,3-gal moieties is a non-mammary cell culture. As described herein, "non-mammary cell culture" means a cell culture system in which the cells used are not of mammary tissue origin. In some embodiments, the non-mammary cell culture is mammalian cell culture. In some embodiments, the non-mammary cell culture is mouse cell culture. In some embodiments, the non-mammary cell culture is mouse myeloma cell culture.

In some embodiments, the disclosure provides a glycosylated antibody or populations of glycosylated antibodies that lack gal-alpha-1,3-gal moieties. As used herein, "a glycosylated antibody or populations of glycosylated antibodies that lack gal-alpha-1,3-gal moieties" describes either an antibody or populations of antibodies that contain no gal-alpha-1,3-gal moieties within the entire antibody, or antibodies that do not have detectable gal-alpha-1,3-gal at a specified location within the antibody. Methods for detecting gal-alpha-1,3-gal are well-known in the art and are also described herein. In some embodiments, the glycosylated antibody or populations of glycosylated antibodies of the disclosure comprise a heavy chain comprising SEQ ID NO:1 and a light chain comprising SEQ ID NO:2. In some embodiments, the glycosylated antibody or populations of glycosylated antibodies disclosed herein are cetuximab antibodies.

In other aspects, the disclosure provides methods for producing glycosylated antibodies with altered glycosylation patterns. In some embodiments, the disclosure provides methods for producing glycosylated antibodies that comprise fewer gal-alpha-1,3-gal moieties than glycosylated antibodies produced in cell culture. In some embodiments, the fewer galactose-alpha-1,3-galactose moieties are located in the variable region of the heavy chain of the glycosylated antibody. In one embodiment, the fewer galactose-alpha-1,3-galactose moieties are located at amino acid position 107 in SEQ ID NO:1. In some embodiments, the cell culture is non-mammary cell culture. In some embodiments, the non-mammary cell culture is mammalian cell culture. In some embodiments, the non-mammary cell culture is mouse cell culture. In some embodiments, the non-mammary cell culture is mouse myeloma cell culture. In some embodiments, the disclosure provides methods for producing glycosylated antibodies that lack gal-alpha-1,3-gal moieties. In some embodiments, the methods above comprise production of glycosylated antibodies comprising a heavy chain comprising SEQ ID NO:1 and a light chain comprising SEQ ID NO:2. In some embodiments, the methods above comprise production of cetuximab.

In some embodiments, a glycosylated antibody or populations of glycosylated antibodies comprise fewer gal-alpha-1,3-gal moieties than a glycosylated antibody or population or glycosylated antibodies produced in cell culture, e.g. non-mammary cell culture. In some embodiments, a glycosylated antibody or populations of glycosylated antibodies comprise at least 10%, at least 20%, at least 30%, at least 40%, at least 50%, at least 60%, at least 70%, at least 80%, at least 90%, or 100% fewer gal-alpha-1,3-gal moieties than a glycosylated antibody or population of glycosylated antibodies produced in cell culture, *e.g.* non-mammary cell culture. The glycosylation pattern, *e.g.,* the number of gal-alpha-1,3-gal moieties, can be determined by many methods known in the art, e.g., liquid chromatography-mass spectrometry (LC/MS) or tandem mass spectrometry. For example, methods of analyzing carbohydrates on proteins have been described in U.S. Patent Applications US 2006/0057638 and US 2006/0127950. Thus, the quantity of gal-alpha-1,3-gal moieties in an antibody or population of antibodies can be measured and compared to another antibody or population of antibodies, determining whether there are fewer gal-alpha-1,3-gal moieties in one population versus the other.

In another aspect, the disclosure provides compositions of glycosylated antibodies or populations of glycosylated antibodies. In one aspect, the disclosure provides a composition comprising a population of glycosylated antibodies, wherein the glycosylated antibodies comprise:
(a) a heavy chain comprising SEQ ID NO:1;
(b) a light chain comprising SEQ ID NO:2; and
wherein the population of antibodies has fewer galactose-alpha-1,3-galactose moieties than the population of glycosylated antibodies produced in non-mammary cell culture.

In another aspect, the disclosure provides a composition comprising monoclonal antibodies, wherein the monoclonal antibodies comprise:
(a) a heavy chain comprising SEQ ID NO:1;
(b) a light chain comprising SEQ ID NO:2; and
wherein the composition is produced in a mammary gland of a transgenic goat.

In yet another aspect, the disclosure provides a composition comprising monoclonal antibodies, wherein the monoclonal antibodies comprise:
(a) a heavy chain comprising SEQ ID NO:1;
(b) a light chain comprising SEQ ID NO:2; and
wherein the monoclonal antibodies lack galactose-alpha-1,3-galactose.

Compositions of antibodies or populations of antibodies as described above may have any further limitations as described herein. For example, in some embodiments, compositions are produced in the mammary gland of a transgenic non-human animal, e.g., a goat. In some embodiments, the composition further comprises milk. In other embodiments, the composition further comprises a pharmaceutically acceptable carrier. In some embodiments, the antibodies or populations of antibodies comprise fewer gal-alpha-1,3-gal moieties than glycosylated antibodies produced in cell culture, e.g. non-mammary cell culture. In other embodiments, the antibodies or populations of antibodies lack gal-alpha-1,3-gal moieties.

### Mammalian Mammary Gland Epithelial Cells and Transgenic Animals

In one aspect, the disclosure relates to mammalian mammary epithelial cells that produce glycosylated antibodies. Methods are provided herein for producing glycosylated antibodies in mammalian mammary epithelial cells.

In some embodiments, the production is accomplished by production in cell culture (*i.e., in vitro* or *ex vivo*). Examples of cells of mammary tissue origin include, but are not limited to, mammary gland epithelial cells, mammary epithelial cells, mammary tumor cells (e.g., mammary carcinoma cells). Cells of mammary tissue origin may be primary cells, immortalized cells, or transformed cells.

In some embodiments, the production is accomplished by production in a transgenic animal (*i.e., in vivo*). In some embodiments, the mammalian mammary gland epithelial cells are in a transgenic animal. In some embodiments, the mammalian mammary gland epithelial cells have been engineered to express recombinant antibodies in the milk of a transgenic animal, such as a mouse or goat. To accomplish this, the expression of the gene(s) encoding the recombinant protein can be, for example, under the control of the goat β-casein regulatory elements. Expression of recombinant proteins, *e.g.* antibodies, in both mice and goat milk has been established previously (see, *e.g.,* US Patent Application No. 2008-0118501). In some embodiments, the expression is optimized for individual mammary duct epithelial cells that produce milk proteins.

Transgenic animals capable of producing recombinant antibodies can be generated according to methods known in the art (see, *e.g.,* U.S. Patent No. 5,945,577 and US Patent Application No 2008-0118501). Animals suitable for transgenic expression, include, but are not limited to goat, sheep, bison, camel, cow, pig, rabbit, buffalo, horse, rat, mouse or llama. Suitable animals also include bovine, caprine, ovine and porcine, which relate to various species of cows, goats, sheep and pigs (or swine), respectively. Suitable animals also include ungulates. As used herein, "ungulate" is of or relating to a hoofed typically herbivorous quadruped mammal, including, without limitation, sheep, swine, goats, cattle and horses. Suitable animals also include dairy animals, such as goats and cattle, or mice. In some embodiments, the animal suitable for transgenic expression is a goat.

In some embodiments, transgenic animals are generated by generation of primary cells comprising a construct of interest followed by nuclear transfer of primary cell nuclei into enucleated oocytes. Primary cells comprising a construct of interest are produced by injecting or transfecting primary cells with a single construct comprising the coding sequence of an antibody of interest, *e.g.,* the heavy and light chains of cetuximab, or by co-transfecting or co-injecting primary cells with separate constructs comprising the coding sequences of the heavy and light chains of an antibody, *e.g.,* cetuximab. These cells are then expanded and characterized to assess transgene copy number, transgene structural integrity and chromosomal integration site. Cells with desired transgene copy number, transgene structural integrity and chromosomal integration site are then used for nuclear transfer to produce transgenic animals. As used herein, "nuclear transfer" refers to a method of cloning wherein the nucleus from a donor cell is transplanted into an enucleated oocyte.

Coding sequences for antibodies of interest to be expressed in mammalian mammary epithelial cells can be obtained by screening libraries of genomic material or reverse-translated messenger RNA derived from the animal of choice (such as humans, cattle or mice), from sequence databases such as NCBI, Genbank, or by obtaining the sequences of antibodies using methods known in the art, *e.g.,* peptide mapping. The sequences can be cloned into an appropriate plasmid vector and amplified in a suitable host organism, like *E*. *coli.* As used herein, a "vector" may be any of a number of nucleic acids into which a desired sequence may be inserted by restriction and ligation for transport between different genetic environments or for expression in a host cell. Vectors are typically composed of DNA although RNA vectors are also available. Vectors include, but are not limited to, plasmids and phagemids. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art (*e.g*., *β*-galactosidase or alkaline phosphatase), and genes which visibly affect the phenotype of transformed or transfected cells, hosts, colonies or plaques.

The coding sequence of antibodies or the heavy and light chains of antibodies of interest can be operatively linked to a control sequence which enables the coding sequence to be expressed in the milk of a transgenic non-human mammal. After amplification of the vector, the DNA construct can be digested with restriction enzymes, purified from the remains of the vector and introduced into fertilized embryos to produce transgenic animals. The transgenic animals will have the desired transgenic protein integrated into their genome.

A DNA sequence which is suitable for directing production to the milk of transgenic animals can carry a 5'-promoter region derived from a naturally-derived milk protein. This promoter is consequently under the control of hormonal and tissue-specific factors and is most active in lactating mammary tissue. In some embodiments, the promoter used is a milk-specific promoter. As used herein, a "milk-specific promoter" is a promoter that naturally directs expression of a gene in a cell that secretes a protein into milk (*e.g.*, a mammary epithelial cell) and includes, for example, the casein promoters, *e.g.,* α-casein promoter (*e.g.*, alpha S-1 casein promoter and alpha S2-casein promoter), β-casein promoter (*e.g*., the goat beta casein gene promoter (DiTullio, BIOTECHNOLOGY 10:74-77, 1992), γ-casein promoter, κ-casein promoter, whey acidic protein (WAP) promoter (Gorton et al., BIOTECHNOLOGY 5: 1183-1187, 1987), β-lactoglobulin promoter (Clark et al., BIOTECHNOLOGY 7: 487-492, 1989) and α-lactalbumin promoter (Soulier et al., FEBS LETTS. 297:13, 1992). Also included in this definition are promoters that are specifically activated in mammary tissue, such as, for example, the long terminal repeat (LTR) promoter of the mouse mammary tumor virus (MMTV). In some embodiments the promoter is a caprine beta casein promoter.

The promoter can be operably linked to a DNA sequence directing the production of a protein leader sequence which directs the secretion of the transgenic protein across the mammary epithelium into the milk. As used herein, a coding sequence and regulatory sequences (e.g., a promoter) are said to be "operably joined" or "operably linked" when they are linked in such a way as to place the expression or transcription of the coding sequence under the influence or control of the regulatory sequences. As used herein, a "leader sequence" or "signal sequence" is a nucleic acid sequence that encodes a protein secretory signal, and, when operably linked to a downstream nucleic acid molecule encoding a transgenic protein directs secretion. The leader sequence may be the native human leader sequence, an artificially-derived leader, or may be obtained from the same gene as the promoter used to direct transcription of the transgene coding sequence, or from another protein that is normally secreted from a cell, such as a mammalian mammary epithelial cell. In some embodiments a 3'-sequence, which can be derived from a naturally secreted milk protein, can be added to improve stability of mRNA.

In some embodiments, to produce primary cell lines containing a construct (*e.g*., encoding an cetuximab antibody) for use in producing transgenic goats by nuclear transfer, the heavy and light chain constructs can be transfected into primary goat skin epithelial cells, which are expanded and fully characterized to assess transgene copy number, transgene structural integrity and chromosomal integration site. As used herein, "nuclear transfer" refers to a method of cloning wherein the nucleus from a donor cell is transplanted into an enucleated oocyte.

Cloning will result in a multiplicity of transgenic animals - each capable of producing an antibody or other gene construct of interest. The production methods include the use of the cloned animals and the offspring of those animals. Cloning also encompasses the nuclear transfer of fetuses, nuclear transfer, tissue and organ transplantation and the creation of chimeric offspring. One step of the cloning process comprises transferring the genome of a cell, *e.g.* a primary cell that contains the transgene of interest into an enucleated oocyte. As used herein, "transgene" refers to any piece of a nucleic acid molecule that is inserted by artifice into a cell, or an ancestor thereof, and becomes part of the genome of an animal which develops from that cell. Such a transgene may include a gene which is partly or entirely exogenous (*i.e.,* foreign) to the transgenic animal, or may represent a gene having identity to an endogenous gene of the animal. Suitable mammalian sources for oocytes include goats, sheep, cows, pigs, rabbits, guinea pigs, mice, hamsters, rats, non-human primates, etc. Preferably, oocytes are obtained from ungulates, and most preferably goats or cattle. Methods for isolation of oocytes are well known in the art. Essentially, the process comprises isolating oocytes from the ovaries or reproductive tract of a mammal, *e.g.,* a goat. A readily available source of ungulate oocytes is from hormonally-induced female animals. For the successful use of techniques such as genetic engineering, nuclear transfer and cloning, oocytes may preferably be matured in vivo before these cells may be used as recipient cells for nuclear transfer, and before they were fertilized by the sperm cell to develop into an embryo. Metaphase II stage oocytes, which have been matured in vivo, have been successfully used in nuclear transfer techniques. Essentially, mature metaphase II oocytes are collected surgically from either non-super ovulated or super ovulated animals several hours past the onset of estrus or past the injection of human chorionic gonadotropin (hCG) or similar hormone.

Thus, in one aspect the disclosure provides mammary gland epithelial cells that produce the antibodies or compositions of the disclosure. In some embodiments, the antibody comprises a nucleic acid comprising SEQ ID NO:3 and a nucleic acid comprising SEQ ID NO:4. In some embodiments, the nucleic acid comprising SEQ ID NO:3 and the nucleic acid comprising SEQ ID NO:4 are connected. "Connected" is used herein to mean the nucleic acids are physically linked, e.g., within the same vector or within approximately the same genomic location. In some embodiments, the mammary epithelial cells above are in a transgenic non-human mammal. In some embodiments, the transgenic non-human mammal is a goat.

In another aspect the disclosure provides a method for the production of a transgenic antibody, and variants and fragments thereof, the process comprising expressing in the milk of a transgenic non-human mammal a transgenic antibody encoded by a nucleic acid construct. In some embodiments, the method for producing the antibodies of the disclosure comprises:
(a) transfecting non-human mammalian cells with a transgene DNA construct encoding a cetuximab antibody;
(b) selecting cells in which said cetuximab transgene DNA construct has been inserted into the genome of the cells; and
(c) performing a first nuclear transfer procedure to generate a non-human transgenic mammal heterozygous for the cetuximab antibody and that can express it in its milk.

In some embodiments, the transgene DNA construct comprises SEQ ID NO:3 and/or SEQ ID NO:4. In some embodiments, the non-human transgenic mammal is a goat.
In another aspect, the disclosure provides a method of:
(a) providing a non-human transgenic mammal engineered to express a cetuximab antibody,
(b) expressing the cetuximab antibody in the milk of the non-human transgenic mammal; and
(c) isolating the cetuximab antibody expressed in the milk.

In some embodiments, the cetuximab antibody comprises a heavy chain comprising SEQ ID NO:1 and a light chain comprising SEQ ID NO:2. One of the tools used to predict the quantity and quality of the recombinant protein expressed in the mammary gland is through the induction of lactation (Ebert KM, 1994). Induced lactation allows for the expression and analysis of protein from the early stage of transgenic production rather than from the first natural lactation resulting from pregnancy, which is at least a year later. Induction of lactation can be done either hormonally or manually.

In some embodiments, the compositions of glycosylated antibodies provided herein further comprise milk. In some embodiments, the methods provided herein include a step of isolating a population of antibodies from the milk of a transgenic animal. Methods for isolating antibodies from the milk of transgenic animal are known in the art and are described for instance in Pollock et al., Journal of Immunological Methods, Volume 231, Issues 1-2, 10 December 1999, Pages 147-157. In some embodiments, the methods provided herein include a step of purifying glycosylated antibodies with a desired amount of gal-alpha-1,3-gal.

### Pharmaceutical Compositions, Dosage, and Administration

In one aspect, the disclosure provides pharmaceutical compositions which comprise an amount of an antibody of interest or a pharmaceutically acceptable salt of said antibody and a pharmaceutically acceptable vehicle, diluent or carrier.

In another aspect the disclosure provides a method of treating a subject, comprising administering to a subject a composition provided in an amount effective to treat a disease the subject has or is at risk of having is provided. In one embodiment the subject is a human. In another embodiment the subject is a non-human animal, *e.g.* a dog, cat, horse, cow, pig, sheep, goat or primate. In some embodiments, the disease is cancer. In some embodiments, the cancer comprises cancer cells expressing EGFR. In some embodiments, the cancer comprises cells with a wild-type KRAS gene. In some embodiments, the cancer is head and neck cancer. In some embodiments, the head and neck cancer is squamous cell carcinoma. In some embodiments, the cancer is colorectal cancer. In some embodiments, the colorectal cancer is metastatic colorectal cancer.

Some methods contemplate combination therapy with known cancer medicaments or therapies, for example, chemotherapeutic agents, immunotherapeutic agents, cancer vaccines, hormone therapy, biological response modifiers, surgical procedures, or radiation therapy. It is to be understood that the administering of the antibodies or compositions of the disclosure and the administering of another medicament or therapy can occur simultaneously or non-simultaneously. In one embodiment, the combination therapy comprises administering the antibodies and compositions provided herein in combination with radiation therapy.

In another embodiment the subject is further administered an additional cancer medicament. In one embodiment the additional cancer medicament is a chemotherapeutic agent. In some embodiments, the chemotherapeutic agent is fluorouracil (also referred to as 5-fluorouracil or 5-FU). In some embodiments, the chemotherapeutic agent is irinotecan. In some embodiments, combination therapy comprises:
(a) an antibody or a pharmaceutical composition of the disclosure (e.g., cetuximab);
(b) leucovorin calcium;
(c) fluorouracil; and
(d) irinotecan hydrochloride.
The combination of (b), (c), and (d) is referred to as FOLFIRI in the art. Thus, in some embodiments, methods of treatment comprise administering an antibody or a pharmaceutical composition of the disclosure and FOLFIRI.

According to embodiments that involve administering to a subject in need of treatment a therapeutically effective amount of the antibodies as provided herein, "therapeutically effective" or "an amount effective to treat" denotes the amount of antibody or of a composition needed to inhibit or reverse a disease condition (*e.g*., reduce or inhibit cancer growth). Determining a therapeutically effective amount specifically depends on such factors as toxicity and efficacy of the medicament. These factors will differ depending on other factors such as potency, relative bioavailability, patient body weight, severity of adverse side-effects and preferred mode of administration. Toxicity may be determined using methods well known in the art. Efficacy may be determined utilizing the same guidance. Efficacy, for example, can be measured by a decrease in mass of a target tissue, *e.g.* a tumor. A pharmaceutically effective amount, therefore, is an amount that is deemed by the clinician to be toxicologically tolerable, yet efficacious.

Dosage may be adjusted appropriately to achieve desired drug levels, local or systemic, depending upon the mode of administration. In the event that the response in a subject is insufficient at such doses, even higher doses (or effective higher doses by a different, more localized delivery route) may be employed to the extent that patient tolerance permits. Multiple doses per day are contemplated to achieve appropriate systemic levels of antibodies. Appropriate systemic levels can be determined by, for example, measurement of the patient's peak or sustained plasma level of the drug. "Dose" and "dosage" are used interchangeably herein.

In some embodiments, the amount of antibody or pharmaceutical composition administered to a subject is 50 to 500 mg/m², 100 to 400 mg/m², or 200 to 300 mg/m² per week. In one embodiment the amount of antibody or pharmaceutical composition administered to a subject is 250 mg/m² per week. In some embodiments, an initial dose of 400 mg/m² is administered to a subject the first week, followed by administration of 250 mg/m² to the subject in subsequent weeks. In some embodiments the administration rate is less than 10mg/min. In some embodiments, administration of the antibody or pharmaceutical composition to a subject occurs at least one hour prior to treatment with another therapeutic agent, *e.g.,* 5-FU or FOLFIRI. In some embodiments, a pre-treatment is administered prior to administration of the antibody or pharmaceutical composition. In one embodiment, the pre-treatment is administration of an H₁ antagonist. In one embodiment, the H₁ antagonist if diphenhydramine.

In some embodiments the compositions provided are employed for *in vivo* applications. Depending on the intended mode of administration in vivo the compositions used may be in the dosage form of solid, semi-solid or liquid such as, *e.g.,* tablets, pills, powders, capsules, gels, ointments, liquids, suspensions, or the like. Preferably the compositions are administered in unit dosage forms suitable for single administration of precise dosage amounts. The compositions may also include, depending on the formulation desired, pharmaceutically acceptable carriers or diluents, which are defined as aqueous-based vehicles commonly used to formulate pharmaceutical compositions for animal or human administration. The diluent is selected so as not to affect the biological activity of the human recombinant protein of interest. Examples of such diluents are distilled water, physiological saline, Ringer's solution, dextrose solution, and Hank's solution. The same diluents may be used to reconstitute a lyophilized recombinant protein of interest. In addition, the pharmaceutical composition may also include other medicinal agents, pharmaceutical agents, carriers, adjuvants, nontoxic, non-therapeutic, non-immunogenic stabilizers, etc. Effective amounts of such diluent or carrier are amounts which are effective to obtain a pharmaceutically acceptable formulation in terms of solubility of components, biological activity, etc. In some embodiments the compositions provided herein are sterile.

Administration during *in vivo* treatment may be by any number of routes, including oral, parenteral, intramuscular, intranasal, sublingual, intratracheal, inhalation, ocular, vaginal, and rectal. Intracapsular, intravenous, and intraperitoneal routes of administration may also be employed. The skilled artisan recognizes that the route of administration varies depending on the disorder to be treated. For example, the compositions or antibodies herein may be administered to a subject via oral, parenteral or topical administration and otherwise systemic forms for anti-cancer treatment, *e.g.* head and neck cancer or colorectal cancer. In one embodiment, the compositions or antibodies herein are administered by intravenous infusion.

The compounds, when it is desirable to deliver them systemically, may be formulated for parenteral administration by injection, *e.g.,* by bolus injection or continuous infusion. Formulations for injection may be presented in unit dosage form, *e.g.,* in ampoules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances which increase the viscosity of the suspension, such as sodium carboxymethyl cellulose, sorbitol, or dextran. Optionally, the suspension may also contain suitable stabilizers or agents which increase the solubility of the compounds to allow for the preparation of highly concentrated solutions. Alternatively, the active compounds may be in powder form for constitution with a suitable vehicle, *e.g.,* sterile pyrogen-free water, before use.

For oral administration, the pharmaceutical compositions may take the form of, for example, tablets or capsules prepared by conventional means with pharmaceutically acceptable excipients such as binding agents (*e.g.*, pregelatinised maize starch, polyvinylpyrrolidone or hydroxypropyl methylcellulose); fillers (*e.g*., lactose, microcrystalline cellulose or calcium hydrogen phosphate); lubricants (*e.g*., magnesium stearate, talc or silica); disintegrants (*e.g*., potato starch or sodium starch glycolate); or wetting agents (*e.g*., sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (*e.g*., sorbitol syrup, cellulose derivatives or hydrogenated edible fats); emulsifying agents (*e.g.,* lecithin or acacia); non-aqueous vehicles (*e.g.,* almond oil, oily esters, ethyl alcohol or fractionated vegetable oils); and preservatives (*e.g*., methyl or propyl-p-hydroxybenzoates or sorbic acid). The preparations may also contain buffer salts, flavoring, coloring and sweetening agents as appropriate. The component or components may be chemically modified so that oral delivery of the antibodies is efficacious. Generally, the chemical modification contemplated is the attachment of at least one molecule to the antibodies, where said molecule permits (a) inhibition of proteolysis; and (b) uptake into the blood stream from the stomach or intestine. Also desired is the increase in overall stability of the antibodies and increase in circulation time in the body. Examples of such molecules include: polyethylene glycol, copolymers of ethylene glycol and propylene glycol, carboxymethyl cellulose, dextran, polyvinyl alcohol, polyvinyl pyrrolidone and polyproline. Abuchowski and Davis, 1981, "Soluble Polymer-Enzyme Adducts" In: Enzymes as Drugs, Hocenberg and Roberts, eds., Wiley-Interscience, New York, NY, pp. 367-383; Newmark, et al., 1982, J. Appl. Biochem. 4:185-189. Other polymers that could be used are poly-1,3-dioxolane and poly-1,3,6-tioxocane. Preferred for pharmaceutical usage, as indicated above, are polyethylene glycol molecules. For oral compositions, the location of release may be the stomach, the small intestine (the duodenum, the jejunum, or the ileum), or the large intestine. One skilled in the art has available formulations which will not dissolve in the stomach, yet will release the material in the duodenum or elsewhere in the intestine. Preferably, the release will avoid the deleterious effects of the stomach environment, either by protection of the antibody or by release of the biologically active material beyond the stomach environment, such as in the intestine.

For buccal administration, the compositions may take the form of tablets or lozenges formulated in conventional manner.

For administration by inhalation, the compounds for use according to the present disclosure may be conveniently delivered in the form of an aerosol spray presentation from pressurized packs or a nebulizer, with the use of a suitable propellant, *e.g.,* dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of *e.g.* gelatin for use in an inhaler or insufflator may be formulated containing a powder mix of the compound and a suitable powder base such as lactose or starch.

Also contemplated herein is pulmonary delivery. The compositions can be delivered to the lungs of a mammal while inhaling and traverses across the lung epithelial lining to the blood stream. Contemplated for use in the practice of this disclosure are a wide range of mechanical devices designed for pulmonary delivery of therapeutic products, including but not limited to nebulizers, metered dose inhalers, and powder inhalers, all of which are familiar to those skilled in the art.

Nasal delivery of a pharmaceutical composition of the present disclosure is also contemplated. Nasal delivery allows the passage of a pharmaceutical composition of the present disclosure to the blood stream directly after administering the therapeutic product to the nose, without the necessity for deposition of the product in the lung. Formulations for nasal delivery include those with dextran or cyclodextran.

The compounds may also be formulated in rectal or vaginal compositions such as suppositories or retention enemas, *e.g.,* containing conventional suppository bases such as cocoa butter or other glycerides.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Suitable liquid or solid pharmaceutical preparation forms are, for example, aqueous or saline solutions for inhalation, microencapsulated, encochleated, coated onto microscopic gold particles, contained in liposomes, nebulized, aerosols, pellets for implantation into the skin, or dried onto a sharp object to be scratched into the skin. The pharmaceutical compositions also include granules, powders, tablets, coated tablets, (micro)capsules, suppositories, syrups, emulsions, suspensions, creams, drops or preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of methods for drug delivery, see Langer, Science 249:1527-1533, 1990.

The antibodies and optionally other therapeutics may be administered per se (neat) or in the form of a pharmaceutically acceptable salt. When used in medicine the salts should be pharmaceutically acceptable, but non-pharmaceutically acceptable salts may conveniently be used to prepare pharmaceutically acceptable salts thereof. Such salts include, but are not limited to, those prepared from the following acids: hydrochloric, hydrobromic, sulphuric, nitric, phosphoric, maleic, acetic, salicylic, p-toluene sulphonic, tartaric, citric, methane sulphonic, formic, malonic, succinic, naphthalene-2-sulphonic, and benzene sulphonic. Also, such salts can be prepared as alkaline metal or alkaline earth salts, such as sodium, potassium or calcium salts of the carboxylic acid group.

Suitable buffering agents include: acetic acid and a salt (1-2% w/v); citric acid and a salt (1-3% w/v); boric acid and a salt (0.5-2.5% w/v); and phosphoric acid and a salt (0.8-2% w/v). Suitable preservatives include benzalkonium chloride (0.003-0.03% w/v); chlorobutanol (0.3-0.9% w/v); parabens (0.01-0.25% w/v) and thimerosal (0.004-0.02% w/v).

The pharmaceutical compositions of the disclosure contain an effective amount of the antibodies and optionally therapeutic agents included in a pharmaceutically-acceptable carrier. The term pharmaceutically-acceptable carrier means one or more compatible solid or liquid filler, diluents or encapsulating substances which are suitable for administration to a human or other vertebrate animal. The term carrier denotes an organic or inorganic ingredient, natural or synthetic, with which the active ingredient is combined to facilitate the application. The components of the pharmaceutical compositions also are capable of being commingled with the compounds of the present disclosure, and with each other, in a manner such that there is no interaction which would substantially impair the desired pharmaceutical efficiency.

The therapeutic agent(s), including specifically but not limited to the antibodies, may be provided in particles. Particles, as used herein, include nano or microparticles (or in some instances larger) which can consist in whole or in part of the antibody or other therapeutic agents administered with the antibody. The particle may include, in addition to the therapeutic agent(s), any of those materials routinely used in the art of pharmacy and medicine, including, but not limited to, erodible, nonerodible, biodegradable, or nonbiodegradable material or combinations thereof. The particles may be microcapsules which contain the antibody in a solution or in a semi-solid state. The particles may be of virtually any shape.

### Methods of Production

One aspect of the disclosure provides a method for the production of a glycosylated antibody or population of glycosylated antibodies, the process comprising expressing in the milk of a transgenic non-human mammal a glycosylated antibody encoded by a nucleic acid construct. In one embodiment the mammalian mammary epithelial cells are of a non-human mammal engineered to express the antibody in its milk. In yet another embodiment the mammalian mammary epithelial cells are mammalian mammary epithelial cells in culture.

In another embodiment the method comprises:
(a) providing a non-human transgenic mammal engineered to express an antibody,
(b) expressing the antibody in the milk of the non-human transgenic mammal;
(c) isolating the antibodies expressed in the milk; and
(d) detecting the presence of gal-alpha-1,3-gal moieties on the isolated antibodies.

In yet another embodiment, the method comprises producing a population of glycosylated antibodies in mammary gland epithelial cells such that the population of glycosylated antibodies produced comprises fewer galactose-alpha-1,3-galactose moieties than the population of glycosylated antibodies produced in non-mammary cell culture, wherein the glycosylated antibodies comprise a heavy chain comprising SEQ ID NO:1 and a light chain comprising SEQ ID NO:2. In some embodiments, this method is performed in vitro. In other embodiments, this method is performed *in vivo, e.g.* in the mammary gland of a transgenic goat.

In some embodiments the methods above further comprise steps for inducing lactation. In still other embodiments the methods further comprise additional isolation and/or purification steps. In yet other embodiments the methods further comprise steps for comparing the glycosylation pattern of the antibodies obtained with antibodies produced in cell culture, e.g. non-mammary cell culture. In further embodiments, the methods further comprise steps for comparing the glycosylation pattern of the antibodies obtained to antibodies produced by non-mammary epithelial cells. Such cells can be cells of a cell culture. In some embodiments, the glycosylation pattern is the gal-alpha-1,3-gal pattern, e.g. the amount of gal-alpha-1,3-gal moieties present on an antibody or population of antibodies. In some embodiments, the method further comprises comparing the number of galactose-alpha-1,3-galactose moieties present in the population of glycosylated antibodies to the number of galactose-alpha-1,3-galactose moieties present in a population of glycosylated antibodies produced in cell culture, e.g. non-mammary cell culture. Experimental techniques for assessing the glycosylation pattern of the antibodies can be any of those known to those of ordinary skill in the art or as provided herein, such as below in the Examples. Such methods include, *e.g.,* liquid chromatography mass spectrometry, tandem mass spectrometry, and Western blot analysis.

The antibodies can be obtained, in some embodiments, by collecting the antibodies from the milk of a transgenic animal produced as provided herein or from an offspring of said transgenic animal. In some embodiments the antibodies produced by the transgenic mammal is produced at a level of at least 1 gram per liter of milk produced.

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well-known in the art. Generally, nomenclatures used in connection with, and techniques of biochemistry, enzymology, molecular and cellular biology, microbiology, genetics and protein and nucleic acid chemistry and hybridization described herein are those well-known and commonly used in the art. The methods and techniques of the present disclosure are generally performed according to conventional methods well known in the art and as described in various general and more specific references that are cited and discussed throughout the present specification unless otherwise indicated.

The present disclosure is further illustrated by the following Examples, which in no way should be construed as further limiting.

### EXAMPLES

### Methods

**Table 1. SEQ ID NO list**

| **SEQ ID NO** | **Description** |
|---|---|
| 1 | Heavy chain amino acid sequence of cetuximab |
| 2 | Light chain amino acid sequence of cetuximab |
| 3 | Heavy chain nucleotide sequence of cetuximab |
| 4 | Light chain nucleotide sequence of cetuximab |
| 5 | Bc2553 |
| 6 | Bc2554 |
| 7 | Bc2584 |

### Gene encoding cetuximab

The amino acid sequence of cetuximab/Erbitux® was published in Charlotte Magdelaine-Beuzelin, et al., Critical Reviews in Oncology/Hematology 64 (2007) 210-225 and used as a reference for confirmation of the sequence of commercially-produced Erbitux®.

### Peptide Map

The commercially produced cetuximab, Erbitux®, was analyzed through generation of the peptide maps of the heavy and light chain and the published amino acid sequence was confirmed. The peptide maps were generated though trypsin and Asp-N digestion, followed by HPLC and mass spec analysis. The amino acid sequences of commercially produced cetuximab were then used to design DNA expression sequences for expression of cetuximab in the milk of transgenic animals.

### Beta Casein Expression Constructs

The DNA sequences of the cetuximab heavy and light chains were optimized for expression in a bovine system (SEQ ID NOs: 3 (heavy chain) and 4 (light chain)). All constructs described herein contain the optimized sequences.

The constructs used herein include the coding sequence of cetuximab, a Kozak consensus sequence as well as flanking XhoI sites to facilitate cloning into beta casein expression vectors. The expression vectors used for this project are Bc450, Bc451, Bc800, and Bc350, all of which were in the supercos background which confers both Amp^{R} and Kan^{R}.

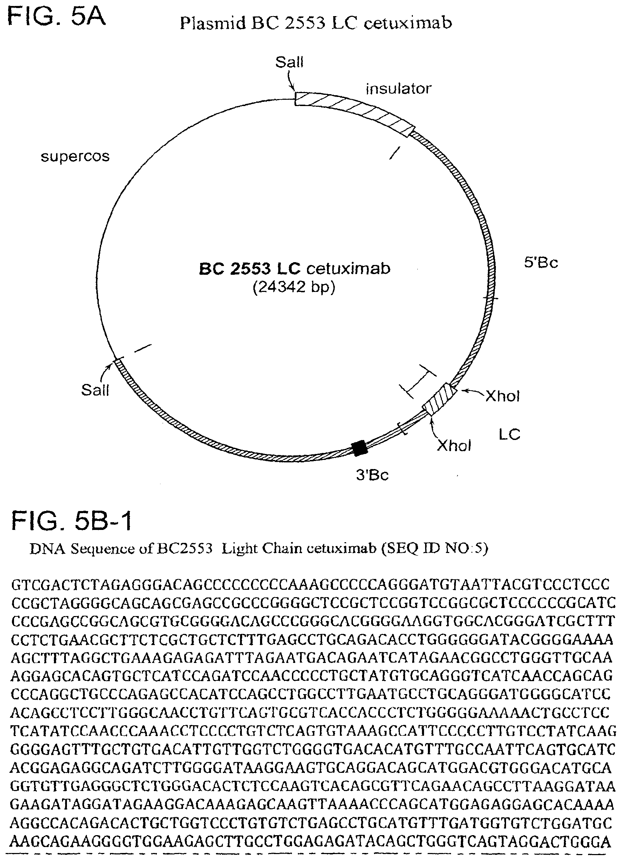

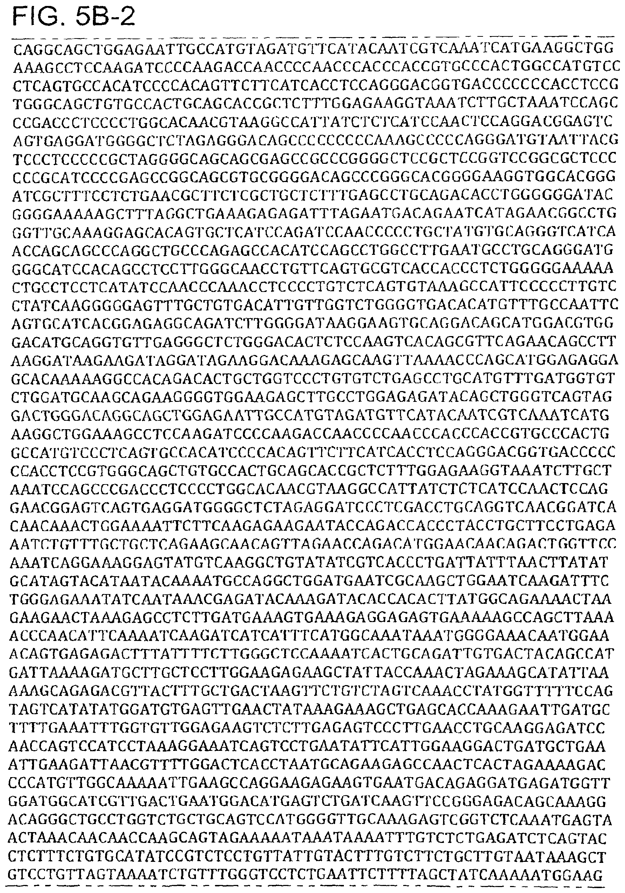

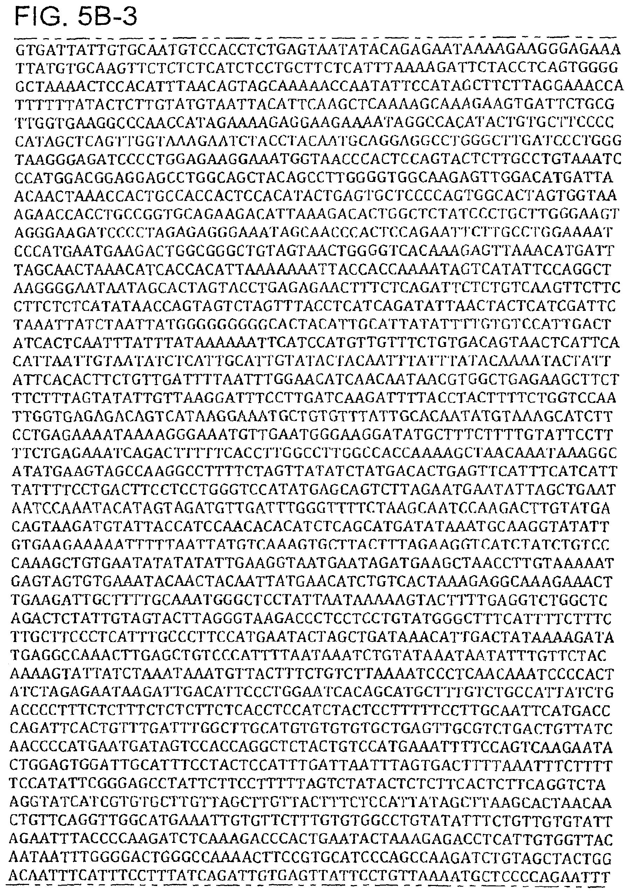

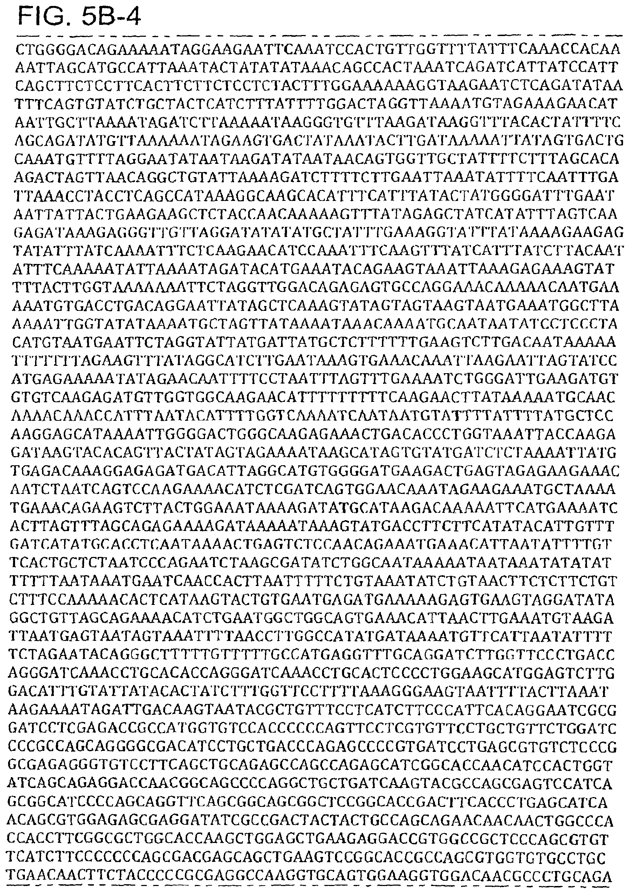

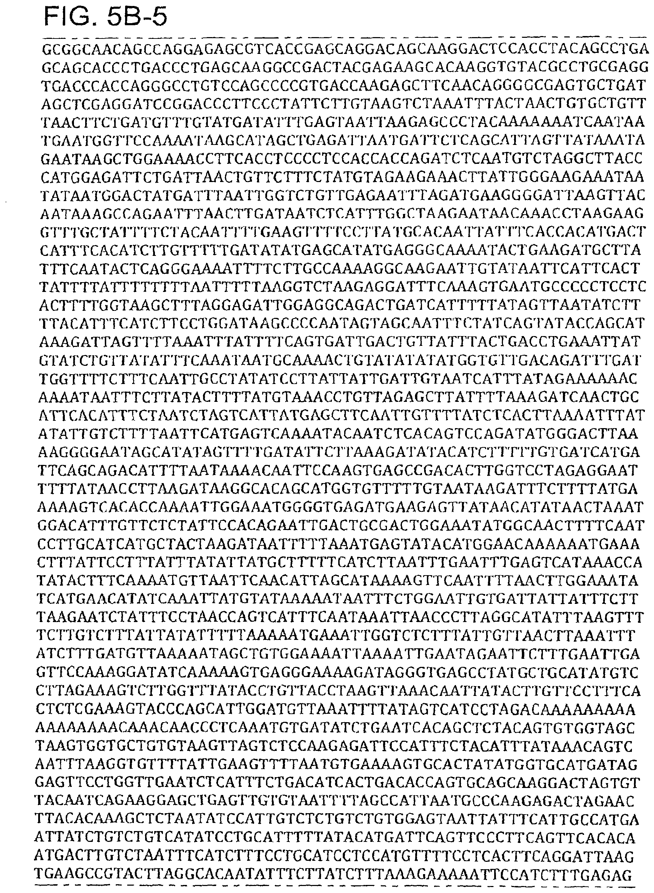

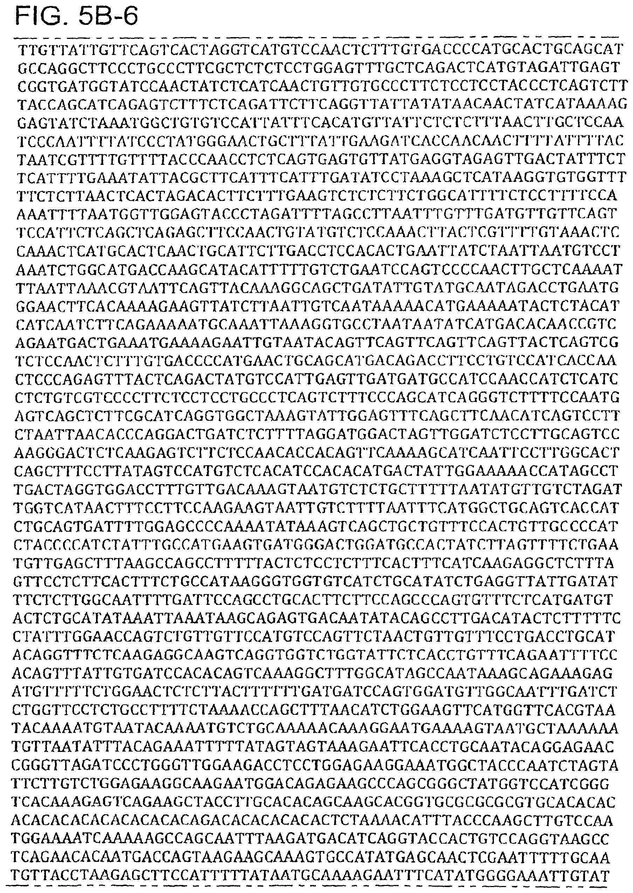

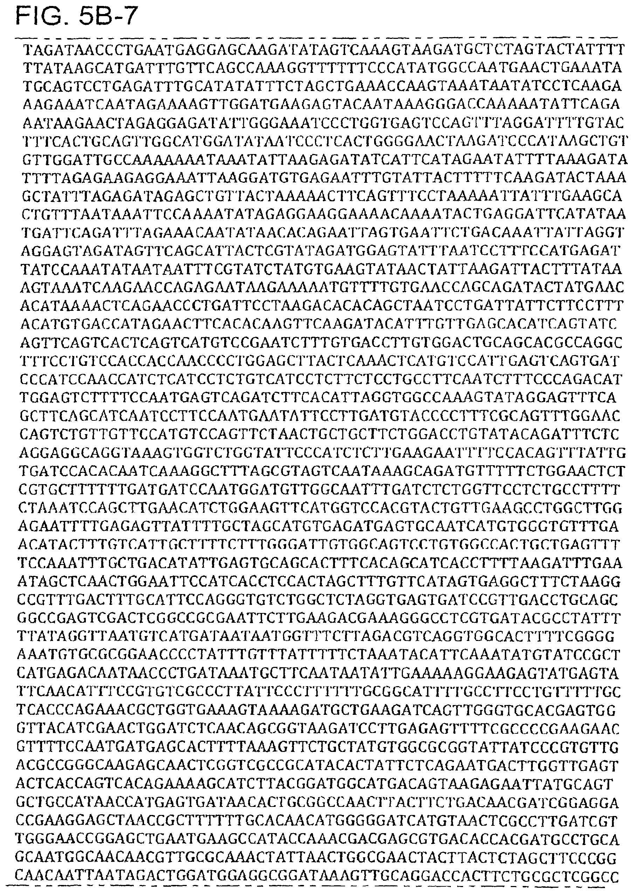

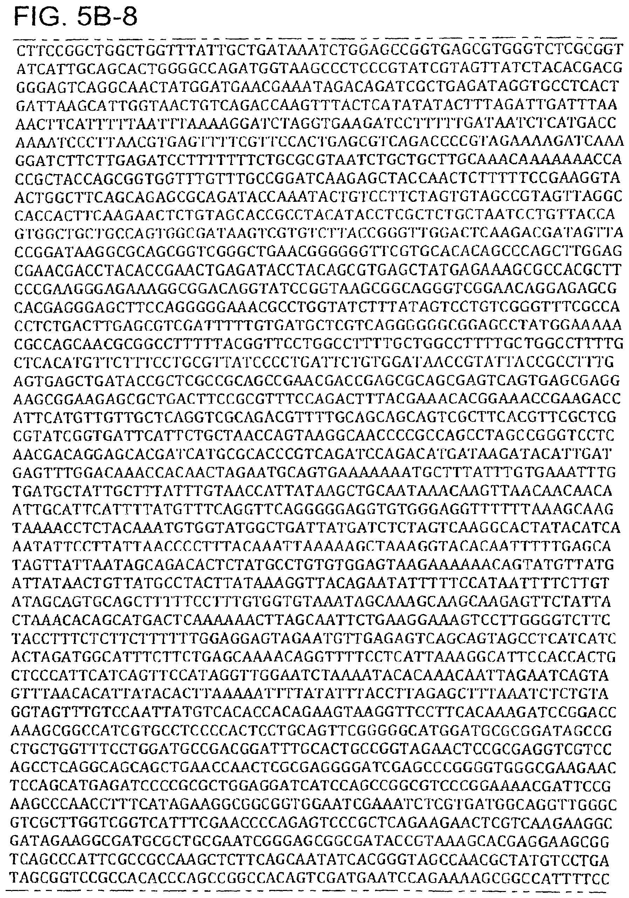

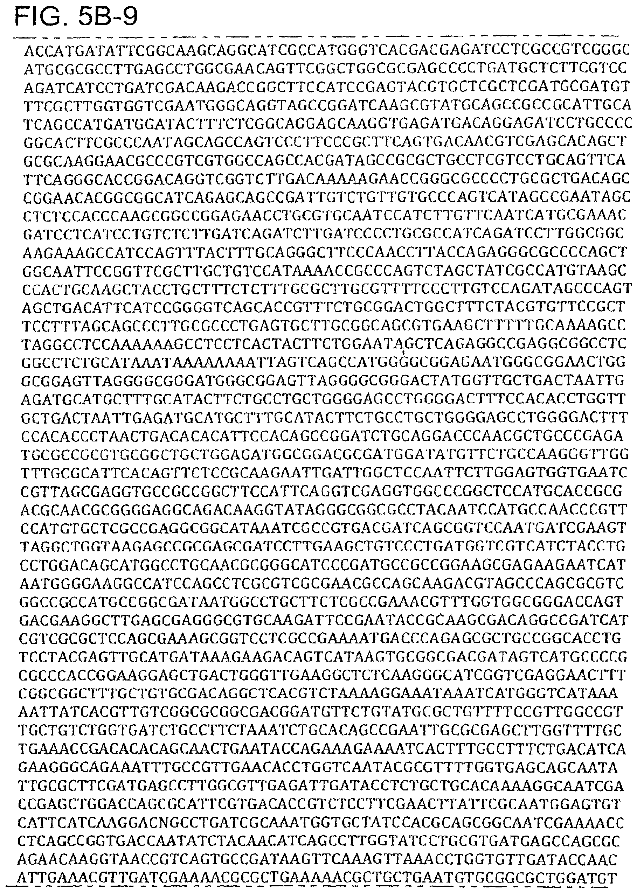

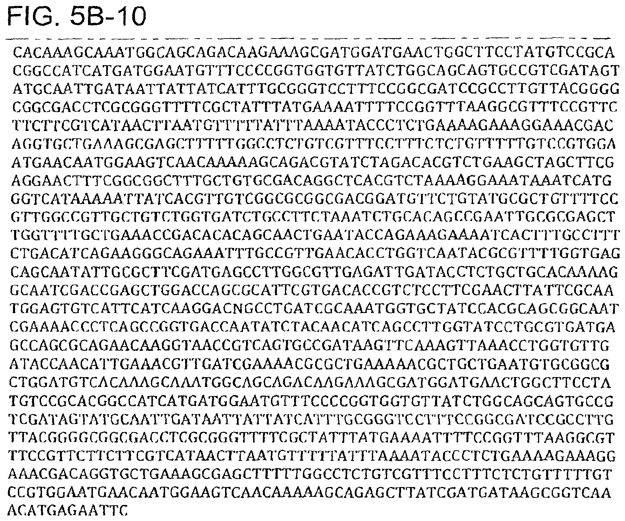

Plasmid Bc2553 (Figure 5) contains the cetuximab light chain nucleotide sequence (SEQ ID NO:4) in expression vector Bc450, which includes a chicken beta globin insulator sequence, 6.2 kb of 5' beta casein sequence, an XhoI cloning site, and 7.1kb of 3' beta casein flanking sequence, in a supercos backbone. The supercos vector can be removed using flanking SalI sites.

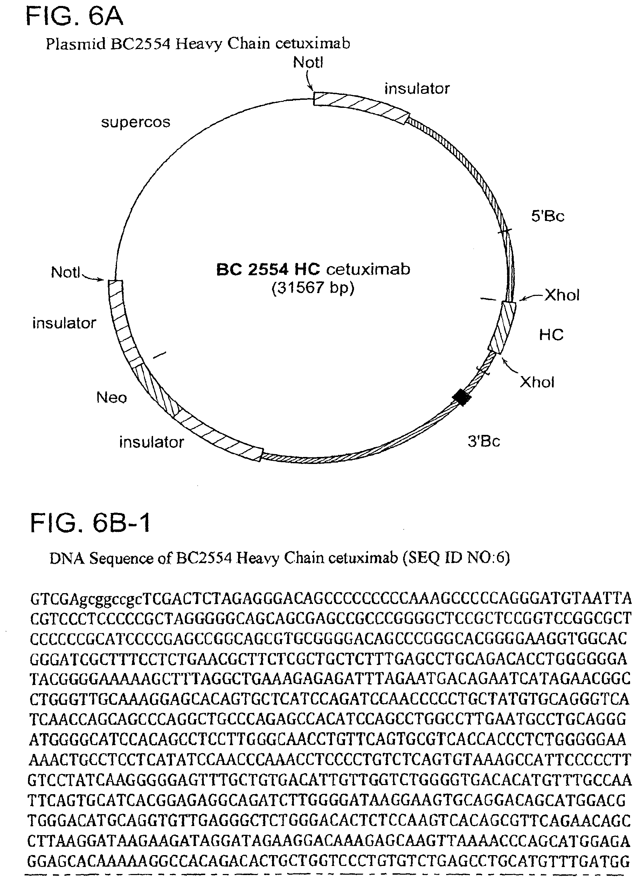

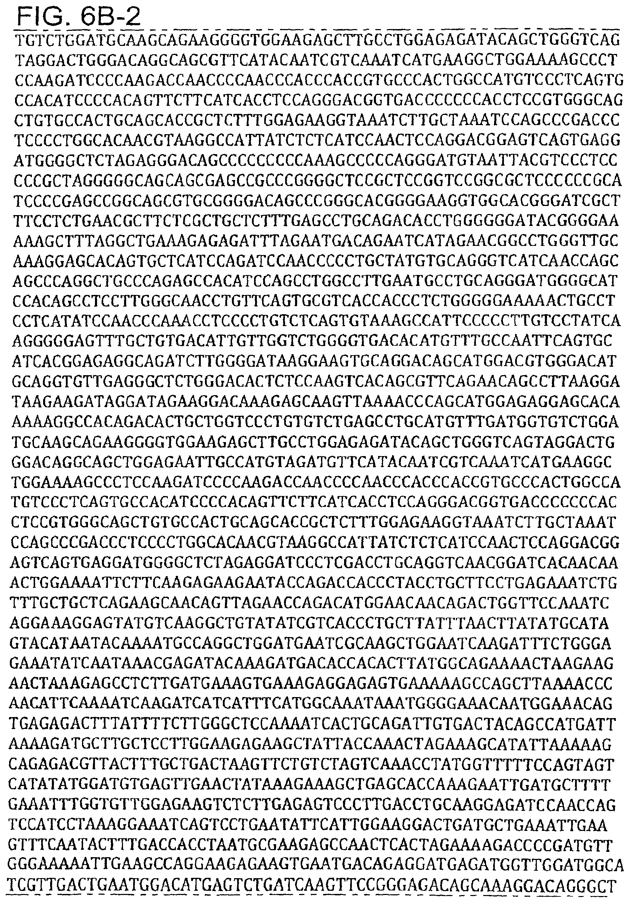

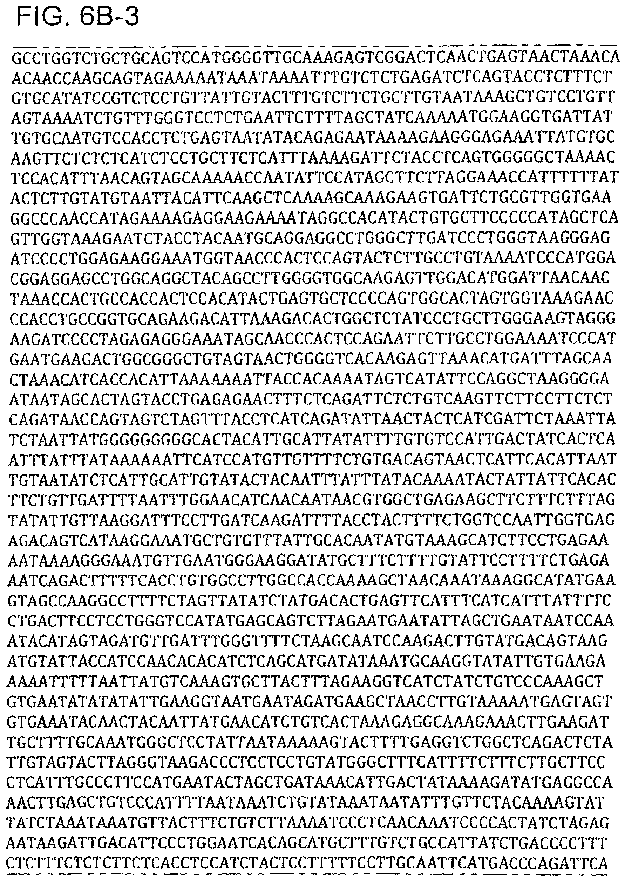

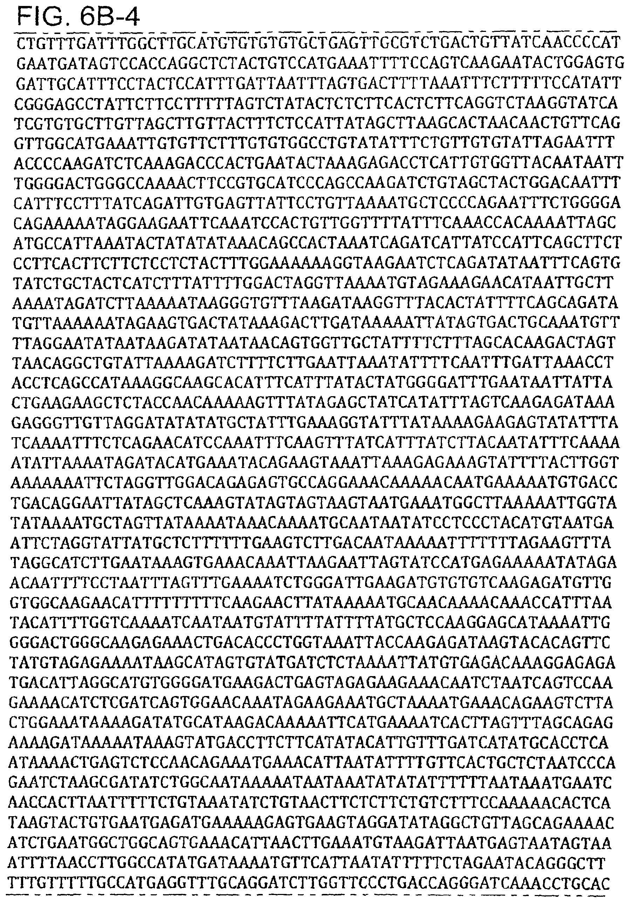

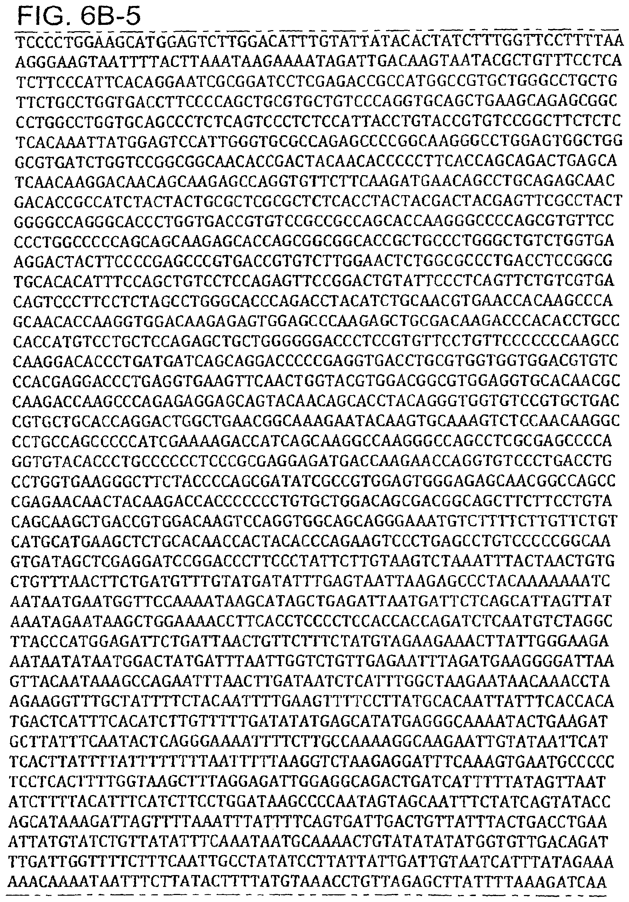

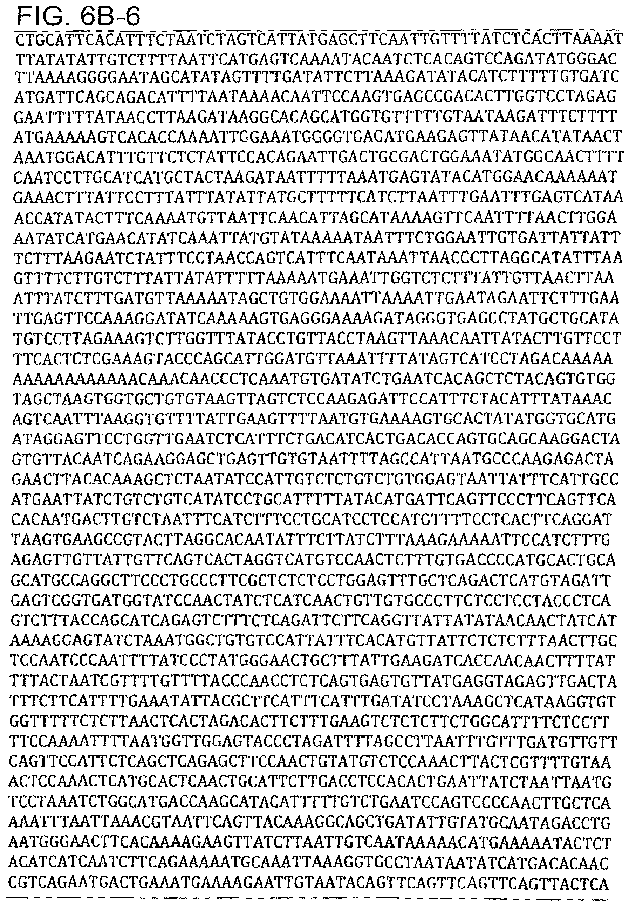

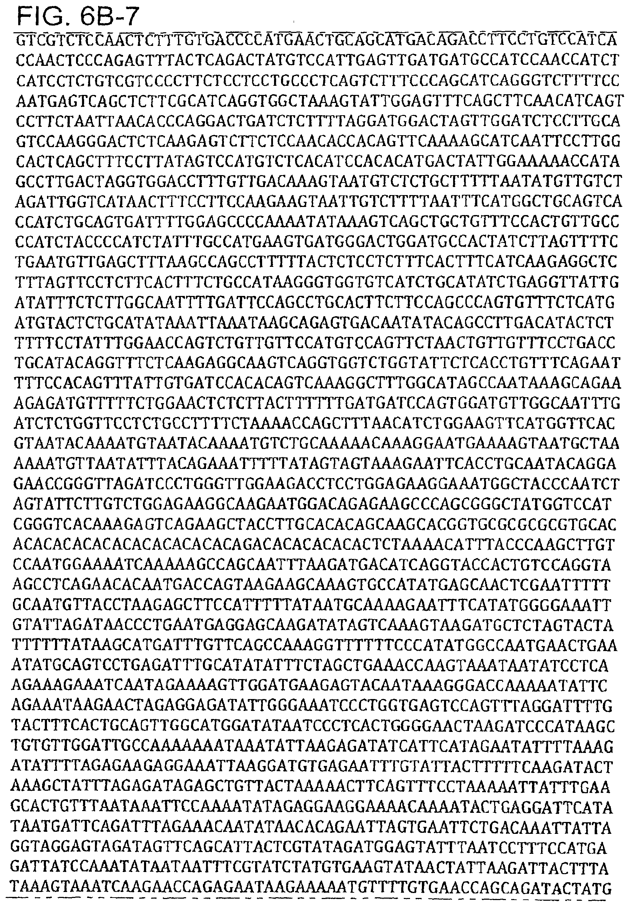

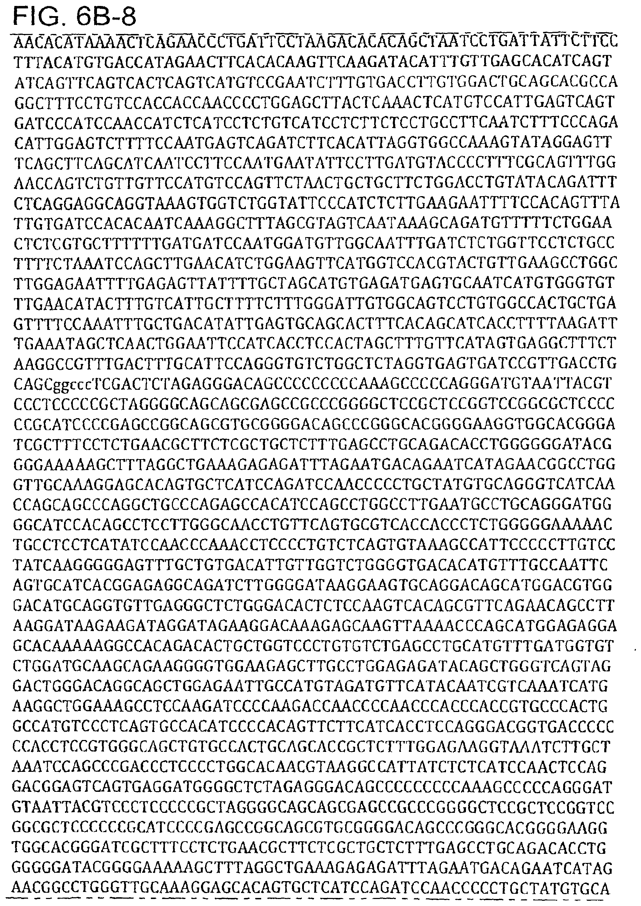

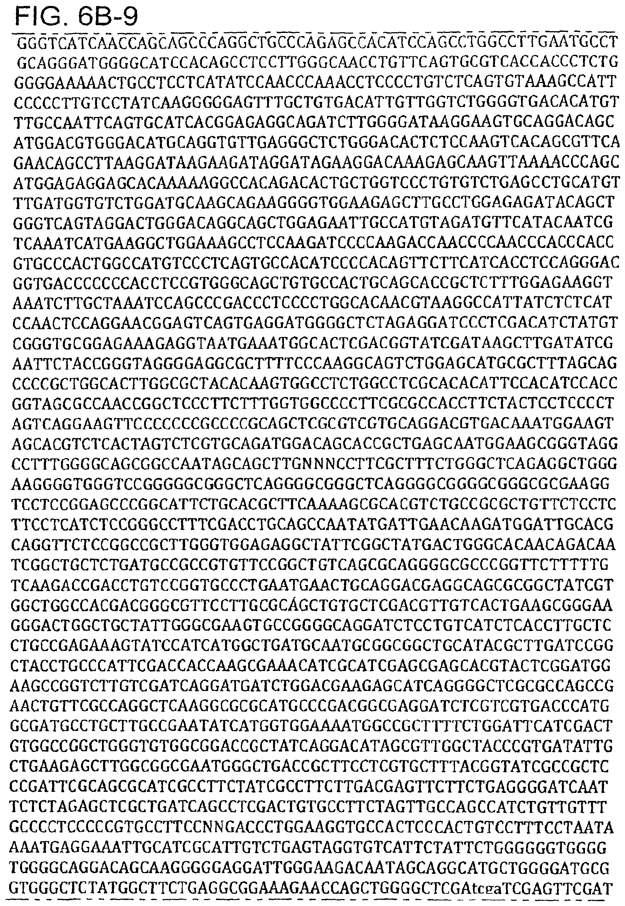

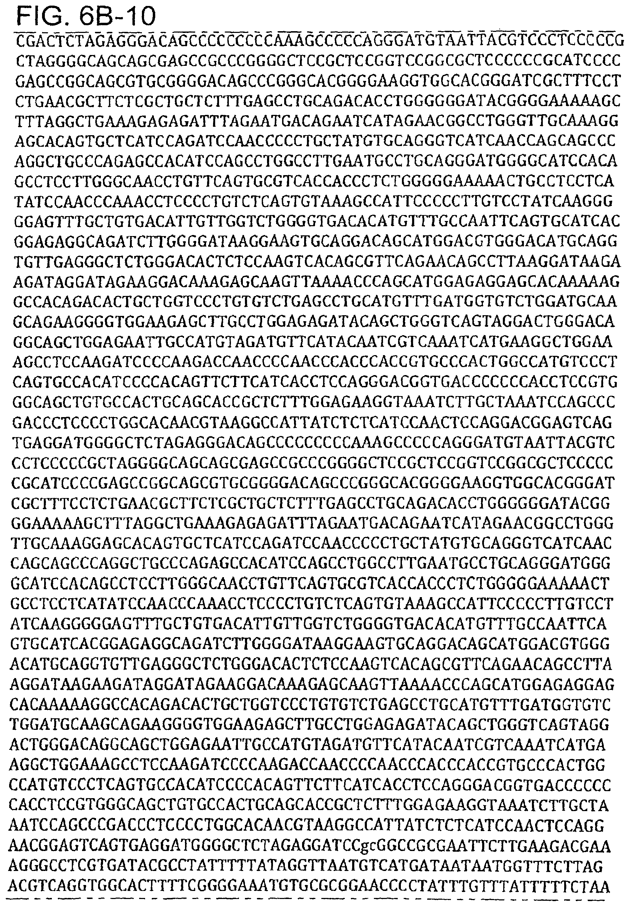

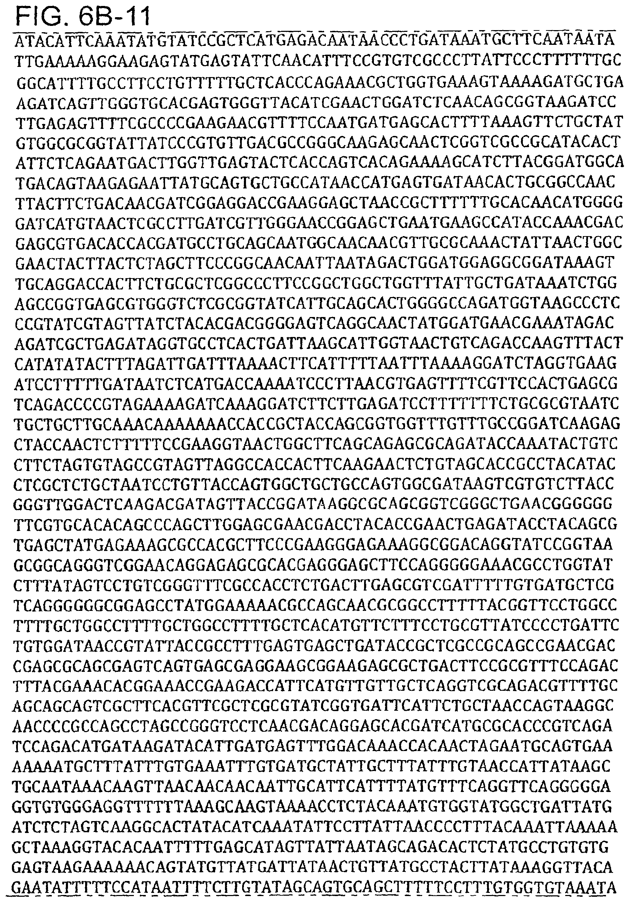

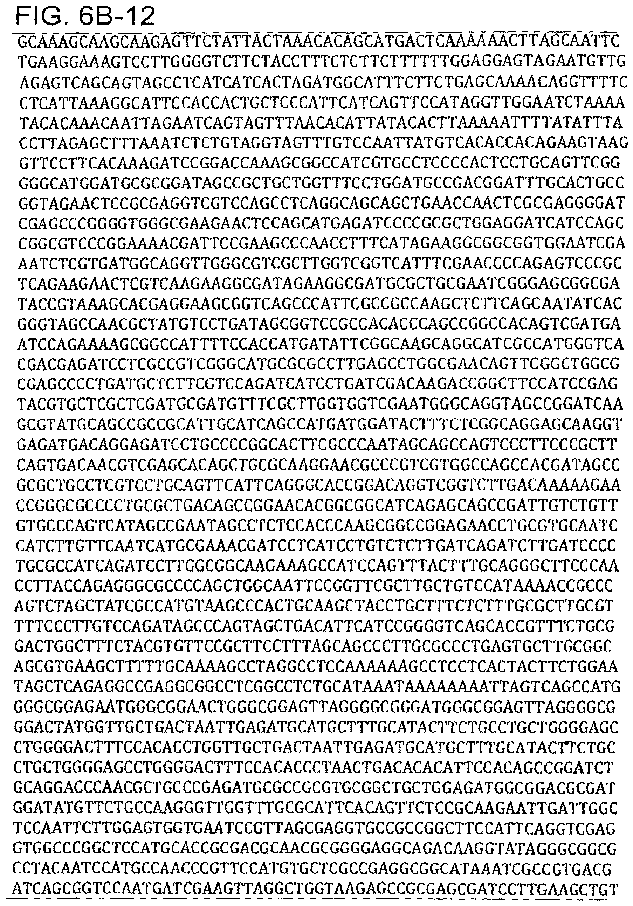

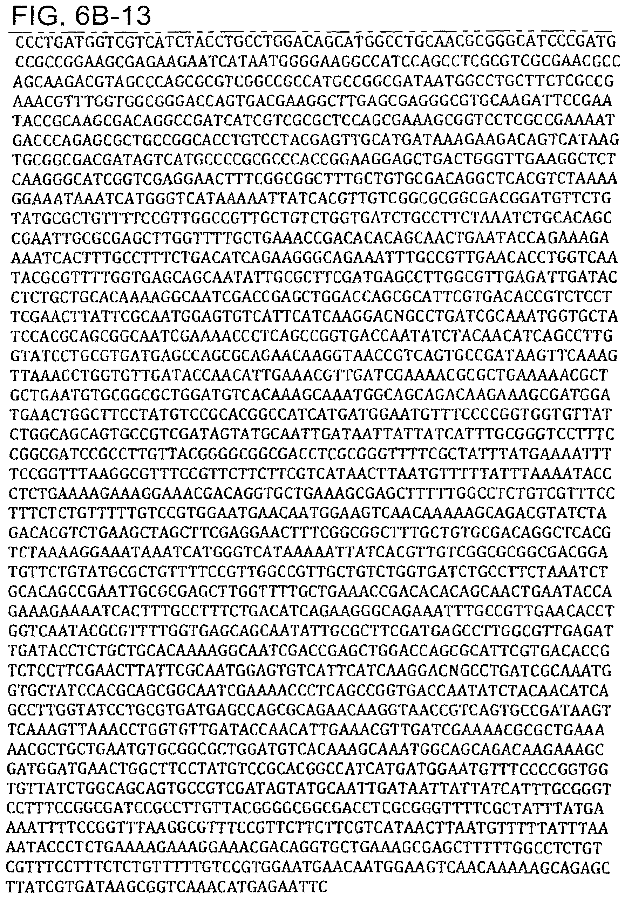

Plasmid Bc2554 (Figure 6) contains the cetuximab heavy chain nucleotide sequence (SEQ ID NO:3) in expression vector Bc800, which includes the same chicken beta globin insulator sequence, 6.2 kb of 5' beta casein sequence, XhoI cloning site, and 7.1kb of 3' beta casein flanking sequence, with the addition of a Neo^{R} marker flanked by additional chicken beta globin insulator sequences, again in a supercos backbone. The supercos vector was removed using NotI sites present at both ends.

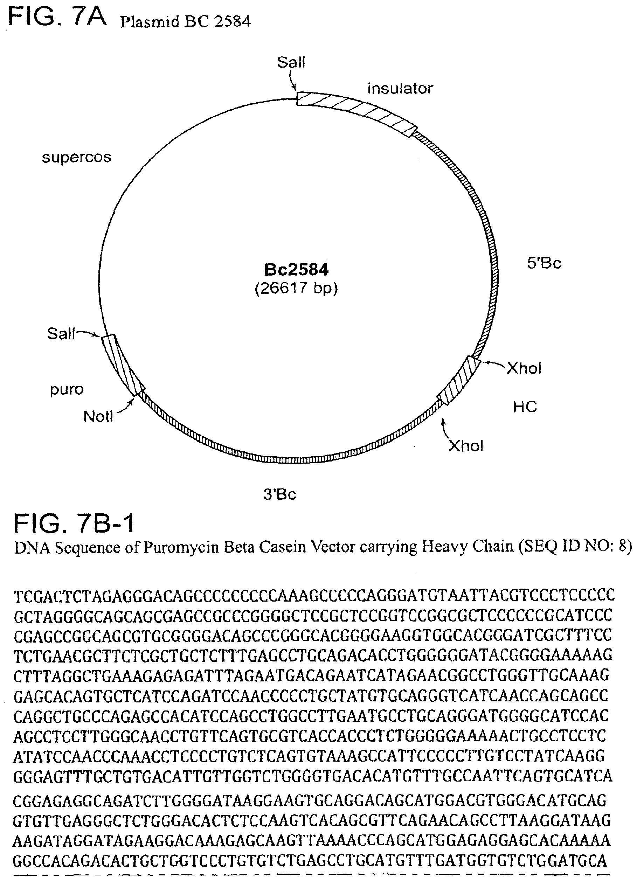

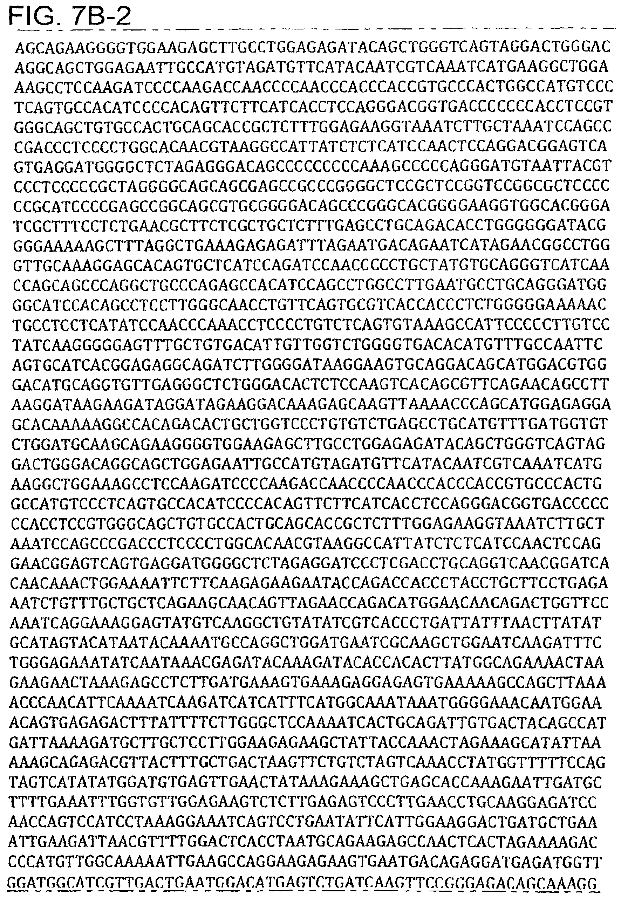

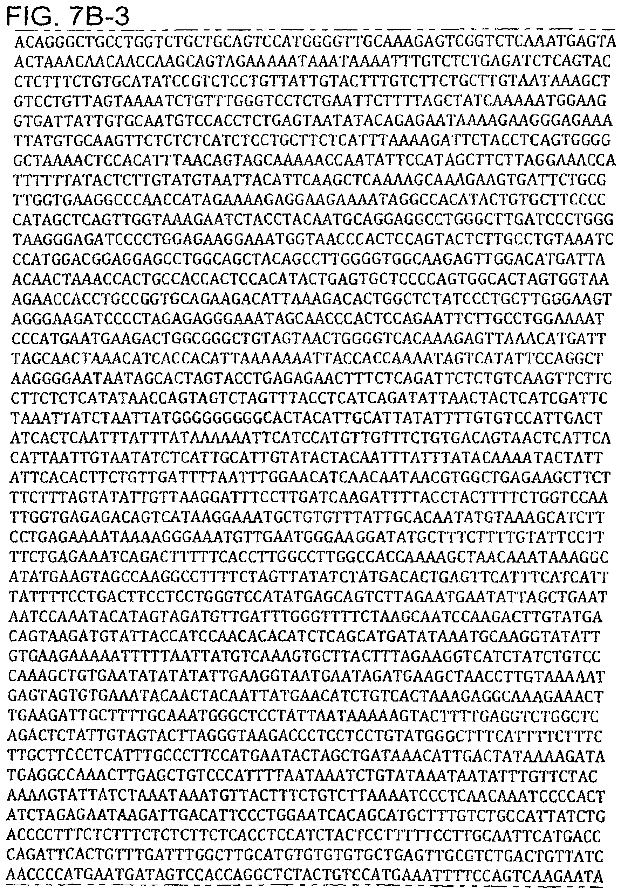

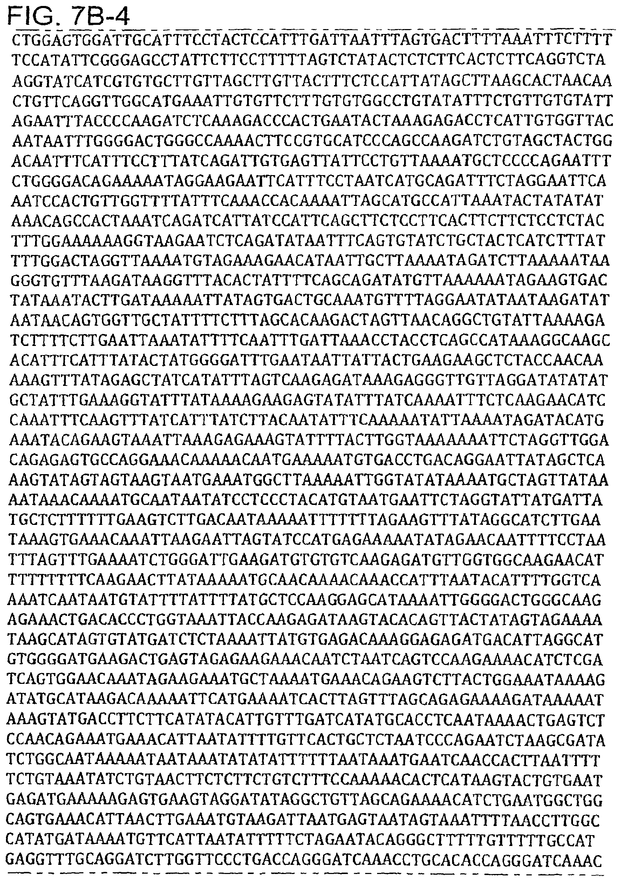

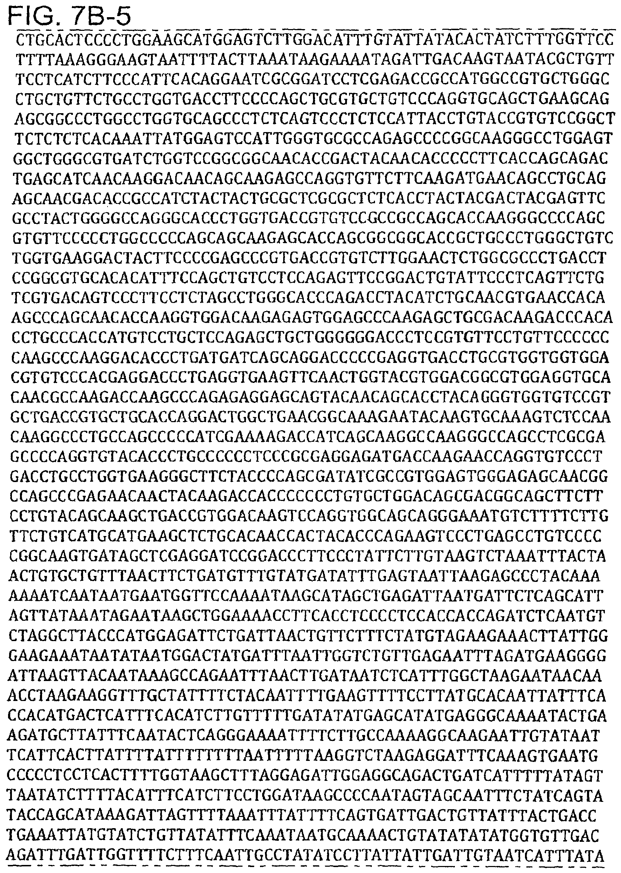

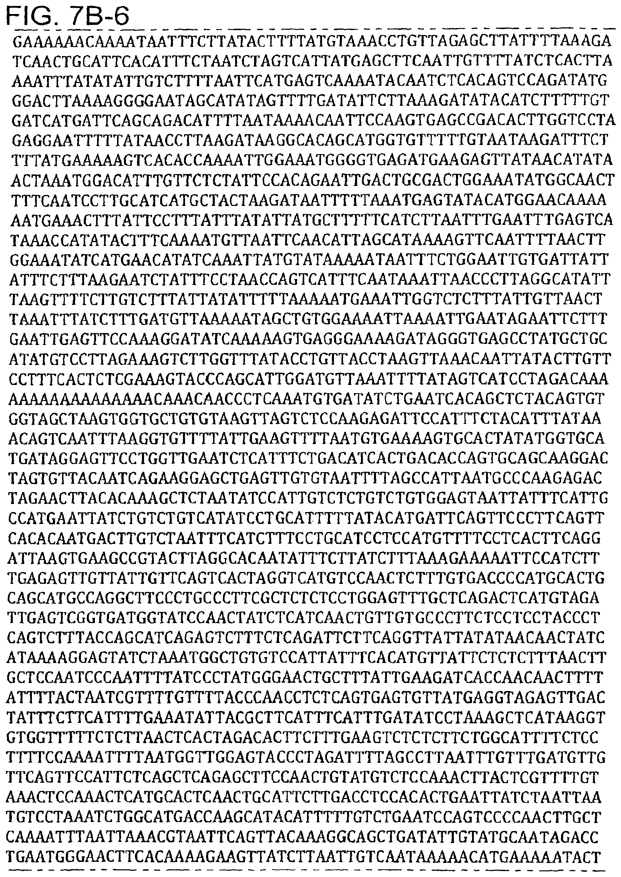

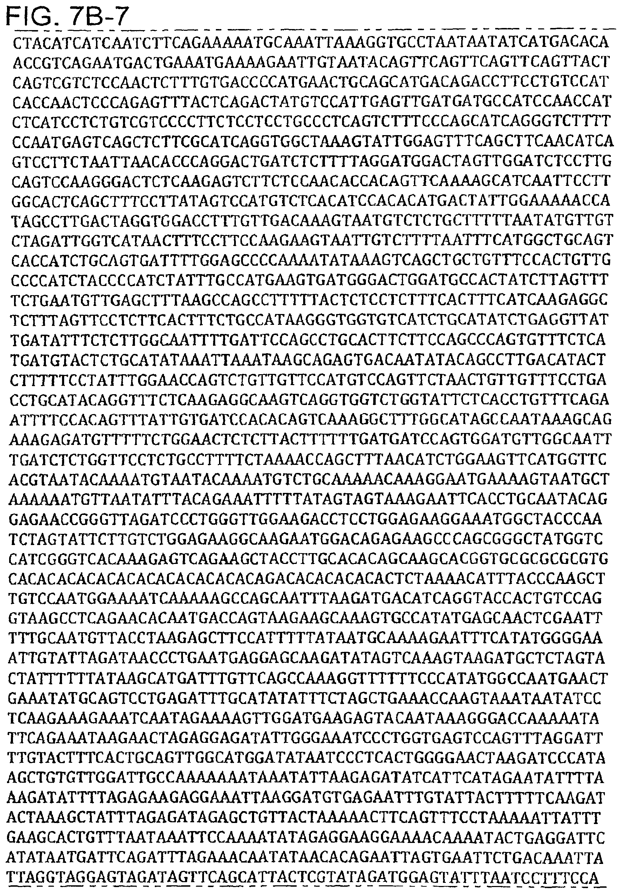

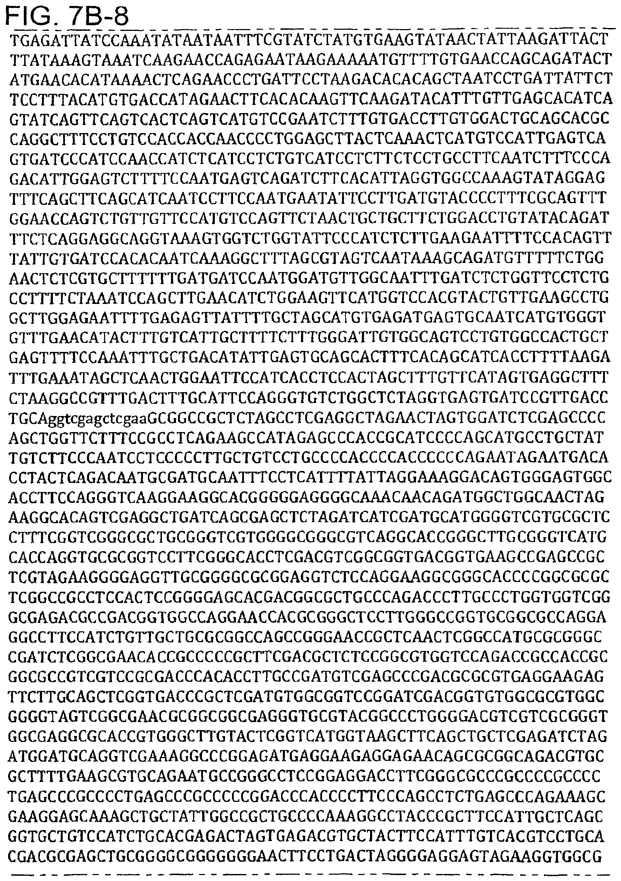

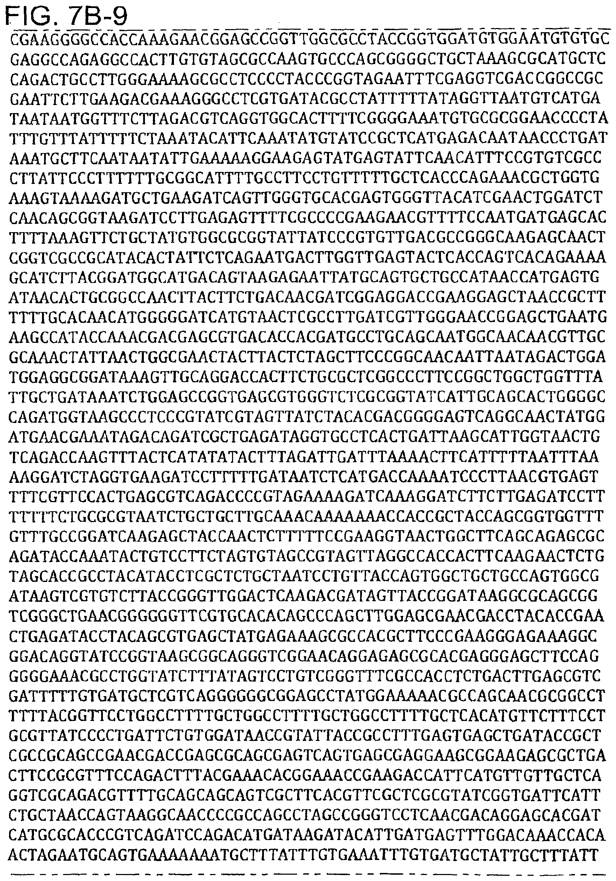

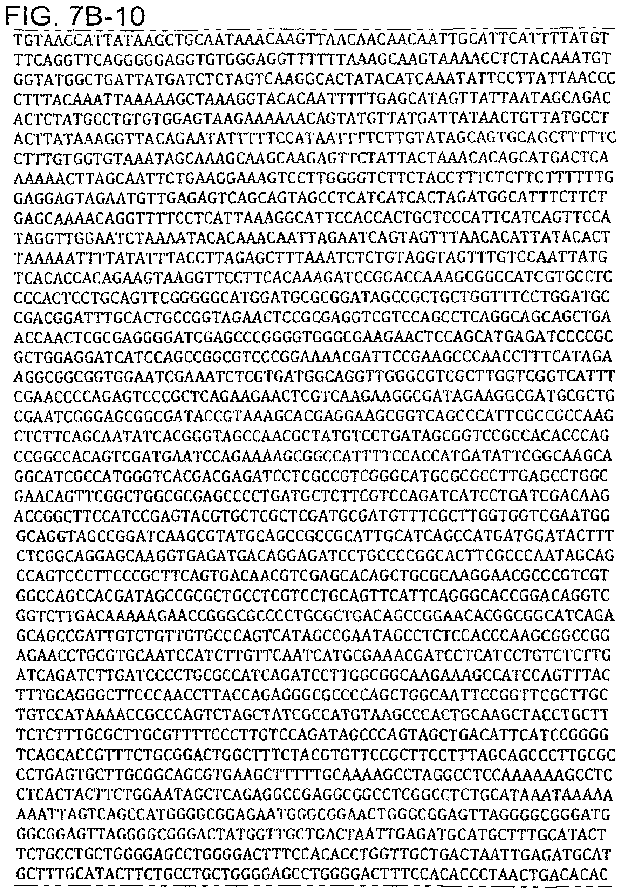

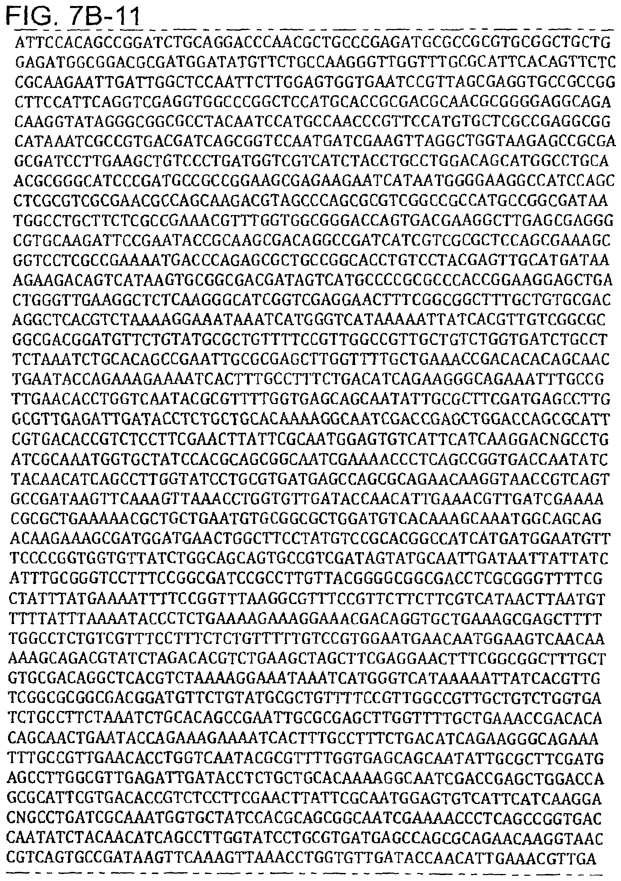

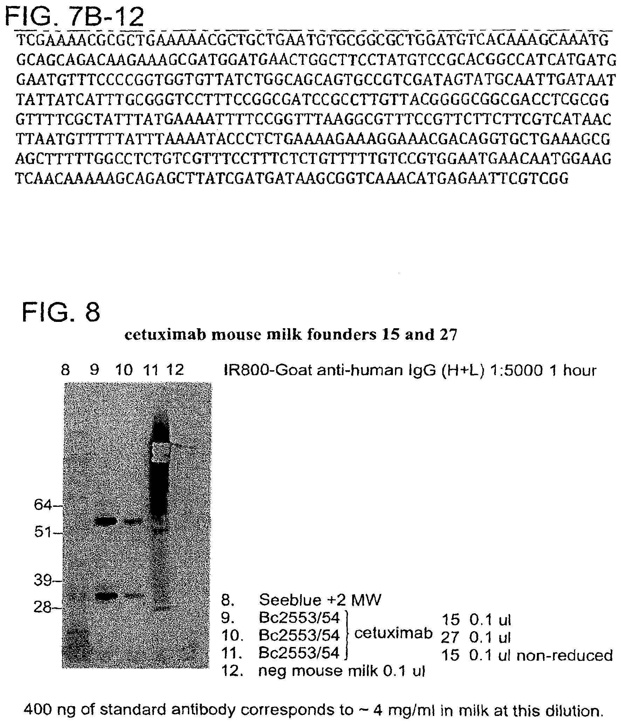

Three pieces of DNA were ligated to generate plasmid Bc2584 (Figure 7). The first piece was made by inserting the heavy chain coding sequence into Bc350, which is identical to Bc450 described above except for the replacement of the downstream SalI site with Not I. This SalI to NotI fragment, containing the insulator, beta casein, and heavy chain sequences, was ligated to a SalI to NotI fragment of pGL71, containing the puromycin resistance gene, and a supercos fragment with flanking SalI sites.

### Generation of Transgenic Mice

The beta casein constructs carrying the heavy and light chains of cetuximab (SEQ ID NO:6 and SEQ ID NO:5; BC2554 and BC2553, respectively) were used to generate transgenic mice using standard pro-nuclear micro-injection technology. The injection fragment for the heavy chain was isolated from BC2554 HC Neo, by digesting with NotI to release the supercos backbone and electro-eluting the beta casein heavy chain injection fragment. Likewise, the supercos was removed from the beta casein light chain fragment by digesting with Sail followed by electro-elution of the injection fragment.

### Generation of Transgenic Goats

Transgenic goats were generated by Somatic Cell Nuclear Transfer, (SCNT) or cloning. A selection marker was added to the beta casein expression constructs in order to select cell cultures with the desired transgenes. The marker was added by ligating the puromycin resistance gene to beta casein heavy chain to yield BC2584 HC puro (SEQ ID NO:7; Figure 7). The DNA constructs were used to transfect primary female fibroblasts. Following selection, the clones were characterized to ensure that both heavy and light chain constructs were integrated. The SCNT was carried out and two female goats were generated from the G100 cell line. These two goats #1 and #2 were induced to lactate in order to produce cetuximab in their milk.

### LC/MS

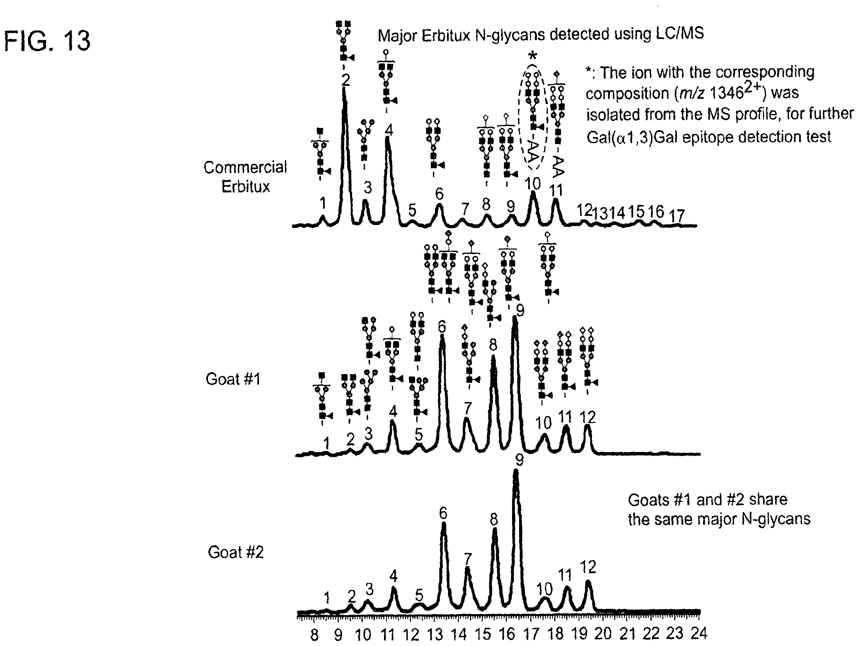

Purified cetuximab from goats #1 and #2 and commercial Erbitux® were analyzed by liquid chromatography-mass spectrometry (LC/MS) using methods well-known in the art (See Figure 13).

### Results

### Peptide Map

The peptide map confirmed that the sequence of the variable region of commercially produced cetuximab (Erbitux®) was correct as published in Charlotte Magdelaine-Beuzelin et al., (Critical Reviews in Oncology/Hematology 64 (2007) 210-225). In addition, the peptide maps confirmed that the heavy chain constant region was human IgG1 and the light chain carried the kappa constant region.

### Expression in Mice

Several lines of mice were identified that carried both the heavy and light chain constructs of cetuximab in their genome. The mice were bred upon maturity and milked following parturition. Milk was analyzed by Western blot for the presence of cetuximab to determine level of expression. The levels of expression of cetuximab in two of the mouse lines (#15 and #27), is shown in Figure 8.

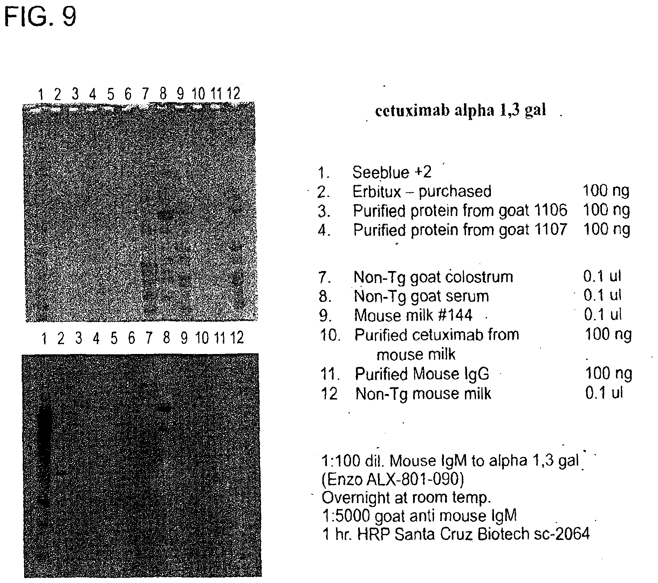

Cetuximab was purified from the milk of the mice and analyzed on a Western blot to determine if alpha-1,3-gal was present. As seen in Figure 9, there is no evidence of alpha-1,3-gal in the mouse produced antibody (Lane 10). In contrast, commercially produced cetuximab (Erbitux®) did show alpha-1,3-gal (Lane 2).

### Expression in Goats

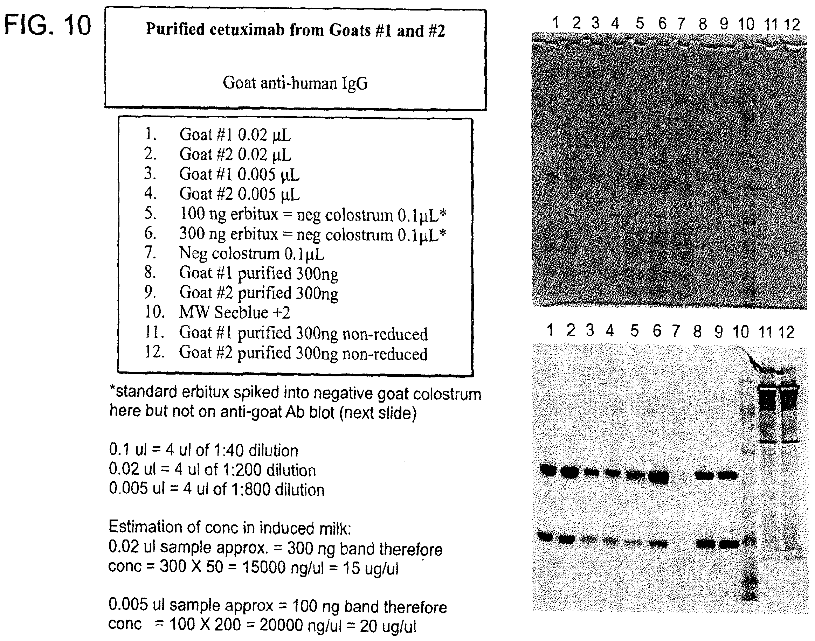

The transgenic goats that were derived from the cell line G100 using the methods described above, were transgenic for the beta casein vector linked to both heavy and light chains of cetuximab. The animals were induced to lactate at 3 months of age. Milk was collected and analyzed by Western blot to determine level of expression of cetuximab. Goats #1 and #2 each produced 10-15 mg/ml of cetuximab in their milk as shown by Western blot in which anti-human IgG1 was used to detect the recombinant antibody (Figure 10).

### Glycosylation analysis

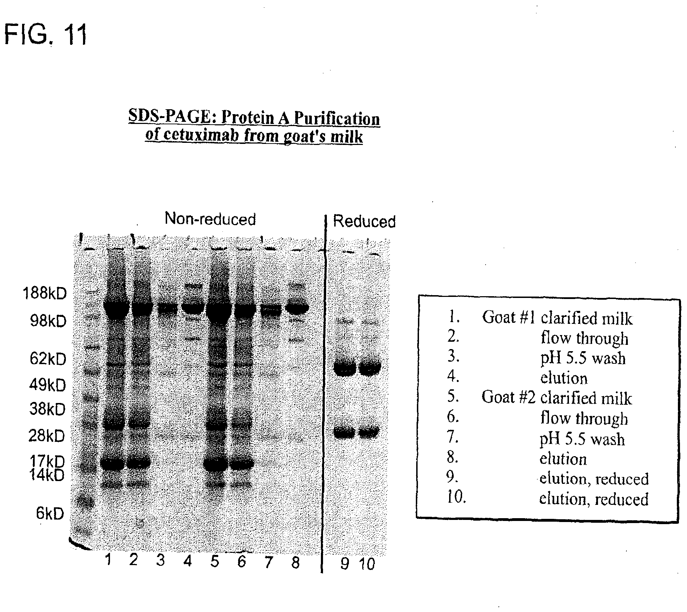

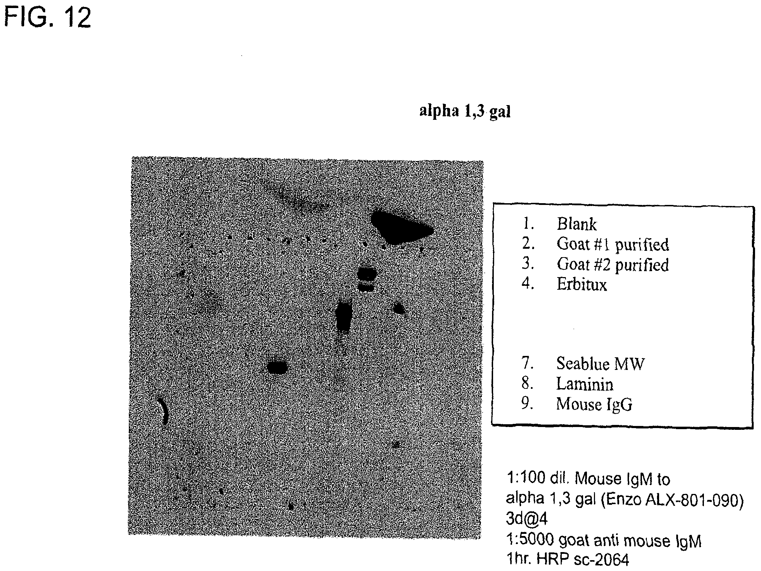

In order to determine if alpha-1,3-gal was present on the goat milk-produced cetuximab, the antibody was purified as shown in Figure 11. A Western blot was subsequently performed using a monoclonal antibody specific to alpha-1,3-gal (Figure 12). As a positive control, bovine laminin was used. The blot showed that commercially produced Erbitux® and laminin bound the alpha-1,3-gal monoclonal antibody, but the cetuximab produced in the milk of either goat #1 or #2 (lanes 2 and 3) did not bind the gal-alpha-1,3-gal monoclonal antibody. Thus, the goat-milk produced antibody does not include alpha-1,3-gal to the N-linked site. However, interestingly, mouse IgG (lane 9), as well as goat serum (Figure 9; lane 8) did show gal-alpha-1,3-gal.

The purified goat milk-produced cetuximab from both goat #1 and goat #2 was also analyzed by LC/MS and compared to commercial Erbitux®. As shown in Figure 13, alpha-1,3-gal was present on Erbitux®, but not present on the cetuximab produced in the milk of transgenic goats. Thus, the glycosylation site in the heavy chain variable region of cetuximab was not decorated with alpha-1,3-gal when produced in the mammary gland of a goat. In contrast, cetuximab produced commercially in mouse cells (*i.e.,* Erbitux®) does show alpha-1,3 gal glycosylation. Thus, production in the mammary gland does not include alpha-1,3-gal glycosylation, even to sites normally decorated in this manner in other systems, *e.g.,* in non-mammary mouse cells.

## Claims

1. A glycosylated antibody comprising:
(a) a heavy chain comprising SEQ ID NO:1; and
(b) a light chain comprising SEQ ID NO:2;
wherein the glycosylated antibody does not have detectable galactose-alpha- 1,3-galactose moieties, and wherein the glycosylated antibody is produced in mammary gland epithelial cells of a non-human transgenic mammal.

2. The glycosylated antibody of claim 1, wherein the glycosylated antibody lacks galactose-alpha- 1,3-galactose moieties.

3. The glycosylated antibody of claim 1 or 2, wherein the non-human transgenic mammal is a goat, sheep, bison, camel, cow, pig, rabbit, buffalo, horse, rat, mouse or llama, preferably wherein the non-human transgenic mammal is a goat.

4. The glycosylated antibody of any one of claims 1 to 3, wherein the antibody is chimeric or humanized antibody.

5. The glycosylated antibody of any one of claims 1 to 4, wherein the antibody is cetuximab.

6. A composition comprising the glycosylated antibody of any one of claims 1 to 5, and a pharmaceutically-acceptable carrier.

7. A method comprising:
producing a population of glycosylated antibodies in mammary gland epithelial cells such that the population of glycosylated antibodies produced does not have detectable galactose-alpha-1,3-galactose moieties, and
wherein the glycosylated antibodies comprise a heavy chain comprising SEQ ID NO: 1 and a light chain comprising SEQ ID NO:2.

8. The method of claim 7, the method further comprising purifying the population of glycosylated antibodies.

9. Mammary gland epithelial cells that produce the antibody of any one of claims 1 to 5, preferably wherein the mammary gland epithelial cells comprise a nucleic acid comprising SEQ ID NO:3 and a nucleic acid comprising SEQ ID NO:4.

10. A transgenic non-human mammal comprising the mammary gland epithelial cells of claim 9.

11. The antibody as defined in any one of claims 1 to 5 or the composition of claim 6, for use in treating cancer in a subject, optionally wherein the subject is administered with at least one additional therapeutic agent.

## Patentansprüche

1. Ein glykosylierter Antikörper, umfassend:
(a) eine schwere Kette, umfassend SEQ ID NO: 1; und
(b) eine leichte Kette, umfassend SEQ ID NO: 2;
wobei der glykosylierte Antikörper keine nachweisbaren Galaktose-alpha-1,3-Galaktose-Einheiten aufweist und wobei der glykosylierte Antikörper in den Brustdrüsenepithelzellen eines nicht-menschlichen transgenen Säugetiers produziert wird.

2. Der glykosylierte Antikörper nach Anspruch 1, wobei der glykosylierte Antikörper keine Galaktose-alpha-1,3-Galaktose-Einheiten aufweist.

3. Der glykosylierter Antikörper nach Anspruch 1 oder 2, wobei das nicht-menschliche Säugetier eine Ziege, ein Schaf, ein Bison, ein Kamel, eine Kuh, ein Schwein, ein Kaninchen, ein Büffel, ein Pferd, eine Ratte, eine Maus oder ein Lama ist, vorzugsweise wobei das nicht-menschliche transgene Säugetier eine Ziege ist.

4. Der glykosylierter Antikörper nach einem der Ansprüche 1 bis 3, wobei der Antikörper ein chimärer oder humanisierter Antikörper ist.

5. Der glykosylierter Antikörper nach einem der Ansprüche 1 bis 4, wobei der Antikörper Cetuximab ist.

6. Eine Zusammensetzung, umfassend den glykosylierten Antikörper nach einem der Ansprüche 1 bis 5 und einen pharmazeutisch verträglichen Träger.

7. Ein Verfahren, umfassend:
Herstellen einer Population von glykosylierten Antikörpern in Brustdrüsenepithelzellen, so dass die Population von produzierten glykosylierten Antikörpern keine nachweisbaren Galaktose-alpha-1,3-Galaktose-Einheiten aufweist, und wobei die glykosylierten Antikörper eine schwere Kette, umfassend SEQ ID NO: 1, und eine leichte Kette, umfassend SEQ ID NO: 2, umfassen.

8. Das Verfahren nach Anspruch 7, das Verfahren umfasst ferner das Reinigen der Population von glykosylierten Antikörpern.

9. Brustdrüsenepithelzellen, die den Antikörper nach einem der Ansprüche 1 bis 5 produzieren, vorzugsweise wobei die Brustdrüsenepithelzellen eine Nukleinsäure umfassen, die SEQ ID NO: 3 umfasst, und eine Nukleinsäure, die SEQ ID NO: 4 umfasst.

10. Ein transgenes nicht-menschliches Säugetier, umfassend die Brustdrüsenepithelzellen nach Anspruch 9.

11. Der Antikörper wie in einem der Ansprüche 1 bis 5 definiert oder die Zusammensetzung nach Anspruch 6 zur Verwendung bei der Behandlung von Krebs in einem Subjekt, optional wobei dem Subjekt mindestens ein zusätzlicher therapeutischer Wirkstoff verabreicht wird.

## Revendications

1. Anticorps glycosylé comprenant :
(a) une chaîne lourde comprenant la SEQ ID NO : 1 ; et
(b) une chaîne légère comprenant la SEQ ID NO : 2 ;
lequel anticorps glycosylé n'a pas de fragments galactose-alpha-1,3-galactose détectables, et lequel anticorps glycosylé est produit dans des cellules épithéliales de glande mammaire d'un mammifère transgénique non humain.

2. Anticorps glycosylé selon la revendication 1, lequel anticorps glycosylé est dépourvu de fragments galactose-alpha-1,3-galactose.

3. Anticorps glycosylé selon la revendication 1 ou 2, dans lequel le mammifère transgénique non humain est une chèvre, une brebis, une bisonne, une chamelle, une vache, une truie, une lapine, une bufflonne, une jument, une rate, une souris ou un lama, de préférence dans lequel le mammifère transgénique non humain est une chèvre.

4. Anticorps glycosylé selon l'une quelconque des revendications 1 à 3, lequel anticorps est un anticorps chimère ou humanisé.

5. Anticorps glycosylé selon l'une quelconque des revendications 1 à 4, dans lequel l'anticorps est le cétuximab.

6. Composition comprenant l'anticorps glycosylé de l'une quelconque des revendications 1 à 5 et un véhicule pharmaceutiquement acceptable.

7. Méthode comprenant :
la production d'une population d'anticorps glycosylés dans des cellules épithéliales de glande mammaire de façon que la population d'anticorps glycosylés produits n'ait pas de fragments galactose-alpha-1;3-galactose détectables, et
dans laquelle les anticorps glycosylés comprennent une chaîne lourde comprenant la SEQ ID NO : 1 et une chaîne légère comprenant la SEQ ID NO : 2.

8. Méthode selon la revendication 7, laquelle méthode comprend en outre la purification de la population d'anticorps glycosylés.

9. Cellules épithéliales de glande mammaire qui produisent l'anticorps de l'une quelconque des revendications 1 à 5, de préférence lesquelles cellules épithéliales de glande mammaire comprennent un acide nucléique comprenant la SEQ ID NO : 3 et un acide nucléique comprenant la SEQ ID NO : 4.

10. Mammifère non humain transgénique comprenant les cellules épithéliales de glande mammaire de la revendication 9.

11. Anticorps selon l'une quelconque des revendications 1 à 5 ou composition selon la revendication 6, pour une utilisation dans le traitement d'un cancer chez un sujet, éventuellement dans lequel il est administré au sujet au moins un agent thérapeutique additionnel.


### SEQUENCE LISTING

<110> Laboratoire Francais du Fractionnement et des
   Biotechnologies
   Meade, Harry M.
<120> Cetuximab with Modified Glycosylation and Uses Thereof
<130> L0719.70004WOO0
<150> 61/764446
   <151> 2013-02-13
<160> 7
<170> PatentIn version 3.5
<210> 1
   <211> 468
   <212> PRT
   <213> artificial sequence
<220>
   <223> synthetic polypeptide
<400> 1
   Met Ala Val Leu Gly Leu Leu Phe Cys Leu Val Thr Phe Pro Ser Cys
   1               5                   10                  15
   Val Leu Ser Gln Val Gln Leu Lys Gln Ser Gly Pro Gly Leu Val Gln
               20                  25                  30
   Pro Ser Gln Ser Leu Ser Ile Thr Cys Thr Val Ser Gly Phe Ser Leu
           35                  40                  45
   Thr Asn Tyr Gly Val His Trp Val Arg Gln Ser Pro Gly Lys Gly Leu
       50                  55                  60
   Glu Trp Leu Gly Val Ile Trp Ser Gly Gly Asn Thr Asp Tyr Asn Thr
   65                  70                  75                  80
   Pro Phe Thr Ser Arg Leu Ser Ile Asn Lys Asp Asn Ser Lys Ser Gln
                   85                  90                  95
   Val Phe Phe Lys Met Asn Ser Leu Gln Ser Asn Asp Thr Ala Ile Tyr
               100                 105                 110
   Tyr Cys Ala Arg Ala Leu Thr Tyr Tyr Asp Tyr Glu Phe Ala Tyr Trp
           115                 120                 125
   Gly Gln Gly Thr Leu Val Thr Val Ser Ala Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Arg Val Glu Pro Lys Ser
   225                 230                 235                 240
   Cys Asp Lys Thr His Thr Cys Pro Pro Cys Pro Ala Pro Glu Leu Leu
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Asn Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Glu Glu Met Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
   420 425 430
   Val Asp Lys Ser Arg Trp Gln Gln Gly Asn Val Phe Ser Cys Ser Val
   435 440 445
   Met His Glu Ala Leu His Asn His Tyr Thr Gln Lys Ser Leu Ser Leu
   450 455 460
   Ser Pro Gly Lys
   465
<210> 2
   <211> 234
   <212> PRT
   <213> artificial sequence
<220>
   <223> synthetic polypeptide
<400> 2
   Met Val Ser Thr Pro Gln Phe Leu Val Phe Leu Leu Phe Trp Ile Pro
   1 5 10 15
   Ala Ser Arg Gly Asp Ile Leu Leu Thr Gln Ser Pro Val Ile Leu Ser
   20 25 30
   Val Ser Pro Gly Glu Arg Val Ser Phe Ser Cys Arg Ala Ser Gln Ser
   35 40 45
   Ile Gly Thr Asn Ile His Trp Tyr Gln Gln Arg Thr Asn Gly Ser Pro
   50 55 60
   Arg Leu Leu Ile Lys Tyr Ala Ser Glu Ser Ile Ser Gly Ile Pro Ser
   65 70 75 80
   Arg Phe Ser Gly Ser Gly Ser Gly Thr Asp Phe Thr Leu Ser Ile Asn
   85 90 95
   Ser Val Glu Ser Glu Asp Ile Ala Asp Tyr Tyr Cys Gln Gln Asn Asn
   100 105 110
   Asn Trp Pro Thr Thr Phe Gly Ala Gly Thr Lys Leu Glu Leu Lys Arg
   115 120 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 3
   <211> 1428
   <212> DNA
   <213> artificial sequence
<220>
   <223> synthetic polynucleotide
<400> 3
   ctcgagaccg ccatggccgt gctgggcctg ctgttctgcc tggtgacctt ccccagctgc      60
   gtgctgtccc aggtgcagct gaagcagagc ggccctggcc tggtgcagcc ctctcagtcc     120
   ctctccatta cctgtaccgt gtccggcttc tctctcacaa attatggagt ccattgggtg     180
   cgccagagcc ccggcaaggg cctggagtgg ctgggcgtga tctggtccgg cggcaacacc     240
   gactacaaca cccccttcac cagcagactg agcatcaaca aggacaacag caagagccag      300
   gtgttcttca agatgaacag cctgcagagc aacgacaccg ccatctacta ctgcgctcgc     360
   gctctcacct actacgacta cgagttcgcc tactggggcc agggcaccct ggtgaccgtg     420
   tccgccgcca gcaccaaggg ccccagcgtg ttccccctgg cccccagcag caagagcacc     480
   agcggcggca ccgctgccct gggctgtctg gtgaaggact acttccccga gcccgtgacc     540
   gtgtcttgga actctggcgc cctgacctcc ggcgtgcaca catttccagc tgtcctccag     600
   agttccggac tgtattccct cagttctgtc gtgacagtcc cttcctctag cctgggcacc     660
   cagacctaca tctgcaacgt gaaccacaag cccagcaaca ccaaggtgga caagagagtg     720
   gagcccaaga gctgcgacaa gacccacacc tgcccaccat gtcctgctcc agagctgctg     780
   gggggaccct ccgtgttcct gttccccccc aagcccaagg acaccctgat gatcagcagg      840
   acccccgagg tgacctgcgt ggtggtggac gtgtcccacg aggaccctga ggtgaagttc     900
   aactggtacg tggacggcgt ggaggtgcac aacgccaaga ccaagcccag agaggagcag     960
   tacaacagca cctacagggt ggtgtccgtg ctgaccgtgc tgcaccagga ctggctgaac    1020
   ggcaaagaat acaagtgcaa agtctccaac aaggccctgc cagcccccat cgaaaagacc    1080
   atcagcaagg ccaagggcca gcctcgcgag ccccaggtgt acaccctgcc cccctcccgc    1140
   gaggagatga ccaagaacca ggtgtccctg acctgcctgg tgaagggctt ctaccccagc    1200
   gatatcgccg tggagtggga gagcaacggc cagcccgaga acaactacaa gaccaccccc    1260
   cctgtgctgg acagcgacgg cagcttcttc ctgtacagca agctgaccgt ggacaagtcc    1320
   aggtggcagc agggaaatgt cttttcttgt tctgtcatgc atgaagctct gcacaaccac    1380
   tacacccaga agtccctgag cctgtccccc ggcaagtgat agctcgag                 1428
<210> 4
   <211> 726
   <212> DNA
   <213> artificial sequence
<220>
   <223> synthetic polynucleotide
<400> 4
   ctcgagaccg ccatggtgtc caccccccag ttcctcgtgt tcctgctgtt ctggatcccc        60
   gccagcaggg gcgacatcct gctgacccag agccccgtga tcctgagcgt gtctcccggc       120
   gagagggtgt ccttcagctg cagagccagc cagagcatcg gcaccaacat ccactggtat       180
   cagcagagga ccaacggcag ccccaggctg ctgatcaagt acgccagcga gtccatcagc       240
   ggcatcccca gcaggttcag cggcagcggc tccggcaccg acttcaccct gagcatcaac       300
   agcgtggaga gcgaggatat cgccgactac tactgccagc agaacaacaa ctggcccacc       360
   accttcggcg ctggcaccaa gctggagctg aagaggaccg tggccgctcc cagcgtgttc       420
   atcttccccc ccagcgacga gcagctgaag tccggcaccg ccagcgtggt gtgcctgctg       480
   aacaacttct acccccgcga ggccaaggtg cagtggaagg tggacaacgc cctgcagagc       540
   ggcaacagcc aggagagcgt caccgagcag gacagcaagg actccaccta cagcctgagc       600
   agcaccctga ccctgagcaa ggccgactac gagaagcaca aggtgtacgc ctgcgaggtg       660
   acccaccagg gcctgtccag ccccgtgacc aagagcttca acaggggcga gtgctgatag       720
   ctcgag                                                                  726
<210> 5
   <211> 24342
   <212> DNA
   <213> artificial sequence
<220>
   <223> synthetic polynucleotide
<220>
   <221> misc_feature
   <222> (22615)..(22615)
   <223> n is a, c, g, or t
<220>
   <221> misc_feature
   <222> (23697)..(23697)
   <223> n is a, c, g, or t
<400> 5
   gtcgactcta gagggacagc ccccccccaa agcccccagg gatgtaatta cgtccctccc      60
   ccgctagggg cagcagcgag ccgcccgggg ctccgctccg gtccggcgct ccccccgcat     120
   ccccgagccg gcagcgtgcg gggacagccc gggcacgggg aaggtggcac gggatcgctt     180
   tcctctgaac gcttctcgct gctctttgag cctgcagaca cctgggggga tacggggaaa     240
   aagctttagg ctgaaagaga gatttagaat gacagaatca tagaacggcc tgggttgcaa     300
   aggagcacag tgctcatcca gatccaaccc cctgctatgt gcagggtcat caaccagcag     360
   cccaggctgc ccagagccac atccagcctg gccttgaatg cctgcaggga tggggcatcc     420
   acagcctcct tgggcaacct gttcagtgcg tcaccaccct ctgggggaaa aactgcctcc     480
   tcatatccaa cccaaacctc ccctgtctca gtgtaaagcc attccccctt gtcctatcaa     540
   gggggagttt gctgtgacat tgttggtctg gggtgacaca tgtttgccaa ttcagtgcat     600
   cacggagagg cagatcttgg ggataaggaa gtgcaggaca gcatggacgt gggacatgca     660
   ggtgttgagg gctctgggac actctccaag tcacagcgtt cagaacagcc ttaaggataa     720
   gaagatagga tagaaggaca aagagcaagt taaaacccag catggagagg agcacaaaaa     780
   ggccacagac actgctggtc cctgtgtctg agcctgcatg tttgatggtg tctggatgca     840
   agcagaaggg gtggaagagc ttgcctggag agatacagct gggtcagtag gactgggaca     900
   ggcagctgga gaattgccat gtagatgttc atacaatcgt caaatcatga aggctggaaa     960
   gcctccaaga tccccaagac caaccccaac ccacccaccg tgcccactgg ccatgtccct    1020
   cagtgccaca tccccacagt tcttcatcac ctccagggac ggtgaccccc ccacctccgt    1080
   gggcagctgt gccactgcag caccgctctt tggagaaggt aaatcttgct aaatccagcc    1140
   cgaccctccc ctggcacaac gtaaggccat tatctctcat ccaactccag gacggagtca    1200
   gtgaggatgg ggctctagag ggacagcccc cccccaaagc ccccagggat gtaattacgt    1260
   ccctcccccg ctaggggcag cagcgagccg cccggggctc cgctccggtc cggcgctccc    1320
   cccgcatccc cgagccggca gcgtgcgggg acagcccggg cacggggaag gtggcacggg    1380
   atcgctttcc tctgaacgct tctcgctgct ctttgagcct gcagacacct ggggggatac    1440
   ggggaaaaag ctttaggctg aaagagagat ttagaatgac agaatcatag aacggcctgg 1500
   gttgcaaagg agcacagtgc tcatccagat ccaaccccct gctatgtgca gggtcatcaa 1560
   ccagcagccc aggctgccca gagccacatc cagcctggcc ttgaatgcct gcagggatgg 1620
   ggcatccaca gcctccttgg gcaacctgtt cagtgcgtca ccaccctctg ggggaaaaac 1680
   tgcctcctca tatccaaccc aaacctcccc tgtctcagtg taaagccatt cccccttgtc 1740
   ctatcaaggg ggagtttgct gtgacattgt tggtctgggg tgacacatgt ttgccaattc 1800
   agtgcatcac ggagaggcag atcttgggga taaggaagtg caggacagca tggacgtggg 1860
   acatgcaggt gttgagggct ctgggacact ctccaagtca cagcgttcag aacagcctta 1920
   aggataagaa gataggatag aaggacaaag agcaagttaa aacccagcat ggagaggagc 1980
   acaaaaaggc cacagacact gctggtccct gtgtctgagc ctgcatgttt gatggtgtct 2040
   ggatgcaagc agaaggggtg gaagagcttg cctggagaga tacagctggg tcagtaggac 2100
   tgggacaggc agctggagaa ttgccatgta gatgttcata caatcgtcaa atcatgaagg 2160
   ctggaaagcc tccaagatcc ccaagaccaa ccccaaccca cccaccgtgc ccactggcca 2220
   tgtccctcag tgccacatcc ccacagttct tcatcacctc cagggacggt gaccccccca 2280
   cctccgtggg cagctgtgcc actgcagcac cgctctttgg agaaggtaaa tcttgctaaa 2340
   tccagcccga ccctcccctg gcacaacgta aggccattat ctctcatcca actccaggaa 2400
   cggagtcagt gaggatgggg ctctagagga tccctcgacc tgcaggtcaa cggatcacaa 2460
   caaactggaa aattcttcaa gagaagaata ccagaccacc ctacctgctt cctgagaaat 2520
   ctgtttgctg ctcagaagca acagttagaa ccagacatgg aacaacagac tggttccaaa 2580
   tcaggaaagg agtatgtcaa ggctgtatat cgtcaccctg attatttaac ttatatgcat 2640
   agtacataat acaaaatgcc aggctggatg aatcgcaagc tggaatcaag atttctggga 2700
   gaaatatcaa taaacgagat acaaagatac accacactta tggcagaaaa ctaagaagaa 2760
   ctaaagagcc tcttgatgaa agtgaaagag gagagtgaaa aagccagctt aaaacccaac 2820
   attcaaaatc aagatcatca tttcatggca aataaatggg gaaacaatgg aaacagtgag 2880
   agactttatt ttcttgggct ccaaaatcac tgcagattgt gactacagcc atgattaaaa 2940
   gatgcttgct ccttggaaga gaagctatta ccaaactaga aagcatatta aaaagcagag 3000
   acgttacttt gctgactaag ttctgtctag tcaaacctat ggtttttcca gtagtcatat 3060
   atggatgtga gttgaactat aaagaaagct gagcaccaaa gaattgatgc ttttgaaatt 3120
   tggtgttgga gaagtctctt gagagtccct tgaacctgca aggagatcca accagtccat 3180
   cctaaaggaa atcagtcctg aatattcatt ggaaggactg atgctgaaat tgaagattaa 3240
   cgttttggac tcacctaatg cagaagagcc aactcactag aaaagacccc atgttggcaa 3300
   aaattgaagc caggaagaga agtgaatgac agaggatgag atggttggat ggcatcgttg 3360
   actgaatgga catgagtctg atcaagttcc gggagacagc aaaggacagg gctgcctggt 3420
   ctgctgcagt ccatggggtt gcaaagagtc ggtctcaaat gagtaactaa acaacaacca 3480
   agcagtagaa aaataaataa aatttgtctc tgagatctca gtacctcttt ctgtgcatat 3540
   ccgtctcctg ttattgtact ttgtcttctg cttgtaataa agctgtcctg ttagtaaaat 3600
   ctgtttgggt cctctgaatt cttttagcta tcaaaaatgg aaggtgatta ttgtgcaatg 3660
   tccacctctg agtaatatac agagaataaa agaagggaga aattatgtgc aagttctctc 3720
   tcatctcctg cttctcattt aaaagattct acctcagtgg gggctaaaac tccacattta 3780
   acagtagcaa aaaccaatat tccatagctt cttaggaaac cattttttat actcttgtat 3840
   gtaattacat tcaagctcaa aagcaaagaa gtgattctgc gttggtgaag gcccaaccat 3900
   agaaaagagg aagaaaatag gccacatact gtgcttcccc catagctcag ttggtaaaga 3960
   atctacctac aatgcaggag gcctgggctt gatccctggg taagggagat cccctggaga 4020
   aggaaatggt aacccactcc agtactcttg cctgtaaatc ccatggacgg aggagcctgg 4080
   cagctacagc cttggggtgg caagagttgg acatgattaa caactaaacc actgccacca 4140
   ctccacatac tgagtgctcc ccagtggcac tagtggtaaa gaaccacctg ccggtgcaga 4200
   agacattaaa gacactggct ctatccctgc ttgggaagta gggaagatcc cctagagagg 4260
   gaaatagcaa cccactccag aattcttgcc tggaaaatcc catgaatgaa gactggcggg 4320
   ctgtagtaac tggggtcaca aagagttaaa catgatttag caactaaaca tcaccacatt 4380
   aaaaaaatta ccaccaaaat agtcatattc caggctaagg ggaataatag cactagtacc 4440
   tgagagaact ttctcagatt ctctgtcaag ttcttccttc tctcatataa ccagtagtct 4500
   agtttacctc atcagatatt aactactcat cgattctaaa ttatctaatt atgggggggg 4560
   gcactacatt gcattatatt ttgtgtccat tgactatcac tcaatttatt tataaaaaat 4620
   tcatccatgt tgtttctgtg acagtaactc attcacatta attgtaatat ctcattgcat 4680
   tgtatactac aatttattta tacaaaatac tattattcac acttctgttg attttaattt 4740
   ggaacatcaa caataacgtg gctgagaagc ttctttcttt agtatattgt taaggatttc 4800
   cttgatcaag attttaccta cttttctggt ccaattggtg agagacagtc ataaggaaat 4860
   gctgtgttta ttgcacaata tgtaaagcat cttcctgaga aaataaaagg gaaatgttga 4920
   atgggaagga tatgctttct tttgtattcc ttttctgaga aatcagactt tttcaccttg 4980
   gccttggcca ccaaaagcta acaaataaag gcatatgaag tagccaaggc cttttctagt 5040
   tatatctatg acactgagtt catttcatca tttattttcc tgacttcctc ctgggtccat 5100
   atgagcagtc ttagaatgaa tattagctga ataatccaaa tacatagtag atgttgattt 5160
   gggttttcta agcaatccaa gacttgtatg acagtaagat gtattaccat ccaacacaca 5220
   tctcagcatg atataaatgc aaggtatatt gtgaagaaaa atttttaatt atgtcaaagt 5280
   gcttacttta gaaggtcatc tatctgtccc aaagctgtga atatatatat tgaaggtaat 5340
   gaatagatga agctaacctt gtaaaaatga gtagtgtgaa atacaactac aattatgaac 5400
   atctgtcact aaagaggcaa agaaacttga agattgcttt tgcaaatggg ctcctattaa 5460
   taaaaagtac ttttgaggtc tggctcagac tctattgtag tacttagggt aagaccctcc 5520
   tcctgtatgg gctttcattt tctttcttgc ttccctcatt tgcccttcca tgaatactag 5580
   ctgataaaca ttgactataa aagatatgag gccaaacttg agctgtccca ttttaataaa 5640
   tctgtataaa taatatttgt tctacaaaag tattatctaa ataaatgtta ctttctgtct 5700
   taaaatccct caacaaatcc ccactatcta gagaataaga ttgacattcc ctggaatcac 5760
   agcatgcttt gtctgccatt atctgacccc tttctctttc tctcttctca cctccatcta 5820
   ctcctttttc cttgcaattc atgacccaga ttcactgttt gatttggctt gcatgtgtgt 5880
   gtgctgagtt gcgtctgact gttatcaacc ccatgaatga tagtccacca ggctctactg 5940
   tccatgaaat tttccagtca agaatactgg agtggattgc atttcctact ccatttgatt 6000
   aatttagtga cttttaaatt tctttttcca tattcgggag cctattcttc ctttttagtc 6060
   tatactctct tcactcttca ggtctaaggt atcatcgtgt gcttgttagc ttgttacttt 6120
   ctccattata gcttaagcac taacaactgt tcaggttggc atgaaattgt gttctttgtg 6180
   tggcctgtat atttctgttg tgtattagaa tttaccccaa gatctcaaag acccactgaa 6240
   tactaaagag acctcattgt ggttacaata atttggggac tgggccaaaa cttccgtgca 6300
   tcccagccaa gatctgtagc tactggacaa tttcatttcc tttatcagat tgtgagttat 6360
   tcctgttaaa atgctcccca gaatttctgg ggacagaaaa ataggaagaa ttcaaatcca 6420
   ctgttggttt tatttcaaac cacaaaatta gcatgccatt aaatactata tataaacagc 6480
   cactaaatca gatcattatc cattcagctt ctccttcact tcttctcctc tactttggaa 6540
   aaaaggtaag aatctcagat ataatttcag tgtatctgct actcatcttt attttggact 6600
   aggttaaaat gtagaaagaa cataattgct taaaatagat cttaaaaata agggtgttta 6660
   agataaggtt tacactattt tcagcagata tgttaaaaaa tagaagtgac tataaatact 6720
   tgataaaaat tatagtgact gcaaatgttt taggaatata ataagatata ataacagtgg 6780
   ttgctatttt ctttagcaca agactagtta acaggctgta ttaaaagatc ttttcttgaa 6840
   ttaaatattt tcaatttgat taaacctacc tcagccataa aggcaagcac atttcattta 6900
   tactatgggg atttgaataa ttattactga agaagctcta ccaacaaaaa gtttatagag 6960
   ctatcatatt tagtcaagag ataaagaggg ttgttaggat atatatgcta tttgaaaggt 7020
   atttataaaa gaagagtata tttatcaaaa tttctcaaga acatccaaat ttcaagttta 7080
   tcatttatct tacaatattt caaaaatatt aaaatagata catgaaatac agaagtaaat 7140
   taaagagaaa gtattttact tggtaaaaaa attctaggtt ggacagagag tgccaggaaa 7200
   caaaaacaat gaaaaatgtg acctgacagg aattatagct caaagtatag tagtaagtaa 7260
   tgaaatggct taaaaattgg tatataaaat gctagttata aaataaacaa aatgcaataa 7320
   tatcctccct acatgtaatg aattctaggt attatgatta tgctcttttt tgaagtcttg 7380
   acaataaaaa tttttttaga agtttatagg catcttgaat aaagtgaaac aaattaagaa 7440
   ttagtatcca tgagaaaaat atagaacaat tttcctaatt tagtttgaaa atctgggatt 7500
   gaagatgtgt gtcaagagat gttggtggca agaacatttt tttttcaaga acttataaaa 7560
   atgcaacaaa acaaaccatt taatacattt tggtcaaaat caataatgta ttttatttta 7620
   tgctccaagg agcataaaat tggggactgg gcaagagaaa ctgacaccct ggtaaattac 7680
   caagagataa gtacacagtt actatagtag aaaataagca tagtgtatga tctctaaaat 7740
   tatgtgagac aaaggagaga tgacattagg catgtgggga tgaagactga gtagagaaga 7800
   aacaatctaa tcagtccaag aaaacatctc gatcagtgga acaaatagaa gaaatgctaa 7860
   aatgaaacag aagtcttact ggaaataaaa gatatgcata agacaaaaat tcatgaaaat 7920
   cacttagttt agcagagaaa agataaaaat aaagtatgac cttcttcata tacattgttt 7980
   gatcatatgc acctcaataa aactgagtct ccaacagaaa tgaaacatta atattttgtt 8040
   cactgctcta atcccagaat ctaagcgata tctggcaata aaaataataa atatatattt 8100
   tttaataaat gaatcaacca cttaattttt ctgtaaatat ctgtaacttc tcttctgtct 8160
   ttccaaaaac actcataagt actgtgaatg agatgaaaaa gagtgaagta ggatataggc 8220
   tgttagcaga aaacatctga atggctggca gtgaaacatt aacttgaaat gtaagattaa 8280
   tgagtaatag taaattttaa ccttggccat atgataaaat gttcattaat atttttctag 8340
   aatacagggc tttttgtttt tgccatgagg tttgcaggat cttggttccc tgaccaggga 8400
   tcaaacctgc acaccaggga tcaaacctgc actcccctgg aagcatggag tcttggacat 8460
   ttgtattata cactatcttt ggttcctttt aaagggaagt aattttactt aaataagaaa 8520
   atagattgac aagtaatacg ctgtttcctc atcttcccat tcacaggaat cgcggatcct 8580
   cgagaccgcc atggtgtcca ccccccagtt cctcgtgttc ctgctgttct ggatccccgc 8640
   cagcaggggc gacatcctgc tgacccagag ccccgtgatc ctgagcgtgt ctcccggcga 8700
   gagggtgtcc ttcagctgca gagccagcca gagcatcggc accaacatcc actggtatca 8760
   gcagaggacc aacggcagcc ccaggctgct gatcaagtac gccagcgagt ccatcagcgg 8820
   catccccagc aggttcagcg gcagcggctc cggcaccgac ttcaccctga gcatcaacag 8880
   cgtggagagc gaggatatcg ccgactacta ctgccagcag aacaacaact ggcccaccac 8940
   cttcggcgct ggcaccaagc tggagctgaa gaggaccgtg gccgctccca gcgtgttcat 9000
   cttccccccc agcgacgagc agctgaagtc cggcaccgcc agcgtggtgt gcctgctgaa 9060
   caacttctac ccccgcgagg ccaaggtgca gtggaaggtg gacaacgccc tgcagagcgg 9120
   caacagccag gagagcgtca ccgagcagga cagcaaggac tccacctaca gcctgagcag 9180
   caccctgacc ctgagcaagg ccgactacga gaagcacaag gtgtacgcct gcgaggtgac 9240
   ccaccagggc ctgtccagcc ccgtgaccaa gagcttcaac aggggcgagt gctgatagct 9300
   cgaggatccg gacccttccc tattcttgta agtctaaatt tactaactgt gctgtttaac 9360
   ttctgatgtt tgtatgatat ttgagtaatt aagagcccta caaaaaaatc aataatgaat 9420
   ggttccaaaa taagcatagc tgagattaat gattctcagc attagttata aatagaataa 9480
   gctggaaaac cttcacctcc cctccaccac cagatctcaa tgtctaggct tacccatgga 9540
   gattctgatt aactgttctt tctatgtaga agaaacttat tgggaagaaa taatataatg 9600
   gactatgatt taattggtct gttgagaatt tagatgaagg ggattaagtt acaataaagc 9660
   cagaatttaa cttgataatc tcatttggct aagaataaca aacctaagaa ggtttgctat 9720
   tttctacaat tttgaagttt tccttatgca caattatttc accacatgac tcatttcaca 9780
   tcttgttttt gatatatgag catatgaggg caaaatactg aagatgctta tttcaatact 9840
   cagggaaaat tttcttgcca aaaggcaaga attgtataat tcattcactt attttatttt 9900
   ttttaatttt taaggtctaa gaggatttca aagtgaatgc cccctcctca cttttggtaa 9960
   gctttaggag attggaggca gactgatcat ttttatagtt aatatctttt acatttcatc 10020
   ttcctggata agccccaata gtagcaattt ctatcagtat accagcataa agattagttt 10080
   taaatttatt ttcagtgatt gactgttatt tactgacctg aaattatgta tctgttatat 10140
   ttcaaataat gcaaaactgt atatatatgg tgttgacaga tttgattggt tttctttcaa 10200
   ttgcctatat ccttattatt gattgtaatc atttatagaa aaaacaaaat aatttcttat 10260
   acttttatgt aaacctgtta gagcttattt taaagatcaa ctgcattcac atttctaatc 10320
   tagtcattat gagcttcaat tgttttatct cacttaaaat ttatatattg tcttttaatt 10380
   catgagtcaa aatacaatct cacagtccag atatgggact taaaagggga atagcatata 10440
   gttttgatat tcttaaagat atacatcttt ttgtgatcat gattcagcag acattttaat 10500
   aaaacaattc caagtgagcc gacacttggt cctagaggaa tttttataac cttaagataa 10560
   ggcacagcat ggtgtttttg taataagatt tcttttatga aaaagtcaca ccaaaattgg 10620
   aaatggggtg agatgaagag ttataacata taactaaatg gacatttgtt ctctattcca 10680
   cagaattgac tgcgactgga aatatggcaa cttttcaatc cttgcatcat gctactaaga 10740
   taatttttaa atgagtatac atggaacaaa aaatgaaact ttattccttt atttatatta 10800
   tgctttttca tcttaatttg aatttgagtc ataaaccata tactttcaaa atgttaattc 10860
   aacattagca taaaagttca attttaactt ggaaatatca tgaacatatc aaattatgta 10920
   taaaaataat ttctggaatt gtgattatta tttctttaag aatctatttc ctaaccagtc 10980
   atttcaataa attaaccctt aggcatattt aagttttctt gtctttatta tatttttaaa 11040
   aatgaaattg gtctctttat tgttaactta aatttatctt tgatgttaaa aatagctgtg 11100
   gaaaattaaa attgaataga attctttgaa ttgagttcca aaggatatca aaaagtgagg 11160
   gaaaagatag ggtgagccta tgctgcatat gtccttagaa agtcttggtt tatacctgtt 11220
   acctaagtta aacaattata cttgttcctt tcactctcga aagtacccag cattggatgt 11280
   taaattttat agtcatccta gacaaaaaaa aaaaaaaaaa caaacaaccc tcaaatgtga 11340
   tatctgaatc acagctctac agtgtggtag ctaagtggtg ctgtgtaagt tagtctccaa 11400
   gagattccat ttctacattt ataaacagtc aatttaaggt gttttattga agttttaatg 11460
   tgaaaagtgc actatatggt gcatgatagg agttcctggt tgaatctcat ttctgacatc 11520
   actgacacca gtgcagcaag gactagtgtt acaatcagaa ggagctgagt tgtgtaattt 11580
   tagccattaa tgcccaagag actagaactt acacaaagct ctaatatcca ttgtctctgt 11640
   ctgtggagta attatttcat tgccatgaat tatctgtctg tcatatcctg catttttata 11700
   catgattcag ttcccttcag ttcacacaat gacttgtcta atttcatctt tcctgcatcc 11760
   tccatgtttt cctcacttca ggattaagtg aagccgtact taggcacaat atttcttatc 11820
   tttaaagaaa aattccatct ttgagagttg ttattgttca gtcactaggt catgtccaac 11880
   tctttgtgac cccatgcact gcagcatgcc aggcttccct gcccttcgct ctctcctgga 11940
   gtttgctcag actcatgtag attgagtcgg tgatggtatc caactatctc atcaactgtt 12000
   gtgcccttct cctcctaccc tcagtcttta ccagcatcag agtctttctc agattcttca 12060
   ggttattata taacaactat cataaaagga gtatctaaat ggctgtgtcc attatttcac 12120
   atgttattct ctctttaact tgctccaatc ccaattttat ccctatggga actgctttat 12180
   tgaagatcac caacaacttt tattttacta atcgttttgt tttacccaac ctctcagtga 12240
   gtgttatgag gtagagttga ctatttcttc attttgaaat attacgcttc atttcatttg 12300
   atatcctaaa gctcataagg tgtggttttt ctcttaactc actagacact tctttgaagt 12360
   ctctcttctg gcattttctc cttttccaaa attttaatgg ttggagtacc ctagatttta 12420
   gccttaattt gtttgatgtt gttcagttcc attctcagct cagagcttcc aactgtatgt 12480
   ctccaaactt actcgttttg taaactccaa actcatgcac tcaactgcat tcttgacctc 12540
   cacactgaat tatctaatta atgtcctaaa tctggcatga ccaagcatac atttttgtct 12600
   gaatccagtc cccaacttgc tcaaaattta attaaacgta attcagttac aaaggcagct 12660
   gatattgtat gcaatagacc tgaatgggaa cttcacaaaa gaagttatct taattgtcaa 12720
   taaaaacatg aaaaatactc tacatcatca atcttcagaa aaatgcaaat taaaggtgcc 12780
   taataatatc atgacacaac cgtcagaatg actgaaatga aaagaattgt aatacagttc 12840
   agttcagttc agttactcag tcgtctccaa ctctttgtga ccccatgaac tgcagcatga 12900
   cagaccttcc tgtccatcac caactcccag agtttactca gactatgtcc attgagttga 12960
   tgatgccatc caaccatctc atcctctgtc gtccccttct cctcctgccc tcagtctttc 13020
   ccagcatcag ggtcttttcc aatgagtcag ctcttcgcat caggtggcta aagtattgga 13080
   gtttcagctt caacatcagt ccttctaatt aacacccagg actgatctct tttaggatgg 13140
   actagttgga tctccttgca gtccaaggga ctctcaagag tcttctccaa caccacagtt 13200
   caaaagcatc aattccttgg cactcagctt tccttatagt ccatgtctca catccacaca 13260
   tgactattgg aaaaaccata gccttgacta ggtggacctt tgttgacaaa gtaatgtctc 13320
   tgctttttaa tatgttgtct agattggtca taactttcct tccaagaagt aattgtcttt 13380
   taatttcatg gctgcagtca ccatctgcag tgattttgga gccccaaaat ataaagtcag 13440
   ctgctgtttc cactgttgcc ccatctaccc catctatttg ccatgaagtg atgggactgg 13500
   atgccactat cttagttttc tgaatgttga gctttaagcc agccttttta ctctcctctt 13560
   tcactttcat caagaggctc tttagttcct cttcactttc tgccataagg gtggtgtcat 13620
   ctgcatatct gaggttattg atatttctct tggcaatttt gattccagcc tgcacttctt 13680
   ccagcccagt gtttctcatg atgtactctg catataaatt aaataagcag agtgacaata 13740
   tacagccttg acatactctt tttcctattt ggaaccagtc tgttgttcca tgtccagttc 13800
   taactgttgt ttcctgacct gcatacaggt ttctcaagag gcaagtcagg tggtctggta 13860
   ttctcacctg tttcagaatt ttccacagtt tattgtgatc cacacagtca aaggctttgg 13920
   catagccaat aaagcagaaa gagatgtttt tctggaactc tcttactttt ttgatgatcc 13980
   agtggatgtt ggcaatttga tctctggttc ctctgccttt tctaaaacca gctttaacat 14040
   ctggaagttc atggttcacg taatacaaaa tgtaatacaa aatgtctgca aaaacaaagg 14100
   aatgaaaagt aatgctaaaa aatgttaata tttacagaaa tttttatagt agtaaagaat 14160
   tcacctgcaa tacaggagaa ccgggttaga tccctgggtt ggaagacctc ctggagaagg 14220
   aaatggctac ccaatctagt attcttgtct ggagaaggca agaatggaca gagaagccca 14280
   gcgggctatg gtccatcggg tcacaaagag tcagaagcta ccttgcacac agcaagcacg 14340
   gtgcgcgcgc gtgcacacac acacacacac acacacacag acacacacac actctaaaac 14400
   atttacccaa gcttgtccaa tggaaaatca aaaagccagc aatttaagat gacatcaggt 14460
   accactgtcc aggtaagcct cagaacacaa tgaccagtaa gaagcaaagt gccatatgag 14520
   caactcgaat ttttgcaatg ttacctaaga gcttccattt ttataatgca aaagaatttc 14580
   atatggggaa attgtattag ataaccctga atgaggagca agatatagtc aaagtaagat 14640
   gctctagtac tattttttat aagcatgatt tgttcagcca aaggtttttt cccatatggc 14700
   caatgaactg aaatatgcag tcctgagatt tgcatatatt tctagctgaa accaagtaaa 14760
   taatatcctc aagaaagaaa tcaatagaaa agttggatga agagtacaat aaagggacca 14820
   aaaatattca gaaataagaa ctagaggaga tattgggaaa tccctggtga gtccagttta 14880
   ggattttgta ctttcactgc agttggcatg gatataatcc ctcactgggg aactaagatc 14940
   ccataagctg tgttggattg ccaaaaaaat aaatattaag agatatcatt catagaatat 15000
   tttaaagata ttttagagaa gaggaaatta aggatgtgag aatttgtatt actttttcaa 15060
   gatactaaag ctatttagag atagagctgt tactaaaaac ttcagtttcc taaaaattat 15120
   ttgaagcact gtttaataaa ttccaaaata tagaggaagg aaaacaaaat actgaggatt 15180
   catataatga ttcagattta gaaacaatat aacacagaat tagtgaattc tgacaaatta 15240
   ttaggtagga gtagatagtt cagcattact cgtatagatg gagtatttaa tcctttccat 15300
   gagattatcc aaatataata atttcgtatc tatgtgaagt ataactatta agattacttt 15360
   ataaagtaaa tcaagaacca gagaataaga aaaatgtttt gtgaaccagc agatactatg 15420
   aacacataaa actcagaacc ctgattccta agacacacag ctaatcctga ttattcttcc 15480
   tttacatgtg accatagaac ttcacacaag ttcaagatac atttgttgag cacatcagta 15540
   tcagttcagt cactcagtca tgtccgaatc tttgtgacct tgtggactgc agcacgccag 15600
   gctttcctgt ccaccaccaa cccctggagc ttactcaaac tcatgtccat tgagtcagtg 15660
   atcccatcca accatctcat cctctgtcat cctcttctcc tgccttcaat ctttcccaga 15720
   cattggagtc ttttccaatg agtcagatct tcacattagg tggccaaagt ataggagttt 15780
   cagcttcagc atcaatcctt ccaatgaata ttccttgatg tacccctttc gcagtttgga 15840
   accagtctgt tgttccatgt ccagttctaa ctgctgcttc tggacctgta tacagatttc 15900
   tcaggaggca ggtaaagtgg tctggtattc ccatctcttg aagaattttc cacagtttat 15960
   tgtgatccac acaatcaaag gctttagcgt agtcaataaa gcagatgttt ttctggaact 16020
   ctcgtgcttt tttgatgatc caatggatgt tggcaatttg atctctggtt cctctgcctt 16080
   ttctaaatcc agcttgaaca tctggaagtt catggtccac gtactgttga agcctggctt 16140
   ggagaatttt gagagttatt ttgctagcat gtgagatgag tgcaatcatg tgggtgtttg 16200
   aacatacttt gtcattgctt ttctttggga ttgtggcagt cctgtggcca ctgctgagtt 16260
   ttccaaattt gctgacatat tgagtgcagc actttcacag catcaccttt taagatttga 16320
   aatagctcaa ctggaattcc atcacctcca ctagctttgt tcatagtgag gctttctaag 16380
   gccgtttgac tttgcattcc agggtgtctg gctctaggtg agtgatccgt tgacctgcag 16440
   cggccgagtc gactcggccg cgaattcttg aagacgaaag ggcctcgtga tacgcctatt 16500
   tttataggtt aatgtcatga taataatggt ttcttagacg tcaggtggca cttttcgggg 16560
   aaatgtgcgc ggaaccccta tttgtttatt tttctaaata cattcaaata tgtatccgct 16620
   catgagacaa taaccctgat aaatgcttca ataatattga aaaaggaaga gtatgagtat 16680
   tcaacatttc cgtgtcgccc ttattccctt ttttgcggca ttttgccttc ctgtttttgc 16740
   tcacccagaa acgctggtga aagtaaaaga tgctgaagat cagttgggtg cacgagtggg 16800
   ttacatcgaa ctggatctca acagcggtaa gatccttgag agttttcgcc ccgaagaacg 16860
   ttttccaatg atgagcactt ttaaagttct gctatgtggc gcggtattat cccgtgttga 16920
   cgccgggcaa gagcaactcg gtcgccgcat acactattct cagaatgact tggttgagta 16980
   ctcaccagtc acagaaaagc atcttacgga tggcatgaca gtaagagaat tatgcagtgc 17040
   tgccataacc atgagtgata acactgcggc caacttactt ctgacaacga tcggaggacc 17100
   gaaggagcta accgcttttt tgcacaacat gggggatcat gtaactcgcc ttgatcgttg 17160
   ggaaccggag ctgaatgaag ccataccaaa cgacgagcgt gacaccacga tgcctgcagc 17220
   aatggcaaca acgttgcgca aactattaac tggcgaacta cttactctag cttcccggca 17280
   acaattaata gactggatgg aggcggataa agttgcagga ccacttctgc gctcggccct 17340
   tccggctggc tggtttattg ctgataaatc tggagccggt gagcgtgggt ctcgcggtat 17400
   cattgcagca ctggggccag atggtaagcc ctcccgtatc gtagttatct acacgacggg 17460
   gagtcaggca actatggatg aacgaaatag acagatcgct gagataggtg cctcactgat 17520
   taagcattgg taactgtcag accaagttta ctcatatata ctttagattg atttaaaact 17580
   tcatttttaa tttaaaagga tctaggtgaa gatccttttt gataatctca tgaccaaaat 17640
   cccttaacgt gagttttcgt tccactgagc gtcagacccc gtagaaaaga tcaaaggatc 17700
   ttcttgagat cctttttttc tgcgcgtaat ctgctgcttg caaacaaaaa aaccaccgct 17760
   accagcggtg gtttgtttgc cggatcaaga gctaccaact ctttttccga aggtaactgg 17820
   cttcagcaga gcgcagatac caaatactgt ccttctagtg tagccgtagt taggccacca 17880
   cttcaagaac tctgtagcac cgcctacata cctcgctctg ctaatcctgt taccagtggc 17940
   tgctgccagt ggcgataagt cgtgtcttac cgggttggac tcaagacgat agttaccgga 18000
   taaggcgcag cggtcgggct gaacgggggg ttcgtgcaca cagcccagct tggagcgaac 18060
   gacctacacc gaactgagat acctacagcg tgagctatga gaaagcgcca cgcttcccga 18120
   agggagaaag gcggacaggt atccggtaag cggcagggtc ggaacaggag agcgcacgag 18180
   ggagcttcca gggggaaacg cctggtatct ttatagtcct gtcgggtttc gccacctctg 18240
   acttgagcgt cgatttttgt gatgctcgtc aggggggcgg agcctatgga aaaacgccag 18300
   caacgcggcc tttttacggt tcctggcctt ttgctggcct tttgctggcc ttttgctcac 18360
   atgttctttc ctgcgttatc ccctgattct gtggataacc gtattaccgc ctttgagtga 18420
   gctgataccg ctcgccgcag ccgaacgacc gagcgcagcg agtcagtgag cgaggaagcg 18480
   gaagagcgct gacttccgcg tttccagact ttacgaaaca cggaaaccga agaccattca 18540
   tgttgttgct caggtcgcag acgttttgca gcagcagtcg cttcacgttc gctcgcgtat 18600
   cggtgattca ttctgctaac cagtaaggca accccgccag cctagccggg tcctcaacga 18660
   caggagcacg atcatgcgca cccgtcagat ccagacatga taagatacat tgatgagttt 18720
   ggacaaacca caactagaat gcagtgaaaa aaatgcttta tttgtgaaat ttgtgatgct 18780
   attgctttat ttgtaaccat tataagctgc aataaacaag ttaacaacaa caattgcatt 18840
   cattttatgt ttcaggttca gggggaggtg tgggaggttt tttaaagcaa gtaaaacctc 18900
   tacaaatgtg gtatggctga ttatgatctc tagtcaaggc actatacatc aaatattcct 18960
   tattaacccc tttacaaatt aaaaagctaa aggtacacaa tttttgagca tagttattaa 19020
   tagcagacac tctatgcctg tgtggagtaa gaaaaaacag tatgttatga ttataactgt 19080
   tatgcctact tataaaggtt acagaatatt tttccataat tttcttgtat agcagtgcag 19140
   ctttttcctt tgtggtgtaa atagcaaagc aagcaagagt tctattacta aacacagcat 19200
   gactcaaaaa acttagcaat tctgaaggaa agtccttggg gtcttctacc tttctcttct 19260
   tttttggagg agtagaatgt tgagagtcag cagtagcctc atcatcacta gatggcattt 19320
   cttctgagca aaacaggttt tcctcattaa aggcattcca ccactgctcc cattcatcag 19380
   ttccataggt tggaatctaa aatacacaaa caattagaat cagtagttta acacattata 19440
   cacttaaaaa ttttatattt accttagagc tttaaatctc tgtaggtagt ttgtccaatt 19500
   atgtcacacc acagaagtaa ggttccttca caaagatccg gaccaaagcg gccatcgtgc 19560
   ctccccactc ctgcagttcg ggggcatgga tgcgcggata gccgctgctg gtttcctgga 19620
   tgccgacgga tttgcactgc cggtagaact ccgcgaggtc gtccagcctc aggcagcagc 19680
   tgaaccaact cgcgagggga tcgagcccgg ggtgggcgaa gaactccagc atgagatccc 19740
   cgcgctggag gatcatccag ccggcgtccc ggaaaacgat tccgaagccc aacctttcat 19800
   agaaggcggc ggtggaatcg aaatctcgtg atggcaggtt gggcgtcgct tggtcggtca 19860
   tttcgaaccc cagagtcccg ctcagaagaa ctcgtcaaga aggcgataga aggcgatgcg 19920
   ctgcgaatcg ggagcggcga taccgtaaag cacgaggaag cggtcagccc attcgccgcc 19980
   aagctcttca gcaatatcac gggtagccaa cgctatgtcc tgatagcggt ccgccacacc 20040
   cagccggcca cagtcgatga atccagaaaa gcggccattt tccaccatga tattcggcaa 20100
   gcaggcatcg ccatgggtca cgacgagatc ctcgccgtcg ggcatgcgcg ccttgagcct 20160
   ggcgaacagt tcggctggcg cgagcccctg atgctcttcg tccagatcat cctgatcgac 20220
   aagaccggct tccatccgag tacgtgctcg ctcgatgcga tgtttcgctt ggtggtcgaa 20280
   tgggcaggta gccggatcaa gcgtatgcag ccgccgcatt gcatcagcca tgatggatac 20340
   tttctcggca ggagcaaggt gagatgacag gagatcctgc cccggcactt cgcccaatag 20400
   cagccagtcc cttcccgctt cagtgacaac gtcgagcaca gctgcgcaag gaacgcccgt 20460
   cgtggccagc cacgatagcc gcgctgcctc gtcctgcagt tcattcaggg caccggacag 20520
   gtcggtcttg acaaaaagaa ccgggcgccc ctgcgctgac agccggaaca cggcggcatc 20580
   agagcagccg attgtctgtt gtgcccagtc atagccgaat agcctctcca cccaagcggc 20640
   cggagaacct gcgtgcaatc catcttgttc aatcatgcga aacgatcctc atcctgtctc 20700
   ttgatcagat cttgatcccc tgcgccatca gatccttggc ggcaagaaag ccatccagtt 20760
   tactttgcag ggcttcccaa ccttaccaga gggcgcccca gctggcaatt ccggttcgct 20820
   tgctgtccat aaaaccgccc agtctagcta tcgccatgta agcccactgc aagctacctg 20880
   ctttctcttt gcgcttgcgt tttcccttgt ccagatagcc cagtagctga cattcatccg 20940
   gggtcagcac cgtttctgcg gactggcttt ctacgtgttc cgcttccttt agcagccctt 21000
   gcgccctgag tgcttgcggc agcgtgaagc tttttgcaaa agcctaggcc tccaaaaaag 21060
   cctcctcact acttctggaa tagctcagag gccgaggcgg cctcggcctc tgcataaata 21120
   aaaaaaatta gtcagccatg gggcggagaa tgggcggaac tgggcggagt taggggcggg 21180
   atgggcggag ttaggggcgg gactatggtt gctgactaat tgagatgcat gctttgcata 21240
   cttctgcctg ctggggagcc tggggacttt ccacacctgg ttgctgacta attgagatgc 21300
   atgctttgca tacttctgcc tgctggggag cctggggact ttccacaccc taactgacac 21360
   acattccaca gccggatctg caggacccaa cgctgcccga gatgcgccgc gtgcggctgc 21420
   tggagatggc ggacgcgatg gatatgttct gccaagggtt ggtttgcgca ttcacagttc 21480
   tccgcaagaa ttgattggct ccaattcttg gagtggtgaa tccgttagcg aggtgccgcc 21540
   ggcttccatt caggtcgagg tggcccggct ccatgcaccg cgacgcaacg cggggaggca 21600
   gacaaggtat agggcggcgc ctacaatcca tgccaacccg ttccatgtgc tcgccgaggc 21660
   ggcataaatc gccgtgacga tcagcggtcc aatgatcgaa gttaggctgg taagagccgc 21720
   gagcgatcct tgaagctgtc cctgatggtc gtcatctacc tgcctggaca gcatggcctg 21780
   caacgcgggc atcccgatgc cgccggaagc gagaagaatc ataatgggga aggccatcca 21840
   gcctcgcgtc gcgaacgcca gcaagacgta gcccagcgcg tcggccgcca tgccggcgat 21900
   aatggcctgc ttctcgccga aacgtttggt ggcgggacca gtgacgaagg cttgagcgag 21960
   ggcgtgcaag attccgaata ccgcaagcga caggccgatc atcgtcgcgc tccagcgaaa 22020
   gcggtcctcg ccgaaaatga cccagagcgc tgccggcacc tgtcctacga gttgcatgat 22080
   aaagaagaca gtcataagtg cggcgacgat agtcatgccc cgcgcccacc ggaaggagct 22140
   gactgggttg aaggctctca agggcatcgg tcgaggaact ttcggcggct ttgctgtgcg 22200
   acaggctcac gtctaaaagg aaataaatca tgggtcataa aaattatcac gttgtcggcg 22260
   cggcgacgga tgttctgtat gcgctgtttt ccgttggccg ttgctgtctg gtgatctgcc 22320
   ttctaaatct gcacagccga attgcgcgag cttggttttg ctgaaaccga cacacagcaa 22380
   ctgaatacca gaaagaaaat cactttgcct ttctgacatc agaagggcag aaatttgccg 22440
   ttgaacacct ggtcaatacg cgttttggtg agcagcaata ttgcgcttcg atgagccttg 22500
   gcgttgagat tgatacctct gctgcacaaa aggcaatcga ccgagctgga ccagcgcatt 22560
   cgtgacaccg tctccttcga acttattcgc aatggagtgt cattcatcaa ggacngcctg 22620
   atcgcaaatg gtgctatcca cgcagcggca atcgaaaacc ctcagccggt gaccaatatc 22680
   tacaacatca gccttggtat cctgcgtgat gagccagcgc agaacaaggt aaccgtcagt 22740
   gccgataagt tcaaagttaa acctggtgtt gataccaaca ttgaaacgtt gatcgaaaac 22800
   gcgctgaaaa acgctgctga atgtgcggcg ctggatgtca caaagcaaat ggcagcagac 22860
   aagaaagcga tggatgaact ggcttcctat gtccgcacgg ccatcatgat ggaatgtttc 22920
   cccggtggtg ttatctggca gcagtgccgt cgatagtatg caattgataa ttattatcat 22980
   ttgcgggtcc tttccggcga tccgccttgt tacggggcgg cgacctcgcg ggttttcgct 23040
   atttatgaaa attttccggt ttaaggcgtt tccgttcttc ttcgtcataa cttaatgttt 23100
   ttatttaaaa taccctctga aaagaaagga aacgacaggt gctgaaagcg agctttttgg 23160
   cctctgtcgt ttcctttctc tgtttttgtc cgtggaatga acaatggaag tcaacaaaaa 23220
   gcagacgtat ctagacacgt ctgaagctag cttcgaggaa ctttcggcgg ctttgctgtg 23280
   cgacaggctc acgtctaaaa ggaaataaat catgggtcat aaaaattatc acgttgtcgg 23340
   cgcggcgacg gatgttctgt atgcgctgtt ttccgttggc cgttgctgtc tggtgatctg 23400
   ccttctaaat ctgcacagcc gaattgcgcg agcttggttt tgctgaaacc gacacacagc 23460
   aactgaatac cagaaagaaa atcactttgc ctttctgaca tcagaagggc agaaatttgc 23520
   cgttgaacac ctggtcaata cgcgttttgg tgagcagcaa tattgcgctt cgatgagcct 23580
   tggcgttgag attgatacct ctgctgcaca aaaggcaatc gaccgagctg gaccagcgca 23640
   ttcgtgacac cgtctccttc gaacttattc gcaatggagt gtcattcatc aaggacngcc 23700
   tgatcgcaaa tggtgctatc cacgcagcgg caatcgaaaa ccctcagccg gtgaccaata 23760
   tctacaacat cagccttggt atcctgcgtg atgagccagc gcagaacaag gtaaccgtca 23820
   gtgccgataa gttcaaagtt aaacctggtg ttgataccaa cattgaaacg ttgatcgaaa 23880
   acgcgctgaa aaacgctgct gaatgtgcgg cgctggatgt cacaaagcaa atggcagcag 23940
   acaagaaagc gatggatgaa ctggcttcct atgtccgcac ggccatcatg atggaatgtt 24000
   tccccggtgg tgttatctgg cagcagtgcc gtcgatagta tgcaattgat aattattatc 24060
   atttgcgggt cctttccggc gatccgcctt gttacggggc ggcgacctcg cgggttttcg 24120
   ctatttatga aaattttccg gtttaaggcg tttccgttct tcttcgtcat aacttaatgt    24180
   ttttatttaa aataccctct gaaaagaaag gaaacgacag gtgctgaaag cgagcttttt    24240
   ggcctctgtc gtttcctttc tctgtttttg tccgtggaat gaacaatgga agtcaacaaa    24300
   aagcagagct tatcgatgat aagcggtcaa acatgagaat tc                       24342
<210> 6
   <211> 31567
   <212> DNA
   <213> artificial sequence
<220>
   <223> synthetic polynucleotide
<220>
   <221> misc_feature
   <222> (19958)..(19960)
   <223> n is a, c, g, or t
<220>
   <221> misc_feature
   <222> (21029)..(21030)
   <223> n is a, c, g, or t
<220>
   <221> misc_feature
   <222> (29841)..(29841)
   <223> n is a, c, g, or t
<220>
   <221> misc_feature
   <222> (30923)..(30923)
   <223> n is a, c, g, or t
<400> 6
   gtcgagcggc cgctcgactc tagagggaca gccccccccc aaagccccca gggatgtaat      60
   tacgtccctc ccccgctagg gggcagcagc gagccgcccg gggctccgct ccggtccggc     120
   gctccccccg catccccgag ccggcagcgt gcggggacag cccgggcacg gggaaggtgg     180
   cacgggatcg ctttcctctg aacgcttctc gctgctcttt gagcctgcag acacctgggg     240
   ggatacgggg aaaaagcttt aggctgaaag agagatttag aatgacagaa tcatagaacg     300
   gcctgggttg caaaggagca cagtgctcat ccagatccaa ccccctgcta tgtgcagggt     360
   catcaaccag cagcccaggc tgcccagagc cacatccagc ctggccttga atgcctgcag     420
   ggatggggca tccacagcct ccttgggcaa cctgttcagt gcgtcaccac cctctggggg     480
   aaaaactgcc tcctcatatc caacccaaac ctcccctgtc tcagtgtaaa gccattcccc     540
   cttgtcctat caagggggag tttgctgtga cattgttggt ctggggtgac acatgtttgc     600
   caattcagtg catcacggag aggcagatct tggggataag gaagtgcagg acagcatgga     660
   cgtgggacat gcaggtgttg agggctctgg gacactctcc aagtcacagc gttcagaaca     720
   gccttaagga taagaagata ggatagaagg acaaagagca agttaaaacc cagcatggag 780
   aggagcacaa aaaggccaca gacactgctg gtccctgtgt ctgagcctgc atgtttgatg 840
   gtgtctggat gcaagcagaa ggggtggaag agcttgcctg gagagataca gctgggtcag 900
   taggactggg acaggcagcg ttcatacaat cgtcaaatca tgaaggctgg aaaagccctc 960
   caagatcccc aagaccaacc ccaacccacc caccgtgccc actggccatg tccctcagtg 1020
   ccacatcccc acagttcttc atcacctcca gggacggtga cccccccacc tccgtgggca 1080
   gctgtgccac tgcagcaccg ctctttggag aaggtaaatc ttgctaaatc cagcccgacc 1140
   ctcccctggc acaacgtaag gccattatct ctcatccaac tccaggacgg agtcagtgag 1200
   gatggggctc tagagggaca gccccccccc aaagccccca gggatgtaat tacgtccctc 1260
   ccccgctagg gggcagcagc gagccgcccg gggctccgct ccggtccggc gctccccccg 1320
   catccccgag ccggcagcgt gcggggacag cccgggcacg gggaaggtgg cacgggatcg 1380
   ctttcctctg aacgcttctc gctgctcttt gagcctgcag acacctgggg ggatacgggg 1440
   aaaaagcttt aggctgaaag agagatttag aatgacagaa tcatagaacg gcctgggttg 1500
   caaaggagca cagtgctcat ccagatccaa ccccctgcta tgtgcagggt catcaaccag 1560
   cagcccaggc tgcccagagc cacatccagc ctggccttga atgcctgcag ggatggggca 1620
   tccacagcct ccttgggcaa cctgttcagt gcgtcaccac cctctggggg aaaaactgcc 1680
   tcctcatatc caacccaaac ctcccctgtc tcagtgtaaa gccattcccc cttgtcctat 1740
   caagggggag tttgctgtga cattgttggt ctggggtgac acatgtttgc caattcagtg 1800
   catcacggag aggcagatct tggggataag gaagtgcagg acagcatgga cgtgggacat 1860
   gcaggtgttg agggctctgg gacactctcc aagtcacagc gttcagaaca gccttaagga 1920
   taagaagata ggatagaagg acaaagagca agttaaaacc cagcatggag aggagcacaa 1980
   aaaggccaca gacactgctg gtccctgtgt ctgagcctgc atgtttgatg gtgtctggat 2040
   gcaagcagaa ggggtggaag agcttgcctg gagagataca gctgggtcag taggactggg 2100
   acaggcagct ggagaattgc catgtagatg ttcatacaat cgtcaaatca tgaaggctgg 2160
   aaaagccctc caagatcccc aagaccaacc ccaacccacc caccgtgccc actggccatg 2220
   tccctcagtg ccacatcccc acagttcttc atcacctcca gggacggtga cccccccacc 2280
   tccgtgggca gctgtgccac tgcagcaccg ctctttggag aaggtaaatc ttgctaaatc 2340
   cagcccgacc ctcccctggc acaacgtaag gccattatct ctcatccaac tccaggacgg 2400
   agtcagtgag gatggggctc tagaggatcc ctcgacctgc aggtcaacgg atcacaacaa 2460
   actggaaaat tcttcaagag aagaatacca gaccacccta cctgcttcct gagaaatctg 2520
   tttgctgctc agaagcaaca gttagaacca gacatggaac aacagactgg ttccaaatca 2580
   ggaaaggagt atgtcaaggc tgtatatcgt caccctgctt atttaactta tatgcatagt 2640
   acataataca aaatgccagg ctggatgaat cgcaagctgg aatcaagatt tctgggagaa 2700
   atatcaataa acgagataca aagatgacac cacacttatg gcagaaaact aagaagaact 2760
   aaagagcctc ttgatgaaag tgaaagagga gagtgaaaaa gccagcttaa aacccaacat 2820
   tcaaaatcaa gatcatcatt tcatggcaaa taaatgggga aacaatggaa acagtgagag 2880
   actttatttt cttgggctcc aaaatcactg cagattgtga ctacagccat gattaaaaga 2940
   tgcttgctcc ttggaagaga agctattacc aaactagaaa gcatattaaa aagcagagac 3000
   gttactttgc tgactaagtt ctgtctagtc aaacctatgg tttttccagt agtcatatat 3060
   ggatgtgagt tgaactataa agaaagctga gcaccaaaga attgatgctt ttgaaatttg 3120
   gtgttggaga agtctcttga gagtcccttg acctgcaagg agatccaacc agtccatcct 3180
   aaaggaaatc agtcctgaat attcattgga aggactgatg ctgaaattga agtttcaata 3240
   ctttgaccac ctaatgcgaa gagccaactc actagaaaag accccgatgt tgggaaaaat 3300
   tgaagccagg aagagaagtg aatgacagag gatgagatgg ttggatggca tcgttgactg 3360
   aatggacatg agtctgatca agttccggga gacagcaaag gacagggctg cctggtctgc 3420
   tgcagtccat ggggttgcaa agagtcggac tcaactgagt aactaaacaa caaccaagca 3480
   gtagaaaaat aaataaaatt tgtctctgag atctcagtac ctctttctgt gcatatccgt 3540
   ctcctgttat tgtactttgt cttctgcttg taataaagct gtcctgttag taaaatctgt 3600
   ttgggtcctc tgaattcttt tagctatcaa aaatggaagg tgattattgt gcaatgtcca 3660
   cctctgagta atatacagag aataaaagaa gggagaaatt atgtgcaagt tctctctcat 3720
   ctcctgcttc tcatttaaaa gattctacct cagtgggggc taaaactcca catttaacag 3780
   tagcaaaaac caatattcca tagcttctta ggaaaccatt ttttatactc ttgtatgtaa 3840
   ttacattcaa gctcaaaagc aaagaagtga ttctgcgttg gtgaaggccc aaccatagaa 3900
   aagaggaaga aaataggcca catactgtgc ttcccccata gctcagttgg taaagaatct 3960
   acctacaatg caggaggcct gggcttgatc cctgggtaag ggagatcccc tggagaagga 4020
   aatggtaacc cactccagta ctcttgcctg taaaatccca tggacggagg agcctggcag 4080
   gctacagcct tggggtggca agagttggac atggattaac aactaaacca ctgccaccac 4140
   tccacatact gagtgctccc cagtggcact agtggtaaag aacccacctg ccggtgcaga 4200
   agacattaaa gacactggct ctatccctgc ttgggaagta gggaagatcc cctagagagg 4260
   gaaatagcaa cccactccag aattcttgcc tggaaaatcc catgaatgaa gactggcggg 4320
   ctgtagtaac tggggtcaca agagttaaac atgatttagc aactaaacat caccacatta 4380
   aaaaaattac cacaaaatag tcatattcca ggctaagggg aataatagca ctagtacctg 4440
   agagaacttt ctcagattct ctgtcaagtt cttccttctc tcagataacc agtagtctag 4500
   tttacctcat cagatattaa ctactcatcg attctaaatt atctaattat gggggggggc 4560
   actacattgc attatatttt gtgtccattg actatcactc aatttattta taaaaaattc 4620
   atccatgttg ttttctgtga cagtaactca ttcacattaa ttgtaatatc tcattgcatt 4680
   gtatactaca atttatttat acaaaatact attattcaca cttctgttga ttttaatttg 4740
   gaacatcaac aataacgtgg ctgagaagct tctttcttta gtatattgtt aaggatttcc 4800
   ttgatcaaga ttttacctac ttttctggtc caattggtga gagacagtca taaggaaatg 4860
   ctgtgtttat tgcacaatat gtaaagcatc ttcctgagaa aataaaaggg aaatgttgaa 4920
   tgggaaggat atgctttctt ttgtattcct tttctgagaa atcagacttt ttcacctgtg 4980
   gccttggcca ccaaaagcta acaaataaag gcatatgaag tagccaaggc cttttctagt 5040
   tatatctatg acactgagtt catttcatca tttattttcc tgacttcctc ctgggtccat 5100
   atgagcagtc ttagaatgaa tattagctga ataatccaaa tacatagtag atgttgattt 5160
   gggttttcta agcaatccaa gacttgtatg acagtaagat gtattaccat ccaacacaca 5220
   tctcagcatg atataaatgc aaggtatatt gtgaagaaaa atttttaatt atgtcaaagt 5280
   gcttacttta gaaggtcatc tatctgtccc aaagctgtga atatatatat tgaaggtaat 5340
   gaatagatga agctaacctt gtaaaaatga gtagtgtgaa atacaactac aattatgaac 5400
   atctgtcact aaagaggcaa agaaacttga agattgcttt tgcaaatggg ctcctattaa 5460
   taaaaagtac ttttgaggtc tggctcagac tctattgtag tacttagggt aagaccctcc 5520
   tcctgtatgg gctttcattt tctttcttgc ttccctcatt tgcccttcca tgaatactag 5580
   ctgataaaca ttgactataa aagatatgag gccaaacttg agctgtccca ttttaataaa 5640
   tctgtataaa taatatttgt tctacaaaag tattatctaa ataaatgtta ctttctgtct 5700
   taaaatccct caacaaatcc ccactatcta gagaataaga ttgacattcc ctggaatcac 5760
   agcatgcttt gtctgccatt atctgacccc tttctctttc tctcttctca cctccatcta 5820
   ctcctttttc cttgcaattc atgacccaga ttcactgttt gatttggctt gcatgtgtgt 5880
   gtgctgagtt gcgtctgact gttatcaacc ccatgaatga tagtccacca ggctctactg 5940
   tccatgaaat tttccagtca agaatactgg agtggattgc atttcctact ccatttgatt 6000
   aatttagtga cttttaaatt tctttttcca tattcgggag cctattcttc ctttttagtc 6060
   tatactctct tcactcttca ggtctaaggt atcatcgtgt gcttgttagc ttgttacttt 6120
   ctccattata gcttaagcac taacaactgt tcaggttggc atgaaattgt gttctttgtg 6180
   tggcctgtat atttctgttg tgtattagaa tttaccccaa gatctcaaag acccactgaa 6240
   tactaaagag acctcattgt ggttacaata atttggggac tgggccaaaa cttccgtgca 6300
   tcccagccaa gatctgtagc tactggacaa tttcatttcc tttatcagat tgtgagttat 6360
   tcctgttaaa atgctcccca gaatttctgg ggacagaaaa ataggaagaa ttcaaatcca 6420
   ctgttggttt tatttcaaac cacaaaatta gcatgccatt aaatactata tataaacagc 6480
   cactaaatca gatcattatc cattcagctt ctccttcact tcttctcctc tactttggaa 6540
   aaaaggtaag aatctcagat ataatttcag tgtatctgct actcatcttt attttggact 6600
   aggttaaaat gtagaaagaa cataattgct taaaatagat cttaaaaata agggtgttta 6660
   agataaggtt tacactattt tcagcagata tgttaaaaaa tagaagtgac tataaagact 6720
   tgataaaaat tatagtgact gcaaatgttt taggaatata ataagatata ataacagtgg 6780
   ttgctatttt ctttagcaca agactagtta acaggctgta ttaaaagatc ttttcttgaa 6840
   ttaaatattt tcaatttgat taaacctacc tcagccataa aggcaagcac atttcattta 6900
   tactatgggg atttgaataa ttattactga agaagctcta ccaacaaaaa gtttatagag 6960
   ctatcatatt tagtcaagag ataaagaggg ttgttaggat atatatgcta tttgaaaggt 7020
   atttataaaa gaagagtata tttatcaaaa tttctcagaa catccaaatt tcaagtttat 7080
   catttatctt acaatatttc aaaaatatta aaatagatac atgaaataca gaagtaaatt 7140
   aaagagaaag tattttactt ggtaaaaaaa ttctaggttg gacagagagt gccaggaaac 7200
   aaaaacaatg aaaaatgtga cctgacagga attatagctc aaagtatagt agtaagtaat 7260
   gaaatggctt aaaaattggt atataaaatg ctagttataa aataaacaaa atgcaataat 7320
   atcctcccta catgtaatga attctaggta ttatgctctt ttttgaagtc ttgacaataa 7380
   aaattttttt agaagtttat aggcatcttg aataaagtga aacaaattaa gaattagtat 7440
   ccatgagaaa aatatagaac aattttccta atttagtttg aaaatctggg attgaagatg 7500
   tgtgtcaaga gatgttggtg gcaagaacat ttttttttca agaacttata aaaatgcaac 7560
   aaaacaaacc atttaataca ttttggtcaa aatcaataat gtattttatt ttatgctcca 7620
   aggagcataa aattggggac tgggcaagag aaactgacac cctggtaaat taccaagaga 7680
   taagtacaca gttctatgta gagaaaataa gcatagtgta tgatctctaa aattatgtga 7740
   gacaaaggag agatgacatt aggcatgtgg ggatgaagac tgagtagaga agaaacaatc 7800
   taatcagtcc aagaaaacat ctcgatcagt ggaacaaata gaagaaatgc taaaatgaaa 7860
   cagaagtctt actggaaata aaagatatgc ataagacaaa aattcatgaa aatcacttag 7920
   tttagcagag aaaagataaa aataaagtat gaccttcttc atatacattg tttgatcata 7980
   tgcacctcaa taaaactgag tctccaacag aaatgaaaca ttaatatttt gttcactgct 8040
   ctaatcccag aatctaagcg atatctggca ataaaaataa taaatatata ttttttaata 8100
   aatgaatcaa ccacttaatt tttctgtaaa tatctgtaac ttctcttctg tctttccaaa 8160
   aacactcata agtactgtga atgagatgaa aaagagtgaa gtaggatata ggctgttagc 8220
   agaaaacatc tgaatggctg gcagtgaaac attaacttga aatgtaagat taatgagtaa 8280
   tagtaaattt taaccttggc catatgataa aatgttcatt aatatttttc tagaatacag 8340
   ggctttttgt ttttgccatg aggtttgcag gatcttggtt ccctgaccag ggatcaaacc 8400
   tgcactcccc tggaagcatg gagtcttgga catttgtatt atacactatc tttggttcct 8460
   tttaaaggga agtaatttta cttaaataag aaaatagatt gacaagtaat acgctgtttc 8520
   ctcatcttcc cattcacagg aatcgcggat cctcgagacc gccatggccg tgctgggcct 8580
   gctgttctgc ctggtgacct tccccagctg cgtgctgtcc caggtgcagc tgaagcagag 8640
   cggccctggc ctggtgcagc cctctcagtc cctctccatt acctgtaccg tgtccggctt 8700
   ctctctcaca aattatggag tccattgggt gcgccagagc cccggcaagg gcctggagtg 8760
   gctgggcgtg atctggtccg gcggcaacac cgactacaac acccccttca ccagcagact 8820
   gagcatcaac aaggacaaca gcaagagcca ggtgttcttc aagatgaaca gcctgcagag 8880
   caacgacacc gccatctact actgcgctcg cgctctcacc tactacgact acgagttcgc 8940
   ctactggggc cagggcaccc tggtgaccgt gtccgccgcc agcaccaagg gccccagcgt 9000
   gttccccctg gcccccagca gcaagagcac cagcggcggc accgctgccc tgggctgtct 9060
   ggtgaaggac tacttccccg agcccgtgac cgtgtcttgg aactctggcg ccctgacctc 9120
   cggcgtgcac acatttccag ctgtcctcca gagttccgga ctgtattccc tcagttctgt 9180
   cgtgacagtc ccttcctcta gcctgggcac ccagacctac atctgcaacg tgaaccacaa 9240
   gcccagcaac accaaggtgg acaagagagt ggagcccaag agctgcgaca agacccacac 9300
   ctgcccacca tgtcctgctc cagagctgct ggggggaccc tccgtgttcc tgttcccccc 9360
   caagcccaag gacaccctga tgatcagcag gacccccgag gtgacctgcg tggtggtgga 9420
   cgtgtcccac gaggaccctg aggtgaagtt caactggtac gtggacggcg tggaggtgca 9480
   caacgccaag accaagccca gagaggagca gtacaacagc acctacaggg tggtgtccgt 9540
   gctgaccgtg ctgcaccagg actggctgaa cggcaaagaa tacaagtgca aagtctccaa 9600
   caaggccctg ccagccccca tcgaaaagac catcagcaag gccaagggcc agcctcgcga 9660
   gccccaggtg tacaccctgc ccccctcccg cgaggagatg accaagaacc aggtgtccct 9720
   gacctgcctg gtgaagggct tctaccccag cgatatcgcc gtggagtggg agagcaacgg 9780
   ccagcccgag aacaactaca agaccacccc ccctgtgctg gacagcgacg gcagcttctt 9840
   cctgtacagc aagctgaccg tggacaagtc caggtggcag cagggaaatg tcttttcttg 9900
   ttctgtcatg catgaagctc tgcacaacca ctacacccag aagtccctga gcctgtcccc 9960
   cggcaagtga tagctcgagg atccggaccc ttccctattc ttgtaagtct aaatttacta 10020
   actgtgctgt ttaacttctg atgtttgtat gatatttgag taattaagag ccctacaaaa 10080
   aaatcaataa tgaatggttc caaaataagc atagctgaga ttaatgattc tcagcattag 10140
   ttataaatag aataagctgg aaaaccttca cctcccctcc accaccagat ctcaatgtct 10200
   aggcttaccc atggagattc tgattaactg ttctttctat gtagaagaaa cttattggga 10260
   agaaataata taatggacta tgatttaatt ggtctgttga gaatttagat gaaggggatt 10320
   aagttacaat aaagccagaa tttaacttga taatctcatt tggctaagaa taacaaacct 10380
   aagaaggttt gctattttct acaattttga agttttcctt atgcacaatt atttcaccac 10440
   atgactcatt tcacatcttg tttttgatat atgagcatat gagggcaaaa tactgaagat 10500
   gcttatttca atactcaggg aaaattttct tgccaaaagg caagaattgt ataattcatt 10560
   cacttatttt atttttttta atttttaagg tctaagagga tttcaaagtg aatgccccct 10620
   cctcactttt ggtaagcttt aggagattgg aggcagactg atcattttta tagttaatat 10680
   cttttacatt tcatcttcct ggataagccc caatagtagc aatttctatc agtataccag 10740
   cataaagatt agttttaaat ttattttcag tgattgactg ttatttactg acctgaaatt 10800
   atgtatctgt tatatttcaa ataatgcaaa actgtatata tatggtgttg acagatttga 10860
   ttggttttct ttcaattgcc tatatcctta ttattgattg taatcattta tagaaaaaac 10920
   aaaataattt cttatacttt tatgtaaacc tgttagagct tattttaaag atcaactgca 10980
   ttcacatttc taatctagtc attatgagct tcaattgttt tatctcactt aaaatttata 11040
   tattgtcttt taattcatga gtcaaaatac aatctcacag tccagatatg ggacttaaaa 11100
   ggggaatagc atatagtttt gatattctta aagatataca tctttttgtg atcatgattc 11160
   agcagacatt ttaataaaac aattccaagt gagccgacac ttggtcctag aggaattttt 11220
   ataaccttaa gataaggcac agcatggtgt ttttgtaata agatttcttt tatgaaaaag 11280
   tcacaccaaa attggaaatg gggtgagatg aagagttata acatataact aaatggacat 11340
   ttgttctcta ttccacagaa ttgactgcga ctggaaatat ggcaactttt caatccttgc 11400
   atcatgctac taagataatt tttaaatgag tatacatgga acaaaaaatg aaactttatt 11460
   cctttattta tattatgctt tttcatctta atttgaattt gagtcataaa ccatatactt 11520
   tcaaaatgtt aattcaacat tagcataaaa gttcaatttt aacttggaaa tatcatgaac 11580
   atatcaaatt atgtataaaa ataatttctg gaattgtgat tattatttct ttaagaatct 11640
   atttcctaac cagtcatttc aataaattaa cccttaggca tatttaagtt ttcttgtctt 11700
   tattatattt ttaaaaatga aattggtctc tttattgtta acttaaattt atctttgatg 11760
   ttaaaaatag ctgtggaaaa ttaaaattga atagaattct ttgaattgag ttccaaagga 11820
   tatcaaaaag tgagggaaaa gatagggtga gcctatgctg catatgtcct tagaaagtct 11880
   tggtttatac ctgttaccta agttaaacaa ttatacttgt tcctttcact ctcgaaagta 11940
   cccagcattg gatgttaaat tttatagtca tcctagacaa aaaaaaaaaa aaaaacaaac 12000
   aaccctcaaa tgtgatatct gaatcacagc tctacagtgt ggtagctaag tggtgctgtg 12060
   taagttagtc tccaagagat tccatttcta catttataaa cagtcaattt aaggtgtttt 12120
   attgaagttt taatgtgaaa agtgcactat atggtgcatg ataggagttc ctggttgaat 12180
   ctcatttctg acatcactga caccagtgca gcaaggacta gtgttacaat cagaaggagc 12240
   tgagttgtgt aattttagcc attaatgccc aagagactag aacttacaca aagctctaat 12300
   atccattgtc tctgtctgtg gagtaattat ttcattgcca tgaattatct gtctgtcata 12360
   tcctgcattt ttatacatga ttcagttccc ttcagttcac acaatgactt gtctaatttc 12420
   atctttcctg catcctccat gttttcctca cttcaggatt aagtgaagcc gtacttaggc 12480
   acaatatttc ttatctttaa agaaaaattc catctttgag agttgttatt gttcagtcac 12540
   taggtcatgt ccaactcttt gtgaccccat gcactgcagc atgccaggct tccctgccct 12600
   tcgctctctc ctggagtttg ctcagactca tgtagattga gtcggtgatg gtatccaact 12660
   atctcatcaa ctgttgtgcc cttctcctcc taccctcagt ctttaccagc atcagagtct 12720
   ttctcagatt cttcaggtta ttatataaca actatcataa aaggagtatc taaatggctg 12780
   tgtccattat ttcacatgtt attctctctt taacttgctc caatcccaat tttatcccta 12840
   tgggaactgc tttattgaag atcaccaaca acttttattt tactaatcgt tttgttttac 12900
   ccaacctctc agtgagtgtt atgaggtaga gttgactatt tcttcatttt gaaatattac 12960
   gcttcatttc atttgatatc ctaaagctca taaggtgtgg tttttctctt aactcactag 13020
   acacttcttt gaagtctctc ttctggcatt ttctcctttt ccaaaatttt aatggttgga 13080
   gtaccctaga ttttagcctt aatttgtttg atgttgttca gttccattct cagctcagag 13140
   cttccaactg tatgtctcca aacttactcg ttttgtaaac tccaaactca tgcactcaac 13200
   tgcattcttg acctccacac tgaattatct aattaatgtc ctaaatctgg catgaccaag 13260
   catacatttt tgtctgaatc cagtccccaa cttgctcaaa atttaattaa acgtaattca 13320
   gttacaaagg cagctgatat tgtatgcaat agacctgaat gggaacttca caaaagaagt 13380
   tatcttaatt gtcaataaaa acatgaaaaa tactctacat catcaatctt cagaaaaatg 13440
   caaattaaag gtgcctaata atatcatgac acaaccgtca gaatgactga aatgaaaaga 13500
   attgtaatac agttcagttc agttcagtta ctcagtcgtc tccaactctt tgtgacccca 13560
   tgaactgcag catgacagac cttcctgtcc atcaccaact cccagagttt actcagacta 13620
   tgtccattga gttgatgatg ccatccaacc atctcatcct ctgtcgtccc cttctcctcc 13680
   tgccctcagt ctttcccagc atcagggtct tttccaatga gtcagctctt cgcatcaggt 13740
   ggctaaagta ttggagtttc agcttcaaca tcagtccttc taattaacac ccaggactga 13800
   tctcttttag gatggactag ttggatctcc ttgcagtcca agggactctc aagagtcttc 13860
   tccaacacca cagttcaaaa gcatcaattc cttggcactc agctttcctt atagtccatg 13920
   tctcacatcc acacatgact attggaaaaa ccatagcctt gactaggtgg acctttgttg 13980
   acaaagtaat gtctctgctt tttaatatgt tgtctagatt ggtcataact ttccttccaa 14040
   gaagtaattg tcttttaatt tcatggctgc agtcaccatc tgcagtgatt ttggagcccc 14100
   aaaatataaa gtcagctgct gtttccactg ttgccccatc taccccatct atttgccatg 14160
   aagtgatggg actggatgcc actatcttag ttttctgaat gttgagcttt aagccagcct 14220
   ttttactctc ctctttcact ttcatcaaga ggctctttag ttcctcttca ctttctgcca 14280
   taagggtggt gtcatctgca tatctgaggt tattgatatt tctcttggca attttgattc 14340
   cagcctgcac ttcttccagc ccagtgtttc tcatgatgta ctctgcatat aaattaaata 14400
   agcagagtga caatatacag ccttgacata ctctttttcc tatttggaac cagtctgttg 14460
   ttccatgtcc agttctaact gttgtttcct gacctgcata caggtttctc aagaggcaag 14520
   tcaggtggtc tggtattctc acctgtttca gaattttcca cagtttattg tgatccacac 14580
   agtcaaaggc tttggcatag ccaataaagc agaaagagat gtttttctgg aactctctta 14640
   cttttttgat gatccagtgg atgttggcaa tttgatctct ggttcctctg ccttttctaa 14700
   aaccagcttt aacatctgga agttcatggt tcacgtaata caaaatgtaa tacaaaatgt 14760
   ctgcaaaaac aaaggaatga aaagtaatgc taaaaaatgt taatatttac agaaattttt 14820
   atagtagtaa agaattcacc tgcaatacag gagaaccggg ttagatccct gggttggaag 14880
   acctcctgga gaaggaaatg gctacccaat ctagtattct tgtctggaga aggcaagaat 14940
   ggacagagaa gcccagcggg ctatggtcca tcgggtcaca aagagtcaga agctaccttg 15000
   cacacagcaa gcacggtgcg cgcgcgtgca cacacacaca cacacacaca cacagacaca 15060
   cacacactct aaaacattta cccaagcttg tccaatggaa aatcaaaaag ccagcaattt 15120
   aagatgacat caggtaccac tgtccaggta agcctcagaa cacaatgacc agtaagaagc 15180
   aaagtgccat atgagcaact cgaatttttg caatgttacc taagagcttc catttttata 15240
   atgcaaaaga atttcatatg gggaaattgt attagataac cctgaatgag gagcaagata 15300
   tagtcaaagt aagatgctct agtactattt tttataagca tgatttgttc agccaaaggt 15360
   tttttcccat atggccaatg aactgaaata tgcagtcctg agatttgcat atatttctag 15420
   ctgaaaccaa gtaaataata tcctcaagaa agaaatcaat agaaaagttg gatgaagagt 15480
   acaataaagg gaccaaaaat attcagaaat aagaactaga ggagatattg ggaaatccct 15540
   ggtgagtcca gtttaggatt ttgtactttc actgcagttg gcatggatat aatccctcac 15600
   tggggaacta agatcccata agctgtgttg gattgccaaa aaaataaata ttaagagata 15660
   tcattcatag aatattttaa agatatttta gagaagagga aattaaggat gtgagaattt 15720
   gtattacttt ttcaagatac taaagctatt tagagataga gctgttacta aaaacttcag 15780
   tttcctaaaa attatttgaa gcactgttta ataaattcca aaatatagag gaaggaaaac 15840
   aaaatactga ggattcatat aatgattcag atttagaaac aatataacac agaattagtg 15900
   aattctgaca aattattagg taggagtaga tagttcagca ttactcgtat agatggagta 15960
   tttaatcctt tccatgagat tatccaaata taataatttc gtatctatgt gaagtataac 16020
   tattaagatt actttataaa gtaaatcaag aaccagagaa taagaaaaat gttttgtgaa 16080
   ccagcagata ctatgaacac ataaaactca gaaccctgat tcctaagaca cacagctaat 16140
   cctgattatt cttcctttac atgtgaccat agaacttcac acaagttcaa gatacatttg 16200
   ttgagcacat cagtatcagt tcagtcactc agtcatgtcc gaatctttgt gaccttgtgg 16260
   actgcagcac gccaggcttt cctgtccacc accaacccct ggagcttact caaactcatg 16320
   tccattgagt cagtgatccc atccaaccat ctcatcctct gtcatcctct tctcctgcct 16380
   tcaatctttc ccagacattg gagtcttttc caatgagtca gatcttcaca ttaggtggcc 16440
   aaagtatagg agtttcagct tcagcatcaa tccttccaat gaatattcct tgatgtaccc 16500
   ctttcgcagt ttggaaccag tctgttgttc catgtccagt tctaactgct gcttctggac 16560
   ctgtatacag atttctcagg aggcaggtaa agtggtctgg tattcccatc tcttgaagaa 16620
   ttttccacag tttattgtga tccacacaat caaaggcttt agcgtagtca ataaagcaga 16680
   tgtttttctg gaactctcgt gcttttttga tgatccaatg gatgttggca atttgatctc 16740
   tggttcctct gccttttcta aatccagctt gaacatctgg aagttcatgg tccacgtact 16800
   gttgaagcct ggcttggaga attttgagag ttattttgct agcatgtgag atgagtgcaa 16860
   tcatgtgggt gtttgaacat actttgtcat tgcttttctt tgggattgtg gcagtcctgt 16920
   ggccactgct gagttttcca aatttgctga catattgagt gcagcacttt cacagcatca 16980
   ccttttaaga tttgaaatag ctcaactgga attccatcac ctccactagc tttgttcata 17040
   gtgaggcttt ctaaggccgt ttgactttgc attccagggt gtctggctct aggtgagtga 17100
   tccgttgacc tgcagcggcc ctcgactcta gagggacagc ccccccccaa agcccccagg 17160
   gatgtaatta cgtccctccc ccgctagggg cagcagcgag ccgcccgggg ctccgctccg 17220
   gtccggcgct ccccccgcat ccccgagccg gcagcgtgcg gggacagccc gggcacgggg 17280
   aaggtggcac gggatcgctt tcctctgaac gcttctcgct gctctttgag cctgcagaca 17340
   cctgggggga tacggggaaa aagctttagg ctgaaagaga gatttagaat gacagaatca 17400
   tagaacggcc tgggttgcaa aggagcacag tgctcatcca gatccaaccc cctgctatgt 17460
   gcagggtcat caaccagcag cccaggctgc ccagagccac atccagcctg gccttgaatg 17520
   cctgcaggga tggggcatcc acagcctcct tgggcaacct gttcagtgcg tcaccaccct 17580
   ctgggggaaa aactgcctcc tcatatccaa cccaaacctc ccctgtctca gtgtaaagcc 17640
   attccccctt gtcctatcaa gggggagttt gctgtgacat tgttggtctg gggtgacaca 17700
   tgtttgccaa ttcagtgcat cacggagagg cagatcttgg ggataaggaa gtgcaggaca 17760
   gcatggacgt gggacatgca ggtgttgagg gctctgggac actctccaag tcacagcgtt 17820
   cagaacagcc ttaaggataa gaagatagga tagaaggaca aagagcaagt taaaacccag 17880
   catggagagg agcacaaaaa ggccacagac actgctggtc cctgtgtctg agcctgcatg 17940
   tttgatggtg tctggatgca agcagaaggg gtggaagagc ttgcctggag agatacagct 18000
   gggtcagtag gactgggaca ggcagctgga gaattgccat gtagatgttc atacaatcgt 18060
   caaatcatga aggctggaaa gcctccaaga tccccaagac caaccccaac ccacccaccg 18120
   tgcccactgg ccatgtccct cagtgccaca tccccacagt tcttcatcac ctccagggac 18180
   ggtgaccccc ccacctccgt gggcagctgt gccactgcag caccgctctt tggagaaggt 18240
   aaatcttgct aaatccagcc cgaccctccc ctggcacaac gtaaggccat tatctctcat 18300
   ccaactccag gacggagtca gtgaggatgg ggctctagag ggacagcccc cccccaaagc 18360
   ccccagggat gtaattacgt ccctcccccg ctaggggcag cagcgagccg cccggggctc 18420
   cgctccggtc cggcgctccc cccgcatccc cgagccggca gcgtgcgggg acagcccggg 18480
   cacggggaag gtggcacggg atcgctttcc tctgaacgct tctcgctgct ctttgagcct 18540
   gcagacacct ggggggatac ggggaaaaag ctttaggctg aaagagagat ttagaatgac 18600
   agaatcatag aacggcctgg gttgcaaagg agcacagtgc tcatccagat ccaaccccct 18660
   gctatgtgca gggtcatcaa ccagcagccc aggctgccca gagccacatc cagcctggcc 18720
   ttgaatgcct gcagggatgg ggcatccaca gcctccttgg gcaacctgtt cagtgcgtca 18780
   ccaccctctg gggaaaaaac tgcctcctca tatccaaccc aaacctcccc tgtctcagtg 18840
   taaagccatt cccccttgtc ctatcaaggg ggagtttgct gtgacattgt tggtctgggg 18900
   tgacacatgt ttgccaattc agtgcatcac ggagaggcag atcttgggga taaggaagtg 18960
   caggacagca tggacgtggg acatgcaggt gttgagggct ctgggacact ctccaagtca 19020
   cagcgttcag aacagcctta aggataagaa gataggatag aaggacaaag agcaagttaa 19080
   aacccagcat ggagaggagc acaaaaaggc cacagacact gctggtccct gtgtctgagc 19140
   ctgcatgttt gatggtgtct ggatgcaagc agaaggggtg gaagagcttg cctggagaga 19200
   tacagctggg tcagtaggac tgggacaggc agctggagaa ttgccatgta gatgttcata 19260
   caatcgtcaa atcatgaagg ctggaaagcc tccaagatcc ccaagaccaa ccccaaccca 19320
   cccaccgtgc ccactggcca tgtccctcag tgccacatcc ccacagttct tcatcacctc 19380
   cagggacggt gaccccccca cctccgtggg cagctgtgcc actgcagcac cgctctttgg 19440
   agaaggtaaa tcttgctaaa tccagcccga ccctcccctg gcacaacgta aggccattat 19500
   ctctcatcca actccaggaa cggagtcagt gaggatgggg ctctagagga tccctcgaca 19560
   tctatgtcgg gtgcggagaa agaggtaatg aaatggcact cgacggtatc gataagcttg 19620
   atatcgaatt ctaccgggta ggggaggcgc ttttcccaag gcagtctgga gcatgcgctt 19680
   tagcagcccc gctggcactt ggcgctacac aagtggcctc tggcctcgca cacattccac 19740
   atccaccggt agcgccaacc ggctcccttc tttggtggcc ccttcgcgcc accttctact 19800
   cctcccctag tcaggaagtt cccccccgcc ccgcagctcg cgtcgtgcag gacgtgacaa 19860
   atggaagtag cacgtctcac tagtctcgtg cagatggaca gcaccgctga gcaatggaag 19920
   cgggtaggcc tttggggcag cggccaatag cagcttgnnn ccttcgcttt ctgggctcag 19980
   aggctgggaa ggggtgggtc cgggggcggg ctcaggggcg ggctcagggg cggggcgggc 20040
   gcgaaggtcc tccggagccc ggcattctgc acgcttcaaa agcgcacgtc tgccgcgctg 20100
   ttctcctctt cctcatctcc gggcctttcg acctgcagcc aatatgattg aacaagatgg 20160
   attgcacgca ggttctccgg ccgcttgggt ggagaggcta ttcggctatg actgggcaca 20220
   acagacaatc ggctgctctg atgccgccgt gttccggctg tcagcgcagg ggcgcccggt 20280
   tctttttgtc aagaccgacc tgtccggtgc cctgaatgaa ctgcaggacg aggcagcgcg 20340
   gctatcgtgg ctggccacga cgggcgttcc ttgcgcagct gtgctcgacg ttgtcactga 20400
   agcgggaagg gactggctgc tattgggcga agtgccgggg caggatctcc tgtcatctca 20460
   ccttgctcct gccgagaaag tatccatcat ggctgatgca atgcggcggc tgcatacgct 20520
   tgatccggct acctgcccat tcgaccacca agcgaaacat cgcatcgagc gagcacgtac 20580
   tcggatggaa gccggtcttg tcgatcagga tgatctggac gaagagcatc aggggctcgc 20640
   gccagccgaa ctgttcgcca ggctcaaggc gcgcatgccc gacggcgagg atctcgtcgt 20700
   gacccatggc gatgcctgct tgccgaatat catggtggaa aatggccgct tttctggatt 20760
   catcgactgt ggccggctgg gtgtggcgga ccgctatcag gacatagcgt tggctacccg 20820
   tgatattgct gaagagcttg gcggcgaatg ggctgaccgc ttcctcgtgc tttacggtat 20880
   cgccgctccc gattcgcagc gcatcgcctt ctatcgcctt cttgacgagt tcttctgagg 20940
   ggatcaattc tctagagctc gctgatcagc ctcgactgtg ccttctagtt gccagccatc 21000
   tgttgtttgc ccctcccccg tgccttccnn gaccctggaa ggtgccactc ccactgtcct 21060
   ttcctaataa aatgaggaaa ttgcatcgca ttgtctgagt aggtgtcatt ctattctggg 21120
   gggtggggtg gggcaggaca gcaaggggga ggattgggaa gacaatagca ggcatgctgg 21180
   ggatgcggtg ggctctatgg cttctgaggc ggaaagaacc agctggggct cgatcgatcg 21240
   agttcgatcg actctagagg gacagccccc ccccaaagcc cccagggatg taattacgtc 21300
   cctcccccgc taggggcagc agcgagccgc ccggggctcc gctccggtcc ggcgctcccc 21360
   ccgcatcccc gagccggcag cgtgcgggga cagcccgggc acggggaagg tggcacggga 21420
   tcgctttcct ctgaacgctt ctcgctgctc tttgagcctg cagacacctg gggggatacg 21480
   gggaaaaagc tttaggctga aagagagatt tagaatgaca gaatcataga acggcctggg 21540
   ttgcaaagga gcacagtgct catccagatc caaccccctg ctatgtgcag ggtcatcaac 21600
   cagcagccca ggctgcccag agccacatcc agcctggcct tgaatgcctg cagggatggg 21660
   gcatccacag cctccttggg caacctgttc agtgcgtcac caccctctgg gggaaaaact 21720
   gcctcctcat atccaaccca aacctcccct gtctcagtgt aaagccattc ccccttgtcc 21780
   tatcaagggg gagtttgctg tgacattgtt ggtctggggt gacacatgtt tgccaattca 21840
   gtgcatcacg gagaggcaga tcttggggat aaggaagtgc aggacagcat ggacgtggga 21900
   catgcaggtg ttgagggctc tgggacactc tccaagtcac agcgttcaga acagccttaa 21960
   ggataagaag ataggataga aggacaaaga gcaagttaaa acccagcatg gagaggagca 22020
   caaaaaggcc acagacactg ctggtccctg tgtctgagcc tgcatgtttg atggtgtctg 22080
   gatgcaagca gaaggggtgg aagagcttgc ctggagagat acagctgggt cagtaggact 22140
   gggacaggca gctggagaat tgccatgtag atgttcatac aatcgtcaaa tcatgaaggc 22200
   tggaaagcct ccaagatccc caagaccaac cccaacccac ccaccgtgcc cactggccat 22260
   gtccctcagt gccacatccc cacagttctt catcacctcc agggacggtg acccccccac 22320
   ctccgtgggc agctgtgcca ctgcagcacc gctctttgga gaaggtaaat cttgctaaat 22380
   ccagcccgac cctcccctgg cacaacgtaa ggccattatc tctcatccaa ctccaggacg 22440
   gagtcagtga ggatggggct ctagagggac agcccccccc caaagccccc agggatgtaa 22500
   ttacgtccct cccccgctag gggcagcagc gagccgcccg gggctccgct ccggtccggc 22560
   gctccccccg catccccgag ccggcagcgt gcggggacag cccgggcacg gggaaggtgg 22620
   cacgggatcg ctttcctctg aacgcttctc gctgctcttt gagcctgcag acacctgggg 22680
   ggatacgggg aaaaagcttt aggctgaaag agagatttag aatgacagaa tcatagaacg 22740
   gcctgggttg caaaggagca cagtgctcat ccagatccaa ccccctgcta tgtgcagggt 22800
   catcaaccag cagcccaggc tgcccagagc cacatccagc ctggccttga atgcctgcag 22860
   ggatggggca tccacagcct ccttgggcaa cctgttcagt gcgtcaccac cctctggggg 22920
   aaaaactgcc tcctcatatc caacccaaac ctcccctgtc tcagtgtaaa gccattcccc 22980
   cttgtcctat caagggggag tttgctgtga cattgttggt ctggggtgac acatgtttgc 23040
   caattcagtg catcacggag aggcagatct tggggataag gaagtgcagg acagcatgga 23100
   cgtgggacat gcaggtgttg agggctctgg gacactctcc aagtcacagc gttcagaaca 23160
   gccttaagga taagaagata ggatagaagg acaaagagca agttaaaacc cagcatggag 23220
   aggagcacaa aaaggccaca gacactgctg gtccctgtgt ctgagcctgc atgtttgatg 23280
   gtgtctggat gcaagcagaa ggggtggaag agcttgcctg gagagataca gctgggtcag 23340
   taggactggg acaggcagct ggagaattgc catgtagatg ttcatacaat cgtcaaatca 23400
   tgaaggctgg aaagcctcca agatccccaa gaccaacccc aacccaccca ccgtgcccac 23460
   tggccatgtc cctcagtgcc acatccccac agttcttcat cacctccagg gacggtgacc 23520
   cccccacctc cgtgggcagc tgtgccactg cagcaccgct ctttggagaa ggtaaatctt 23580
   gctaaatcca gcccgaccct cccctggcac aacgtaaggc cattatctct catccaactc 23640
   caggaacgga gtcagtgagg atggggctct agaggatccg cggccgcgaa ttcttgaaga 23700
   cgaaagggcc tcgtgatacg cctattttta taggttaatg tcatgataat aatggtttct 23760
   tagacgtcag gtggcacttt tcggggaaat gtgcgcggaa cccctatttg tttatttttc 23820
   taaatacatt caaatatgta tccgctcatg agacaataac cctgataaat gcttcaataa 23880
   tattgaaaaa ggaagagtat gagtattcaa catttccgtg tcgcccttat tccctttttt 23940
   gcggcatttt gccttcctgt ttttgctcac ccagaaacgc tggtgaaagt aaaagatgct 24000
   gaagatcagt tgggtgcacg agtgggttac atcgaactgg atctcaacag cggtaagatc 24060
   cttgagagtt ttcgccccga agaacgtttt ccaatgatga gcacttttaa agttctgcta 24120
   tgtggcgcgg tattatcccg tgttgacgcc gggcaagagc aactcggtcg ccgcatacac 24180
   tattctcaga atgacttggt tgagtactca ccagtcacag aaaagcatct tacggatggc 24240
   atgacagtaa gagaattatg cagtgctgcc ataaccatga gtgataacac tgcggccaac 24300
   ttacttctga caacgatcgg aggaccgaag gagctaaccg cttttttgca caacatgggg 24360
   gatcatgtaa ctcgccttga tcgttgggaa ccggagctga atgaagccat accaaacgac 24420
   gagcgtgaca ccacgatgcc tgcagcaatg gcaacaacgt tgcgcaaact attaactggc 24480
   gaactactta ctctagcttc ccggcaacaa ttaatagact ggatggaggc ggataaagtt 24540
   gcaggaccac ttctgcgctc ggcccttccg gctggctggt ttattgctga taaatctgga 24600
   gccggtgagc gtgggtctcg cggtatcatt gcagcactgg ggccagatgg taagccctcc 24660
   cgtatcgtag ttatctacac gacggggagt caggcaacta tggatgaacg aaatagacag 24720
   atcgctgaga taggtgcctc actgattaag cattggtaac tgtcagacca agtttactca 24780
   tatatacttt agattgattt aaaacttcat ttttaattta aaaggatcta ggtgaagatc 24840
   ctttttgata atctcatgac caaaatccct taacgtgagt tttcgttcca ctgagcgtca 24900
   gaccccgtag aaaagatcaa aggatcttct tgagatcctt tttttctgcg cgtaatctgc 24960
   tgcttgcaaa caaaaaaacc accgctacca gcggtggttt gtttgccgga tcaagagcta 25020
   ccaactcttt ttccgaaggt aactggcttc agcagagcgc agataccaaa tactgtcctt 25080
   ctagtgtagc cgtagttagg ccaccacttc aagaactctg tagcaccgcc tacatacctc 25140
   gctctgctaa tcctgttacc agtggctgct gccagtggcg ataagtcgtg tcttaccggg 25200
   ttggactcaa gacgatagtt accggataag gcgcagcggt cgggctgaac ggggggttcg 25260
   tgcacacagc ccagcttgga gcgaacgacc tacaccgaac tgagatacct acagcgtgag 25320
   ctatgagaaa gcgccacgct tcccgaaggg agaaaggcgg acaggtatcc ggtaagcggc 25380
   agggtcggaa caggagagcg cacgagggag cttccagggg gaaacgcctg gtatctttat 25440
   agtcctgtcg ggtttcgcca cctctgactt gagcgtcgat ttttgtgatg ctcgtcaggg 25500
   gggcggagcc tatggaaaaa cgccagcaac gcggcctttt tacggttcct ggccttttgc 25560
   tggccttttg ctggcctttt gctcacatgt tctttcctgc gttatcccct gattctgtgg 25620
   ataaccgtat taccgccttt gagtgagctg ataccgctcg ccgcagccga acgaccgagc 25680
   gcagcgagtc agtgagcgag gaagcggaag agcgctgact tccgcgtttc cagactttac 25740
   gaaacacgga aaccgaagac cattcatgtt gttgctcagg tcgcagacgt tttgcagcag 25800
   cagtcgcttc acgttcgctc gcgtatcggt gattcattct gctaaccagt aaggcaaccc 25860
   cgccagccta gccgggtcct caacgacagg agcacgatca tgcgcacccg tcagatccag 25920
   acatgataag atacattgat gagtttggac aaaccacaac tagaatgcag tgaaaaaaat 25980
   gctttatttg tgaaatttgt gatgctattg ctttatttgt aaccattata agctgcaata 26040
   aacaagttaa caacaacaat tgcattcatt ttatgtttca ggttcagggg gaggtgtggg 26100
   aggtttttta aagcaagtaa aacctctaca aatgtggtat ggctgattat gatctctagt 26160
   caaggcacta tacatcaaat attccttatt aaccccttta caaattaaaa agctaaaggt 26220
   acacaatttt tgagcatagt tattaatagc agacactcta tgcctgtgtg gagtaagaaa 26280
   aaacagtatg ttatgattat aactgttatg cctacttata aaggttacag aatatttttc 26340
   cataattttc ttgtatagca gtgcagcttt ttcctttgtg gtgtaaatag caaagcaagc 26400
   aagagttcta ttactaaaca cagcatgact caaaaaactt agcaattctg aaggaaagtc 26460
   cttggggtct tctacctttc tcttcttttt tggaggagta gaatgttgag agtcagcagt 26520
   agcctcatca tcactagatg gcatttcttc tgagcaaaac aggttttcct cattaaaggc 26580
   attccaccac tgctcccatt catcagttcc ataggttgga atctaaaata cacaaacaat 26640
   tagaatcagt agtttaacac attatacact taaaaatttt atatttacct tagagcttta 26700
   aatctctgta ggtagtttgt ccaattatgt cacaccacag aagtaaggtt ccttcacaaa 26760
   gatccggacc aaagcggcca tcgtgcctcc ccactcctgc agttcggggg catggatgcg 26820
   cggatagccg ctgctggttt cctggatgcc gacggatttg cactgccggt agaactccgc 26880
   gaggtcgtcc agcctcaggc agcagctgaa ccaactcgcg aggggatcga gcccggggtg 26940
   ggcgaagaac tccagcatga gatccccgcg ctggaggatc atccagccgg cgtcccggaa 27000
   aacgattccg aagcccaacc tttcatagaa ggcggcggtg gaatcgaaat ctcgtgatgg 27060
   caggttgggc gtcgcttggt cggtcatttc gaaccccaga gtcccgctca gaagaactcg 27120
   tcaagaaggc gatagaaggc gatgcgctgc gaatcgggag cggcgatacc gtaaagcacg 27180
   aggaagcggt cagcccattc gccgccaagc tcttcagcaa tatcacgggt agccaacgct 27240
   atgtcctgat agcggtccgc cacacccagc cggccacagt cgatgaatcc agaaaagcgg 27300
   ccattttcca ccatgatatt cggcaagcag gcatcgccat gggtcacgac gagatcctcg 27360
   ccgtcgggca tgcgcgcctt gagcctggcg aacagttcgg ctggcgcgag cccctgatgc 27420
   tcttcgtcca gatcatcctg atcgacaaga ccggcttcca tccgagtacg tgctcgctcg 27480
   atgcgatgtt tcgcttggtg gtcgaatggg caggtagccg gatcaagcgt atgcagccgc 27540
   cgcattgcat cagccatgat ggatactttc tcggcaggag caaggtgaga tgacaggaga 27600
   tcctgccccg gcacttcgcc caatagcagc cagtcccttc ccgcttcagt gacaacgtcg 27660
   agcacagctg cgcaaggaac gcccgtcgtg gccagccacg atagccgcgc tgcctcgtcc 27720
   tgcagttcat tcagggcacc ggacaggtcg gtcttgacaa aaagaaccgg gcgcccctgc 27780
   gctgacagcc ggaacacggc ggcatcagag cagccgattg tctgttgtgc ccagtcatag 27840
   ccgaatagcc tctccaccca agcggccgga gaacctgcgt gcaatccatc ttgttcaatc 27900
   atgcgaaacg atcctcatcc tgtctcttga tcagatcttg atcccctgcg ccatcagatc 27960
   cttggcggca agaaagccat ccagtttact ttgcagggct tcccaacctt accagagggc 28020
   gccccagctg gcaattccgg ttcgcttgct gtccataaaa ccgcccagtc tagctatcgc 28080
   catgtaagcc cactgcaagc tacctgcttt ctctttgcgc ttgcgttttc ccttgtccag 28140
   atagcccagt agctgacatt catccggggt cagcaccgtt tctgcggact ggctttctac 28200
   gtgttccgct tcctttagca gcccttgcgc cctgagtgct tgcggcagcg tgaagctttt 28260
   tgcaaaagcc taggcctcca aaaaagcctc ctcactactt ctggaatagc tcagaggccg 28320
   aggcggcctc ggcctctgca taaataaaaa aaattagtca gccatggggc ggagaatggg 28380
   cggaactggg cggagttagg ggcgggatgg gcggagttag gggcgggact atggttgctg 28440
   actaattgag atgcatgctt tgcatacttc tgcctgctgg ggagcctggg gactttccac 28500
   acctggttgc tgactaattg agatgcatgc tttgcatact tctgcctgct ggggagcctg 28560
   gggactttcc acaccctaac tgacacacat tccacagccg gatctgcagg acccaacgct 28620
   gcccgagatg cgccgcgtgc ggctgctgga gatggcggac gcgatggata tgttctgcca 28680
   agggttggtt tgcgcattca cagttctccg caagaattga ttggctccaa ttcttggagt 28740
   ggtgaatccg ttagcgaggt gccgccggct tccattcagg tcgaggtggc ccggctccat 28800
   gcaccgcgac gcaacgcggg gaggcagaca aggtataggg cggcgcctac aatccatgcc 28860
   aacccgttcc atgtgctcgc cgaggcggca taaatcgccg tgacgatcag cggtccaatg 28920
   atcgaagtta ggctggtaag agccgcgagc gatccttgaa gctgtccctg atggtcgtca 28980
   tctacctgcc tggacagcat ggcctgcaac gcgggcatcc cgatgccgcc ggaagcgaga 29040
   agaatcataa tggggaaggc catccagcct cgcgtcgcga acgccagcaa gacgtagccc 29100
   agcgcgtcgg ccgccatgcc ggcgataatg gcctgcttct cgccgaaacg tttggtggcg 29160
   ggaccagtga cgaaggcttg agcgagggcg tgcaagattc cgaataccgc aagcgacagg 29220
   ccgatcatcg tcgcgctcca gcgaaagcgg tcctcgccga aaatgaccca gagcgctgcc 29280
   ggcacctgtc ctacgagttg catgataaag aagacagtca taagtgcggc gacgatagtc 29340
   atgccccgcg cccaccggaa ggagctgact gggttgaagg ctctcaaggg catcggtcga 29400
   ggaactttcg gcggctttgc tgtgcgacag gctcacgtct aaaaggaaat aaatcatggg 29460
   tcataaaaat tatcacgttg tcggcgcggc gacggatgtt ctgtatgcgc tgttttccgt 29520
   tggccgttgc tgtctggtga tctgccttct aaatctgcac agccgaattg cgcgagcttg 29580
   gttttgctga aaccgacaca cagcaactga ataccagaaa gaaaatcact ttgcctttct 29640
   gacatcagaa gggcagaaat ttgccgttga acacctggtc aatacgcgtt ttggtgagca 29700
   gcaatattgc gcttcgatga gccttggcgt tgagattgat acctctgctg cacaaaaggc 29760
   aatcgaccga gctggaccag cgcattcgtg acaccgtctc cttcgaactt attcgcaatg 29820
   gagtgtcatt catcaaggac ngcctgatcg caaatggtgc tatccacgca gcggcaatcg 29880
   aaaaccctca gccggtgacc aatatctaca acatcagcct tggtatcctg cgtgatgagc 29940
   cagcgcagaa caaggtaacc gtcagtgccg ataagttcaa agttaaacct ggtgttgata 30000
   ccaacattga aacgttgatc gaaaacgcgc tgaaaaacgc tgctgaatgt gcggcgctgg 30060
   atgtcacaaa gcaaatggca gcagacaaga aagcgatgga tgaactggct tcctatgtcc 30120
   gcacggccat catgatggaa tgtttccccg gtggtgttat ctggcagcag tgccgtcgat 30180
   agtatgcaat tgataattat tatcatttgc gggtcctttc cggcgatccg ccttgttacg 30240
   gggcggcgac ctcgcgggtt ttcgctattt atgaaaattt tccggtttaa ggcgtttccg 30300
   ttcttcttcg tcataactta atgtttttat ttaaaatacc ctctgaaaag aaaggaaacg 30360
   acaggtgctg aaagcgagct ttttggcctc tgtcgtttcc tttctctgtt tttgtccgtg 30420
   gaatgaacaa tggaagtcaa caaaaagcag acgtatctag acacgtctga agctagcttc 30480
   gaggaacttt cggcggcttt gctgtgcgac aggctcacgt ctaaaaggaa ataaatcatg 30540
   ggtcataaaa attatcacgt tgtcggcgcg gcgacggatg ttctgtatgc gctgttttcc 30600
   gttggccgtt gctgtctggt gatctgcctt ctaaatctgc acagccgaat tgcgcgagct 30660
   tggttttgct gaaaccgaca cacagcaact gaataccaga aagaaaatca ctttgccttt 30720
   ctgacatcag aagggcagaa atttgccgtt gaacacctgg tcaatacgcg ttttggtgag 30780
   cagcaatatt gcgcttcgat gagccttggc gttgagattg atacctctgc tgcacaaaag 30840
   gcaatcgacc gagctggacc agcgcattcg tgacaccgtc tccttcgaac ttattcgcaa 30900
   tggagtgtca ttcatcaagg acngcctgat cgcaaatggt gctatccacg cagcggcaat 30960
   cgaaaaccct cagccggtga ccaatatcta caacatcagc cttggtatcc tgcgtgatga 31020
   gccagcgcag aacaaggtaa ccgtcagtgc cgataagttc aaagttaaac ctggtgttga 31080
   taccaacatt gaaacgttga tcgaaaacgc gctgaaaaac gctgctgaat gtgcggcgct 31140
   ggatgtcaca aagcaaatgg cagcagacaa gaaagcgatg gatgaactgg cttcctatgt 31200
   ccgcacggcc atcatgatgg aatgtttccc cggtggtgtt atctggcagc agtgccgtcg 31260
   atagtatgca attgataatt attatcattt gcgggtcctt tccggcgatc cgccttgtta 31320
   cggggcggcg acctcgcggg ttttcgctat ttatgaaaat tttccggttt aaggcgtttc 31380
   cgttcttctt cgtcataact taatgttttt atttaaaata ccctctgaaa agaaaggaaa 31440
   cgacaggtgc tgaaagcgag ctttttggcc tctgtcgttt cctttctctg tttttgtccg 31500
   tggaatgaac aatggaagtc aacaaaaagc agagcttatc gtgataagcg gtcaaacatg 31560
   agaattc 31567
<210> 7
   <211> 26621
   <212> DNA
   <213> artificial sequence
<220>
   <223> synthetic polynucleotide
<220>
   <221> misc_feature
   <222> (24889)..(24889)
   <223> n is a, c, g, or t
<220>
   <221> misc_feature
   <222> (25971)..(25971)
   <223> n is a, c, g, or t
<400> 7
   artctcgact ctagagggac agcccccccc caaagccccc agggatgtaa ttacgtccct      60
   cccccgctag gggcagcagc gagccgcccg gggctccgct ccggtccggc gctccccccg     120
   catccccgag ccggcagcgt gcggggacag cccgggcacg gggaaggtgg cacgggatcg     180
   ctttcctctg aacgcttctc gctgctcttt gagcctgcag acacctgggg ggatacgggg     240
   aaaaagcttt aggctgaaag agagatttag aatgacagaa tcatagaacg gcctgggttg     300
   caaaggagca cagtgctcat ccagatccaa ccccctgcta tgtgcagggt catcaaccag     360
   cagcccaggc tgcccagagc cacatccagc ctggccttga atgcctgcag ggatggggca     420
   tccacagcct ccttgggcaa cctgttcagt gcgtcaccac cctctggggg aaaaactgcc     480
   tcctcatatc caacccaaac ctcccctgtc tcagtgtaaa gccattcccc cttgtcctat     540
   caagggggag tttgctgtga cattgttggt ctggggtgac acatgtttgc caattcagtg     600
   catcacggag aggcagatct tggggataag gaagtgcagg acagcatgga cgtgggacat 660
   gcaggtgttg agggctctgg gacactctcc aagtcacagc gttcagaaca gccttaagga 720
   taagaagata ggatagaagg acaaagagca agttaaaacc cagcatggag aggagcacaa 780
   aaaggccaca gacactgctg gtccctgtgt ctgagcctgc atgtttgatg gtgtctggat 840
   gcaagcagaa ggggtggaag agcttgcctg gagagataca gctgggtcag taggactggg 900
   acaggcagct ggagaattgc catgtagatg ttcatacaat cgtcaaatca tgaaggctgg 960
   aaagcctcca agatccccaa gaccaacccc aacccaccca ccgtgcccac tggccatgtc 1020
   cctcagtgcc acatccccac agttcttcat cacctccagg gacggtgacc cccccacctc 1080
   cgtgggcagc tgtgccactg cagcaccgct ctttggagaa ggtaaatctt gctaaatcca 1140
   gcccgaccct cccctggcac aacgtaaggc cattatctct catccaactc caggacggag 1200
   tcagtgagga tggggctcta gagggacagc ccccccccaa agcccccagg gatgtaatta 1260
   cgtccctccc ccgctagggg cagcagcgag ccgcccgggg ctccgctccg gtccggcgct 1320
   ccccccgcat ccccgagccg gcagcgtgcg gggacagccc gggcacgggg aaggtggcac 1380
   gggatcgctt tcctctgaac gcttctcgct gctctttgag cctgcagaca cctgggggga 1440
   tacggggaaa aagctttagg ctgaaagaga gatttagaat gacagaatca tagaacggcc 1500
   tgggttgcaa aggagcacag tgctcatcca gatccaaccc cctgctatgt gcagggtcat 1560
   caaccagcag cccaggctgc ccagagccac atccagcctg gccttgaatg cctgcaggga 1620
   tggggcatcc acagcctcct tgggcaacct gttcagtgcg tcaccaccct ctgggggaaa 1680
   aactgcctcc tcatatccaa cccaaacctc ccctgtctca gtgtaaagcc attccccctt 1740
   gtcctatcaa gggggagttt gctgtgacat tgttggtctg gggtgacaca tgtttgccaa 1800
   ttcagtgcat cacggagagg cagatcttgg ggataaggaa gtgcaggaca gcatggacgt 1860
   gggacatgca ggtgttgagg gctctgggac actctccaag tcacagcgtt cagaacagcc 1920
   ttaaggataa gaagatagga tagaaggaca aagagcaagt taaaacccag catggagagg 1980
   agcacaaaaa ggccacagac actgctggtc cctgtgtctg agcctgcatg tttgatggtg 2040
   tctggatgca agcagaaggg gtggaagagc ttgcctggag agatacagct gggtcagtag 2100
   gactgggaca ggcagctgga gaattgccat gtagatgttc atacaatcgt caaatcatga 2160
   aggctggaaa gcctccaaga tccccaagac caaccccaac ccacccaccg tgcccactgg 2220
   ccatgtccct cagtgccaca tccccacagt tcttcatcac ctccagggac ggtgaccccc 2280
   ccacctccgt gggcagctgt gccactgcag caccgctctt tggagaaggt aaatcttgct 2340
   aaatccagcc cgaccctccc ctggcacaac gtaaggccat tatctctcat ccaactccag 2400
   gaacggagtc agtgaggatg gggctctaga ggatccctcg acctgcaggt caacggatca 2460
   caacaaactg gaaaattctt caagagaaga ataccagacc accctacctg cttcctgaga 2520
   aatctgtttg ctgctcagaa gcaacagtta gaaccagaca tggaacaaca gactggttcc 2580
   aaatcaggaa aggagtatgt caaggctgta tatcgtcacc ctgattattt aacttatatg 2640
   catagtacat aatacaaaat gccaggctgg atgaatcgca agctggaatc aagatttctg 2700
   ggagaaatat caataaacga gatacaaaga tacaccacac ttatggcaga aaactaagaa 2760
   gaactaaaga gcctcttgat gaaagtgaaa gaggagagtg aaaaagccag cttaaaaccc 2820
   aacattcaaa atcaagatca tcatttcatg gcaaataaat ggggaaacaa tggaaacagt 2880
   gagagacttt attttcttgg gctccaaaat cactgcagat tgtgactaca gccatgatta 2940
   aaagatgctt gctccttgga agagaagcta ttaccaaact agaaagcata ttaaaaagca 3000
   gagacgttac tttgctgact aagttctgtc tagtcaaacc tatggttttt ccagtagtca 3060
   tatatggatg tgagttgaac tataaagaaa gctgagcacc aaagaattga tgcttttgaa 3120
   atttggtgtt ggagaagtct cttgagagtc ccttgaacct gcaaggagat ccaaccagtc 3180
   catcctaaag gaaatcagtc ctgaatattc attggaagga ctgatgctga aattgaagat 3240
   taacgttttg gactcaccta atgcagaaga gccaactcac tagaaaagac cccatgttgg 3300
   caaaaattga agccaggaag agaagtgaat gacagaggat gagatggttg gatggcatcg 3360
   ttgactgaat ggacatgagt ctgatcaagt tccgggagac agcaaaggac agggctgcct 3420
   ggtctgctgc agtccatggg gttgcaaaga gtcggtctca aatgagtaac taaacaacaa 3480
   ccaagcagta gaaaaataaa taaaatttgt ctctgagatc tcagtacctc tttctgtgca 3540
   tatccgtctc ctgttattgt actttgtctt ctgcttgtaa taaagctgtc ctgttagtaa 3600
   aatctgtttg ggtcctctga attcttttag ctatcaaaaa tggaaggtga ttattgtgca 3660
   atgtccacct ctgagtaata tacagagaat aaaagaaggg agaaattatg tgcaagttct 3720
   ctctcatctc ctgcttctca tttaaaagat tctacctcag tgggggctaa aactccacat 3780
   ttaacagtag caaaaaccaa tattccatag cttcttagga aaccattttt tatactcttg 3840
   tatgtaatta cattcaagct caaaagcaaa gaagtgattc tgcgttggtg aaggcccaac 3900
   catagaaaag aggaagaaaa taggccacat actgtgcttc ccccatagct cagttggtaa 3960
   agaatctacc tacaatgcag gaggcctggg cttgatccct gggtaaggga gatcccctgg 4020
   agaaggaaat ggtaacccac tccagtactc ttgcctgtaa atcccatgga cggaggagcc 4080
   tggcagctac agccttgggg tggcaagagt tggacatgat taacaactaa accactgcca 4140
   ccactccaca tactgagtgc tccccagtgg cactagtggt aaagaaccac ctgccggtgc 4200
   agaagacatt aaagacactg gctctatccc tgcttgggaa gtagggaaga tcccctagag 4260
   agggaaatag caacccactc cagaattctt gcctggaaaa tcccatgaat gaagactggc 4320
   gggctgtagt aactggggtc acaaagagtt aaacatgatt tagcaactaa acatcaccac 4380
   attaaaaaaa ttaccaccaa aatagtcata ttccaggcta aggggaataa tagcactagt 4440
   acctgagaga actttctcag attctctgtc aagttcttcc ttctctcata taaccagtag 4500
   tctagtttac ctcatcagat attaactact catcgattct aaattatcta attatggggg 4560
   ggggcactac attgcattat attttgtgtc cattgactat cactcaattt atttataaaa 4620
   aattcatcca tgttgtttct gtgacagtaa ctcattcaca ttaattgtaa tatctcattg 4680
   cattgtatac tacaatttat ttatacaaaa tactattatt cacacttctg ttgattttaa 4740
   tttggaacat caacaataac gtggctgaga agcttctttc tttagtatat tgttaaggat 4800
   ttccttgatc aagattttac ctacttttct ggtccaattg gtgagagaca gtcataagga 4860
   aatgctgtgt ttattgcaca atatgtaaag catcttcctg agaaaataaa agggaaatgt 4920
   tgaatgggaa ggatatgctt tcttttgtat tccttttctg agaaatcaga ctttttcacc 4980
   ttggccttgg ccaccaaaag ctaacaaata aaggcatatg aagtagccaa ggccttttct 5040
   agttatatct atgacactga gttcatttca tcatttattt tcctgacttc ctcctgggtc 5100
   catatgagca gtcttagaat gaatattagc tgaataatcc aaatacatag tagatgttga 5160
   tttgggtttt ctaagcaatc caagacttgt atgacagtaa gatgtattac catccaacac 5220
   acatctcagc atgatataaa tgcaaggtat attgtgaaga aaaattttta attatgtcaa 5280
   agtgcttact ttagaaggtc atctatctgt cccaaagctg tgaatatata tattgaaggt 5340
   aatgaataga tgaagctaac cttgtaaaaa tgagtagtgt gaaatacaac tacaattatg 5400
   aacatctgtc actaaagagg caaagaaact tgaagattgc ttttgcaaat gggctcctat 5460
   taataaaaag tacttttgag gtctggctca gactctattg tagtacttag ggtaagaccc 5520
   tcctcctgta tgggctttca ttttctttct tgcttccctc atttgccctt ccatgaatac 5580
   tagctgataa acattgacta taaaagatat gaggccaaac ttgagctgtc ccattttaat 5640
   aaatctgtat aaataatatt tgttctacaa aagtattatc taaataaatg ttactttctg 5700
   tcttaaaatc cctcaacaaa tccccactat ctagagaata agattgacat tccctggaat 5760
   cacagcatgc tttgtctgcc attatctgac ccctttctct ttctctcttc tcacctccat 5820
   ctactccttt ttccttgcaa ttcatgaccc agattcactg tttgatttgg cttgcatgtg 5880
   tgtgtgctga gttgcgtctg actgttatca accccatgaa tgatagtcca ccaggctcta 5940
   ctgtccatga aattttccag tcaagaatac tggagtggat tgcatttcct actccatttg 6000
   attaatttag tgacttttaa atttcttttt ccatattcgg gagcctattc ttccttttta 6060
   gtctatactc tcttcactct tcaggtctaa ggtatcatcg tgtgcttgtt agcttgttac 6120
   tttctccatt atagcttaag cactaacaac tgttcaggtt ggcatgaaat tgtgttcttt 6180
   gtgtggcctg tatatttctg ttgtgtatta gaatttaccc caagatctca aagacccact 6240
   gaatactaaa gagacctcat tgtggttaca ataatttggg gactgggcca aaacttccgt 6300
   gcatcccagc caagatctgt agctactgga caatttcatt tcctttatca gattgtgagt 6360
   tattcctgtt aaaatgctcc ccagaatttc tggggacaga aaaataggaa gaattcattt 6420
   cctaatcatg cagatttcta ggaattcaaa tccactgttg gttttatttc aaaccacaaa 6480
   attagcatgc cattaaatac tatatataaa cagccactaa atcagatcat tatccattca 6540
   gcttctcctt cacttcttct cctctacttt ggaaaaaagg taagaatctc agatataatt 6600
   tcagtgtatc tgctactcat ctttattttg gactaggtta aaatgtagaa agaacataat 6660
   tgcttaaaat agatcttaaa aataagggtg tttaagataa ggtttacact attttcagca 6720
   gatatgttaa aaaatagaag tgactataaa tacttgataa aaattatagt gactgcaaat 6780
   gttttaggaa tataataaga tataataaca gtggttgcta ttttctttag cacaagacta 6840
   gttaacaggc tgtattaaaa gatcttttct tgaattaaat attttcaatt tgattaaacc 6900
   tacctcagcc ataaaggcaa gcacatttca tttatactat ggggatttga ataattatta 6960
   ctgaagaagc tctaccaaca aaaagtttat agagctatca tatttagtca agagataaag 7020
   agggttgtta ggatatatat gctatttgaa aggtatttat aaaagaagag tatatttatc 7080
   aaaatttctc aagaacatcc aaatttcaag tttatcattt atcttacaat atttcaaaaa 7140
   tattaaaata gatacatgaa atacagaagt aaattaaaga gaaagtattt tacttggtaa 7200
   aaaaattcta ggttggacag agagtgccag gaaacaaaaa caatgaaaaa tgtgacctga 7260
   caggaattat agctcaaagt atagtagtaa gtaatgaaat ggcttaaaaa ttggtatata 7320
   aaatgctagt tataaaataa acaaaatgca ataatatcct ccctacatgt aatgaattct 7380
   aggtattatg attatgctct tttttgaagt cttgacaata aaaatttttt tagaagttta 7440
   taggcatctt gaataaagtg aaacaaatta agaattagta tccatgagaa aaatatagaa 7500
   caattttcct aatttagttt gaaaatctgg gattgaagat gtgtgtcaag agatgttggt 7560
   ggcaagaaca tttttttttc aagaacttat aaaaatgcaa caaaacaaac catttaatac 7620
   attttggtca aaatcaataa tgtattttat tttatgctcc aaggagcata aaattgggga 7680
   ctgggcaaga gaaactgaca ccctggtaaa ttaccaagag ataagtacac agttactata 7740
   gtagaaaata agcatagtgt atgatctcta aaattatgtg agacaaagga gagatgacat 7800
   taggcatgtg gggatgaaga ctgagtagag aagaaacaat ctaatcagtc caagaaaaca 7860
   tctcgatcag tggaacaaat agaagaaatg ctaaaatgaa acagaagtct tactggaaat 7920
   aaaagatatg cataagacaa aaattcatga aaatcactta gtttagcaga gaaaagataa 7980
   aaataaagta tgaccttctt catatacatt gtttgatcat atgcacctca ataaaactga 8040
   gtctccaaca gaaatgaaac attaatattt tgttcactgc tctaatccca gaatctaagc 8100
   gatatctggc aataaaaata ataaatatat attttttaat aaatgaatca accacttaat 8160
   ttttctgtaa atatctgtaa cttctcttct gtctttccaa aaacactcat aagtactgtg 8220
   aatgagatga aaaagagtga agtaggatat aggctgttag cagaaaacat ctgaatggct 8280
   ggcagtgaaa cattaacttg aaatgtaaga ttaatgagta atagtaaatt ttaaccttgg 8340
   ccatatgata aaatgttcat taatattttt ctagaataca gggctttttg tttttgccat 8400
   gaggtttgca ggatcttggt tccctgacca gggatcaaac ctgcacacca gggatcaaac 8460
   ctgcactccc ctggaagcat ggagtcttgg acatttgtat tatacactat ctttggttcc 8520
   ttttaaaggg aagtaatttt acttaaataa gaaaatagat tgacaagtaa tacgctgttt 8580
   cctcatcttc ccattcacag gaatcgcgga tcctcgagac cgccatggcc gtgctgggcc 8640
   tgctgttctg cctggtgacc ttccccagct gcgtgctgtc ccaggtgcag ctgaagcaga 8700
   gcggccctgg cctggtgcag ccctctcagt ccctctccat tacctgtacc gtgtccggct 8760
   tctctctcac aaattatgga gtccattggg tgcgccagag ccccggcaag ggcctggagt 8820
   ggctgggcgt gatctggtcc ggcggcaaca ccgactacaa cacccccttc accagcagac 8880
   tgagcatcaa caaggacaac agcaagagcc aggtgttctt caagatgaac agcctgcaga 8940
   gcaacgacac cgccatctac tactgcgctc gcgctctcac ctactacgac tacgagttcg 9000
   cctactgggg ccagggcacc ctggtgaccg tgtccgccgc cagcaccaag ggccccagcg 9060
   tgttccccct ggcccccagc agcaagagca ccagcggcgg caccgctgcc ctgggctgtc 9120
   tggtgaagga ctacttcccc gagcccgtga ccgtgtcttg gaactctggc gccctgacct 9180
   ccggcgtgca cacatttcca gctgtcctcc agagttccgg actgtattcc ctcagttctg 9240
   tcgtgacagt cccttcctct agcctgggca cccagaccta catctgcaac gtgaaccaca 9300
   agcccagcaa caccaaggtg gacaagagag tggagcccaa gagctgcgac aagacccaca 9360
   cctgcccacc atgtcctgct ccagagctgc tggggggacc ctccgtgttc ctgttccccc 9420
   ccaagcccaa ggacaccctg atgatcagca ggacccccga ggtgacctgc gtggtggtgg 9480
   acgtgtccca cgaggaccct gaggtgaagt tcaactggta cgtggacggc gtggaggtgc 9540
   acaacgccaa gaccaagccc agagaggagc agtacaacag cacctacagg gtggtgtccg 9600
   tgctgaccgt gctgcaccag gactggctga acggcaaaga atacaagtgc aaagtctcca 9660
   acaaggccct gccagccccc atcgaaaaga ccatcagcaa ggccaagggc cagcctcgcg 9720
   agccccaggt gtacaccctg cccccctccc gcgaggagat gaccaagaac caggtgtccc 9780
   tgacctgcct ggtgaagggc ttctacccca gcgatatcgc cgtggagtgg gagagcaacg 9840
   gccagcccga gaacaactac aagaccaccc cccctgtgct ggacagcgac ggcagcttct 9900
   tcctgtacag caagctgacc gtggacaagt ccaggtggca gcagggaaat gtcttttctt 9960
   gttctgtcat gcatgaagct ctgcacaacc actacaccca gaagtccctg agcctgtccc 10020
   ccggcaagtg atagctcgag gatccggacc cttccctatt cttgtaagtc taaatttact 10080
   aactgtgctg tttaacttct gatgtttgta tgatatttga gtaattaaga gccctacaaa 10140
   aaaatcaata atgaatggtt ccaaaataag catagctgag attaatgatt ctcagcatta 10200
   gttataaata gaataagctg gaaaaccttc acctcccctc caccaccaga tctcaatgtc 10260
   taggcttacc catggagatt ctgattaact gttctttcta tgtagaagaa acttattggg 10320
   aagaaataat ataatggact atgatttaat tggtctgttg agaatttaga tgaaggggat 10380
   taagttacaa taaagccaga atttaacttg ataatctcat ttggctaaga ataacaaacc 10440
   taagaaggtt tgctattttc tacaattttg aagttttcct tatgcacaat tatttcacca 10500
   catgactcat ttcacatctt gtttttgata tatgagcata tgagggcaaa atactgaaga 10560
   tgcttatttc aatactcagg gaaaattttc ttgccaaaag gcaagaattg tataattcat 10620
   tcacttattt tatttttttt aatttttaag gtctaagagg atttcaaagt gaatgccccc 10680
   tcctcacttt tggtaagctt taggagattg gaggcagact gatcattttt atagttaata 10740
   tcttttacat ttcatcttcc tggataagcc ccaatagtag caatttctat cagtatacca 10800
   gcataaagat tagttttaaa tttattttca gtgattgact gttatttact gacctgaaat 10860
   tatgtatctg ttatatttca aataatgcaa aactgtatat atatggtgtt gacagatttg 10920
   attggttttc tttcaattgc ctatatcctt attattgatt gtaatcattt atagaaaaaa 10980
   caaaataatt tcttatactt ttatgtaaac ctgttagagc ttattttaaa gatcaactgc 11040
   attcacattt ctaatctagt cattatgagc ttcaattgtt ttatctcact taaaatttat 11100
   atattgtctt ttaattcatg agtcaaaata caatctcaca gtccagatat gggacttaaa 11160
   aggggaatag catatagttt tgatattctt aaagatatac atctttttgt gatcatgatt 11220
   cagcagacat tttaataaaa caattccaag tgagccgaca cttggtccta gaggaatttt 11280
   tataacctta agataaggca cagcatggtg tttttgtaat aagatttctt ttatgaaaaa 11340
   gtcacaccaa aattggaaat ggggtgagat gaagagttat aacatataac taaatggaca 11400
   tttgttctct attccacaga attgactgcg actggaaata tggcaacttt tcaatccttg 11460
   catcatgcta ctaagataat ttttaaatga gtatacatgg aacaaaaaat gaaactttat 11520
   tcctttattt atattatgct ttttcatctt aatttgaatt tgagtcataa accatatact 11580
   ttcaaaatgt taattcaaca ttagcataaa agttcaattt taacttggaa atatcatgaa 11640
   catatcaaat tatgtataaa aataatttct ggaattgtga ttattatttc tttaagaatc 11700
   tatttcctaa ccagtcattt caataaatta acccttaggc atatttaagt tttcttgtct 11760
   ttattatatt tttaaaaatg aaattggtct ctttattgtt aacttaaatt tatctttgat 11820
   gttaaaaata gctgtggaaa attaaaattg aatagaattc tttgaattga gttccaaagg 11880
   atatcaaaaa gtgagggaaa agatagggtg agcctatgct gcatatgtcc ttagaaagtc 11940
   ttggtttata cctgttacct aagttaaaca attatacttg ttcctttcac tctcgaaagt 12000
   acccagcatt ggatgttaaa ttttatagtc atcctagaca aaaaaaaaaa aaaaaacaaa 12060
   caaccctcaa atgtgatatc tgaatcacag ctctacagtg tggtagctaa gtggtgctgt 12120
   gtaagttagt ctccaagaga ttccatttct acatttataa acagtcaatt taaggtgttt 12180
   tattgaagtt ttaatgtgaa aagtgcacta tatggtgcat gataggagtt cctggttgaa 12240
   tctcatttct gacatcactg acaccagtgc agcaaggact agtgttacaa tcagaaggag 12300
   ctgagttgtg taattttagc cattaatgcc caagagacta gaacttacac aaagctctaa 12360
   tatccattgt ctctgtctgt ggagtaatta tttcattgcc atgaattatc tgtctgtcat 12420
   atcctgcatt tttatacatg attcagttcc cttcagttca cacaatgact tgtctaattt 12480
   catctttcct gcatcctcca tgttttcctc acttcaggat taagtgaagc cgtacttagg 12540
   cacaatattt cttatcttta aagaaaaatt ccatctttga gagttgttat tgttcagtca 12600
   ctaggtcatg tccaactctt tgtgacccca tgcactgcag catgccaggc ttccctgccc 12660
   ttcgctctct cctggagttt gctcagactc atgtagattg agtcggtgat ggtatccaac 12720
   tatctcatca actgttgtgc ccttctcctc ctaccctcag tctttaccag catcagagtc 12780
   tttctcagat tcttcaggtt attatataac aactatcata aaaggagtat ctaaatggct 12840
   gtgtccatta tttcacatgt tattctctct ttaacttgct ccaatcccaa ttttatccct 12900
   atgggaactg ctttattgaa gatcaccaac aacttttatt ttactaatcg ttttgtttta 12960
   cccaacctct cagtgagtgt tatgaggtag agttgactat ttcttcattt tgaaatatta 13020
   cgcttcattt catttgatat cctaaagctc ataaggtgtg gtttttctct taactcacta 13080
   gacacttctt tgaagtctct cttctggcat tttctccttt tccaaaattt taatggttgg 13140
   agtaccctag attttagcct taatttgttt gatgttgttc agttccattc tcagctcaga 13200
   gcttccaact gtatgtctcc aaacttactc gttttgtaaa ctccaaactc atgcactcaa 13260
   ctgcattctt gacctccaca ctgaattatc taattaatgt cctaaatctg gcatgaccaa 13320
   gcatacattt ttgtctgaat ccagtcccca acttgctcaa aatttaatta aacgtaattc 13380
   agttacaaag gcagctgata ttgtatgcaa tagacctgaa tgggaacttc acaaaagaag 13440
   ttatcttaat tgtcaataaa aacatgaaaa atactctaca tcatcaatct tcagaaaaat 13500
   gcaaattaaa ggtgcctaat aatatcatga cacaaccgtc agaatgactg aaatgaaaag 13560
   aattgtaata cagttcagtt cagttcagtt actcagtcgt ctccaactct ttgtgacccc 13620
   atgaactgca gcatgacaga ccttcctgtc catcaccaac tcccagagtt tactcagact 13680
   atgtccattg agttgatgat gccatccaac catctcatcc tctgtcgtcc ccttctcctc 13740
   ctgccctcag tctttcccag catcagggtc ttttccaatg agtcagctct tcgcatcagg 13800
   tggctaaagt attggagttt cagcttcaac atcagtcctt ctaattaaca cccaggactg 13860
   atctctttta ggatggacta gttggatctc cttgcagtcc aagggactct caagagtctt 13920
   ctccaacacc acagttcaaa agcatcaatt ccttggcact cagctttcct tatagtccat 13980
   gtctcacatc cacacatgac tattggaaaa accatagcct tgactaggtg gacctttgtt 14040
   gacaaagtaa tgtctctgct ttttaatatg ttgtctagat tggtcataac tttccttcca 14100
   agaagtaatt gtcttttaat ttcatggctg cagtcaccat ctgcagtgat tttggagccc 14160
   caaaatataa agtcagctgc tgtttccact gttgccccat ctaccccatc tatttgccat 14220
   gaagtgatgg gactggatgc cactatctta gttttctgaa tgttgagctt taagccagcc 14280
   tttttactct cctctttcac tttcatcaag aggctcttta gttcctcttc actttctgcc 14340
   ataagggtgg tgtcatctgc atatctgagg ttattgatat ttctcttggc aattttgatt 14400
   ccagcctgca cttcttccag cccagtgttt ctcatgatgt actctgcata taaattaaat 14460
   aagcagagtg acaatataca gccttgacat actctttttc ctatttggaa ccagtctgtt 14520
   gttccatgtc cagttctaac tgttgtttcc tgacctgcat acaggtttct caagaggcaa 14580
   gtcaggtggt ctggtattct cacctgtttc agaattttcc acagtttatt gtgatccaca 14640
   cagtcaaagg ctttggcata gccaataaag cagaaagaga tgtttttctg gaactctctt 14700
   acttttttga tgatccagtg gatgttggca atttgatctc tggttcctct gccttttcta 14760
   aaaccagctt taacatctgg aagttcatgg ttcacgtaat acaaaatgta atacaaaatg 14820
   tctgcaaaaa caaaggaatg aaaagtaatg ctaaaaaatg ttaatattta cagaaatttt 14880
   tatagtagta aagaattcac ctgcaataca ggagaaccgg gttagatccc tgggttggaa 14940
   gacctcctgg agaaggaaat ggctacccaa tctagtattc ttgtctggag aaggcaagaa 15000
   tggacagaga agcccagcgg gctatggtcc atcgggtcac aaagagtcag aagctacctt 15060
   gcacacagca agcacggtgc gcgcgcgtgc acacacacac acacacacac acacagacac 15120
   acacacactc taaaacattt acccaagctt gtccaatgga aaatcaaaaa gccagcaatt 15180
   taagatgaca tcaggtacca ctgtccaggt aagcctcaga acacaatgac cagtaagaag 15240
   caaagtgcca tatgagcaac tcgaattttt gcaatgttac ctaagagctt ccatttttat 15300
   aatgcaaaag aatttcatat ggggaaattg tattagataa ccctgaatga ggagcaagat 15360
   atagtcaaag taagatgctc tagtactatt ttttataagc atgatttgtt cagccaaagg 15420
   tttttcccca tatggccaat gaactgaaat atgcagtcct gagatttgca tatatttcta 15480
   gctgaaacca agtaaataat atcctcaaga aagaaatcaa tagaaaagtt ggatgaagag 15540
   tacaataaag ggaccaaaaa tattcagaaa taagaactag aggagatatt gggaaatccc 15600
   tggtgagtcc agtttaggat tttgtacttt cactgcagtt ggcatggata taatccctca 15660
   ctggggaact aagatcccat aagctgtgtt ggattgccaa aaaaataaat attaagagat 15720
   atcattcata gaatatttta aagatatttt agagaagagg aaattaagga tgtgagaatt 15780
   tgtattactt tttcaagata ctaaagctat ttagagatag agctgttact aaaaacttca 15840
   gtttcctaaa aattatttga agcactgttt aataaattcc aaaatataga ggaaggaaaa 15900
   caaaatactg aggattcata taatgattca gatttagaaa caatataaca cagaattagt 15960
   gaattctgac aaattattag gtaggagtag atagttcagc attactcgta tagatggagt 16020
   atttaatcct ttccatgaga ttatccaaat ataataattt cgtatctatg tgaagtataa 16080
   ctattaagat tactttataa agtaaatcaa gaaccagaga ataagaaaaa tgttttgtga 16140
   accagcagat actatgaaca cataaaactc agaaccctga ttcctaagac acacagctaa 16200
   tcctgattat tcttccttta catgtgacca tagaacttca cacaagttca agatacattt 16260
   gttgagcaca tcagtatcag ttcagtcact cagtcatgtc cgaatctttg tgaccttgtg 16320
   gactgcagca cgccaggctt tcctgtccac caccaacccc tggagcttac tcaaactcat 16380
   gtccattgag tcagtgatcc catccaacca tctcatcctc tgtcatcctc ttctcctgcc 16440
   ttcaatcttt cccagacatt ggagtctttt ccaatgagtc agatcttcac attaggtggc 16500
   caaagtatag gagtttcagc ttcagcatca atccttccaa tgaatattcc ttgatgtacc 16560
   cctttcgcag tttggaacca gtctgttgtt ccatgtccag ttctaactgc tgcttctgga 16620
   cctgtataca gatttctcag gaggcaggta aagtggtctg gtattcccat ctcttgaaga 16680
   attttccaca gtttattgtg atccacacaa tcaaaggctt tagcgtagtc aataaagcag 16740
   atgtttttct ggaactctcg tgcttttttg atgatccaat ggatgttggc aatttgatct 16800
   ctggttcctc tgccttttct aaatccagct tgaacatctg gaagttcatg gtccacgtac 16860
   tgttgaagcc tggcttggag aattttgaga gttattttgc tagcatgtga gatgagtgca 16920
   atcatgtggg tgtttgaaca tactttgtca ttgcttttct ttgggattgt ggcagtcctg 16980
   tggccactgc tgagttttcc aaatttgctg acatattgag tgcagcactt tcacagcatc 17040
   accttttaag atttgaaata gctcaactgg aattccatca cctccactag ctttgttcat 17100
   agtgaggctt tctaaggccg tttgactttg cattccaggg tgtctggctc taggtgagtg 17160
   atccgttgac ctgcaggtcg agctcgaagc ggccgctcta gcctcgaggc tagaactagt 17220
   ggatctcgag ccccagctgg ttctttccgc ctcagaagcc atagagccca ccgcatcccc 17280
   agcatgcctg ctattgtctt cccaatcctc ccccttgctg tcctgcccca ccccaccccc 17340
   cagaatagaa tgacacctac tcagacaatg cgatgcaatt tcctcatttt attaggaaag 17400
   gacagtggga gtggcacctt ccagggtcaa ggaaggcacg gggagggggc aaacaacaga 17460
   tggctggcaa ctagaaggca cagtcgaggc tgatcagcga gctctagatc atcgatgcat 17520
   ggggtcgtgc gctcctttcg gtcgggcgct gcgggtcgtg gggcgggcgt caggcaccgg 17580
   gcttgcgggt catgcaccag gtgcgcggtc cttcgggcac ctcgacgtcg gcggtgacgg 17640
   tgaagccgag ccgctcgtag aaggggaggt tgcggggcgc ggaggtctcc aggaaggcgg 17700
   gcaccccggc gcgctcggcc gcctccactc cggggagcac gacggcgctg cccagaccct 17760
   tgccctggtg gtcgggcgag acgccgacgg tggccaggaa ccacgcgggc tccttgggcc 17820
   ggtgcggcgc caggaggcct tccatctgtt gctgcgcggc cagccgggaa ccgctcaact 17880
   cggccatgcg cgggccgatc tcggcgaaca ccgcccccgc ttcgacgctc tccggcgtgg 17940
   tccagaccgc caccgcggcg ccgtcgtccg cgacccacac cttgccgatg tcgagcccga 18000
   cgcgcgtgag gaagagttct tgcagctcgg tgacccgctc gatgtggcgg tccggatcga 18060
   cggtgtggcg cgtggcgggg tagtcggcga acgcggcggc gagggtgcgt acggccctgg 18120
   ggacgtcgtc gcgggtggcg aggcgcaccg tgggcttgta ctcggtcatg gtaagcttca 18180
   gctgctcgag atctagatgg atgcaggtcg aaaggcccgg agatgaggaa gaggagaaca 18240
   gcgcggcaga cgtgcgcttt tgaagcgtgc agaatgccgg gcctccggag gaccttcggg 18300
   cgcccgcccc gcccctgagc ccgcccctga gcccgccccc ggacccaccc cttcccagcc 18360
   tctgagccca gaaagcgaag gagcaaagct gctattggcc gctgccccaa aggcctaccc 18420
   gcttccattg ctcagcggtg ctgtccatct gcacgagact agtgagacgt gctacttcca 18480
   tttgtcacgt cctgcacgac gcgagctgcg ggcggggggg gaacttcctg actaggggag 18540
   gagtagaagg tggcgcgaag gggccaccaa agaacggagc cggttggcgc ctaccggtgg 18600
   atgtggaatg tgtgcgaggc cagaggccac ttgtgtagcg ccaagtgccc agcggggctg 18660
   ctaaagcgca tgctccagac tgccttggga aaagcgcctc ccctacccgg tagaatttcg 18720
   aggtcgaccg gccgcgaatt cttgaagacg aaagggcctc gtgatacgcc tatttttata 18780
   ggttaatgtc atgataataa tggtttctta gacgtcaggt ggcacttttc ggggaaatgt 18840
   gcgcggaacc cctatttgtt tatttttcta aatacattca aatatgtatc cgctcatgag 18900
   acaataaccc tgataaatgc ttcaataata ttgaaaaagg aagagtatga gtattcaaca 18960
   tttccgtgtc gcccttattc ccttttttgc ggcattttgc cttcctgttt ttgctcaccc 19020
   agaaacgctg gtgaaagtaa aagatgctga agatcagttg ggtgcacgag tgggttacat 19080
   cgaactggat ctcaacagcg gtaagatcct tgagagtttt cgccccgaag aacgttttcc 19140
   aatgatgagc acttttaaag ttctgctatg tggcgcggta ttatcccgtg ttgacgccgg 19200
   gcaagagcaa ctcggtcgcc gcatacacta ttctcagaat gacttggttg agtactcacc 19260
   agtcacagaa aagcatctta cggatggcat gacagtaaga gaattatgca gtgctgccat 19320
   aaccatgagt gataacactg cggccaactt acttctgaca acgatcggag gaccgaagga 19380
   gctaaccgct tttttgcaca acatggggga tcatgtaact cgccttgatc gttgggaacc 19440
   ggagctgaat gaagccatac caaacgacga gcgtgacacc acgatgcctg cagcaatggc 19500
   aacaacgttg cgcaaactat taactggcga actacttact ctagcttccc ggcaacaatt 19560
   aatagactgg atggaggcgg ataaagttgc aggaccactt ctgcgctcgg cccttccggc 19620
   tggctggttt attgctgata aatctggagc cggtgagcgt gggtctcgcg gtatcattgc 19680
   agcactgggg ccagatggta agccctcccg tatcgtagtt atctacacga cggggagtca 19740
   ggcaactatg gatgaacgaa atagacagat cgctgagata ggtgcctcac tgattaagca 19800
   ttggtaactg tcagaccaag tttactcata tatactttag attgatttaa aacttcattt 19860
   ttaatttaaa aggatctagg tgaagatcct ttttgataat ctcatgacca aaatccctta 19920
   acgtgagttt tcgttccact gagcgtcaga ccccgtagaa aagatcaaag gatcttcttg 19980
   agatcctttt tttctgcgcg taatctgctg cttgcaaaca aaaaaaccac cgctaccagc 20040
   ggtggtttgt ttgccggatc aagagctacc aactcttttt ccgaaggtaa ctggcttcag 20100
   cagagcgcag ataccaaata ctgtccttct agtgtagccg tagttaggcc accacttcaa 20160
   gaactctgta gcaccgccta catacctcgc tctgctaatc ctgttaccag tggctgctgc 20220
   cagtggcgat aagtcgtgtc ttaccgggtt ggactcaaga cgatagttac cggataaggc 20280
   gcagcggtcg ggctgaacgg ggggttcgtg cacacagccc agcttggagc gaacgaccta 20340
   caccgaactg agatacctac agcgtgagct atgagaaagc gccacgcttc ccgaagggag 20400
   aaaggcggac aggtatccgg taagcggcag ggtcggaaca ggagagcgca cgagggagct 20460
   tccaggggga aacgcctggt atctttatag tcctgtcggg tttcgccacc tctgacttga 20520
   gcgtcgattt ttgtgatgct cgtcaggggg gcggagccta tggaaaaacg ccagcaacgc 20580
   ggccttttta cggttcctgg ccttttgctg gccttttgct ggccttttgc tcacatgttc 20640
   tttcctgcgt tatcccctga ttctgtggat aaccgtatta ccgcctttga gtgagctgat 20700
   accgctcgcc gcagccgaac gaccgagcgc agcgagtcag tgagcgagga agcggaagag 20760
   cgctgacttc cgcgtttcca gactttacga aacacggaaa ccgaagacca ttcatgttgt 20820
   tgctcaggtc gcagacgttt tgcagcagca gtcgcttcac gttcgctcgc gtatcggtga 20880
   ttcattctgc taaccagtaa ggcaaccccg ccagcctagc cgggtcctca acgacaggag 20940
   cacgatcatg cgcacccgtc agatccagac atgataagat acattgatga gtttggacaa 21000
   accacaacta gaatgcagtg aaaaaaatgc tttatttgtg aaatttgtga tgctattgct 21060
   ttatttgtaa ccattataag ctgcaataaa caagttaaca acaacaattg cattcatttt 21120
   atgtttcagg ttcaggggga ggtgtgggag gttttttaaa gcaagtaaaa cctctacaaa 21180
   tgtggtatgg ctgattatga tctctagtca aggcactata catcaaatat tccttattaa 21240
   cccctttaca aattaaaaag ctaaaggtac acaatttttg agcatagtta ttaatagcag 21300
   acactctatg cctgtgtgga gtaagaaaaa acagtatgtt atgattataa ctgttatgcc 21360
   tacttataaa ggttacagaa tatttttcca taattttctt gtatagcagt gcagcttttt 21420
   cctttgtggt gtaaatagca aagcaagcaa gagttctatt actaaacaca gcatgactca 21480
   aaaaacttag caattctgaa ggaaagtcct tggggtcttc tacctttctc ttcttttttg 21540
   gaggagtaga atgttgagag tcagcagtag cctcatcatc actagatggc atttcttctg 21600
   agcaaaacag gttttcctca ttaaaggcat tccaccactg ctcccattca tcagttccat 21660
   aggttggaat ctaaaataca caaacaatta gaatcagtag tttaacacat tatacactta 21720
   aaaattttat atttacctta gagctttaaa tctctgtagg tagtttgtcc aattatgtca 21780
   caccacagaa gtaaggttcc ttcacaaaga tccggaccaa agcggccatc gtgcctcccc 21840
   actcctgcag ttcgggggca tggatgcgcg gatagccgct gctggtttcc tggatgccga 21900
   cggatttgca ctgccggtag aactccgcga ggtcgtccag cctcaggcag cagctgaacc 21960
   aactcgcgag gggatcgagc ccggggtggg cgaagaactc cagcatgaga tccccgcgct 22020
   ggaggatcat ccagccggcg tcccggaaaa cgattccgaa gcccaacctt tcatagaagg 22080
   cggcggtgga atcgaaatct cgtgatggca ggttgggcgt cgcttggtcg gtcatttcga 22140
   accccagagt cccgctcaga agaactcgtc aagaaggcga tagaaggcga tgcgctgcga 22200
   atcgggagcg gcgataccgt aaagcacgag gaagcggtca gcccattcgc cgccaagctc 22260
   ttcagcaata tcacgggtag ccaacgctat gtcctgatag cggtccgcca cacccagccg 22320
   gccacagtcg atgaatccag aaaagcggcc attttccacc atgatattcg gcaagcaggc 22380
   atcgccatgg gtcacgacga gatcctcgcc gtcgggcatg cgcgccttga gcctggcgaa 22440
   cagttcggct ggcgcgagcc cctgatgctc ttcgtccaga tcatcctgat cgacaagacc 22500
   ggcttccatc cgagtacgtg ctcgctcgat gcgatgtttc gcttggtggt cgaatgggca 22560
   ggtagccgga tcaagcgtat gcagccgccg cattgcatca gccatgatgg atactttctc 22620
   ggcaggagca aggtgagatg acaggagatc ctgccccggc acttcgccca atagcagcca 22680
   gtcccttccc gcttcagtga caacgtcgag cacagctgcg caaggaacgc ccgtcgtggc 22740
   cagccacgat agccgcgctg cctcgtcctg cagttcattc agggcaccgg acaggtcggt 22800
   cttgacaaaa agaaccgggc gcccctgcgc tgacagccgg aacacggcgg catcagagca 22860
   gccgattgtc tgttgtgccc agtcatagcc gaatagcctc tccacccaag cggccggaga 22920
   acctgcgtgc aatccatctt gttcaatcat gcgaaacgat cctcatcctg tctcttgatc 22980
   agatcttgat cccctgcgcc atcagatcct tggcggcaag aaagccatcc agtttacttt 23040
   gcagggcttc ccaaccttac cagagggcgc cccagctggc aattccggtt cgcttgctgt 23100
   ccataaaacc gcccagtcta gctatcgcca tgtaagccca ctgcaagcta cctgctttct 23160
   ctttgcgctt gcgttttccc ttgtccagat agcccagtag ctgacattca tccggggtca 23220
   gcaccgtttc tgcggactgg ctttctacgt gttccgcttc ctttagcagc ccttgcgccc 23280
   tgagtgcttg cggcagcgtg aagctttttg caaaagccta ggcctccaaa aaagcctcct 23340
   cactacttct ggaatagctc agaggccgag gcggcctcgg cctctgcata aataaaaaaa 23400
   attagtcagc catggggcgg agaatgggcg gaactgggcg gagttagggg cgggatgggc 23460
   ggagttaggg gcgggactat ggttgctgac taattgagat gcatgctttg catacttctg 23520
   cctgctgggg agcctgggga ctttccacac ctggttgctg actaattgag atgcatgctt 23580
   tgcatacttc tgcctgctgg ggagcctggg gactttccac accctaactg acacacattc 23640
   cacagccgga tctgcaggac ccaacgctgc ccgagatgcg ccgcgtgcgg ctgctggaga 23700
   tggcggacgc gatggatatg ttctgccaag ggttggtttg cgcattcaca gttctccgca 23760
   agaattgatt ggctccaatt cttggagtgg tgaatccgtt agcgaggtgc cgccggcttc 23820
   cattcaggtc gaggtggccc ggctccatgc accgcgacgc aacgcgggga ggcagacaag 23880
   gtatagggcg gcgcctacaa tccatgccaa cccgttccat gtgctcgccg aggcggcata 23940
   aatcgccgtg acgatcagcg gtccaatgat cgaagttagg ctggtaagag ccgcgagcga 24000
   tccttgaagc tgtccctgat ggtcgtcatc tacctgcctg gacagcatgg cctgcaacgc 24060
   gggcatcccg atgccgccgg aagcgagaag aatcataatg gggaaggcca tccagcctcg 24120
   cgtcgcgaac gccagcaaga cgtagcccag cgcgtcggcc gccatgccgg cgataatggc 24180
   ctgcttctcg ccgaaacgtt tggtggcggg accagtgacg aaggcttgag cgagggcgtg 24240
   caagattccg aataccgcaa gcgacaggcc gatcatcgtc gcgctccagc gaaagcggtc 24300
   ctcgccgaaa atgacccaga gcgctgccgg cacctgtcct acgagttgca tgataaagaa 24360
   gacagtcata agtgcggcga cgatagtcat gccccgcgcc caccggaagg agctgactgg 24420
   gttgaaggct ctcaagggca tcggtcgagg aactttcggc ggctttgctg tgcgacaggc 24480
   tcacgtctaa aaggaaataa atcatgggtc ataaaaatta tcacgttgtc ggcgcggcga 24540
   cggatgttct gtatgcgctg ttttccgttg gccgttgctg tctggtgatc tgccttctaa 24600
   atctgcacag ccgaattgcg cgagcttggt tttgctgaaa ccgacacaca gcaactgaat 24660
   accagaaaga aaatcacttt gcctttctga catcagaagg gcagaaattt gccgttgaac 24720
   acctggtcaa tacgcgtttt ggtgagcagc aatattgcgc ttcgatgagc cttggcgttg 24780
   agattgatac ctctgctgca caaaaggcaa tcgaccgagc tggaccagcg cattcgtgac 24840
   accgtctcct tcgaacttat tcgcaatgga gtgtcattca tcaaggacng cctgatcgca 24900
   aatggtgcta tccacgcagc ggcaatcgaa aaccctcagc cggtgaccaa tatctacaac 24960
   atcagccttg gtatcctgcg tgatgagcca gcgcagaaca aggtaaccgt cagtgccgat 25020
   aagttcaaag ttaaacctgg tgttgatacc aacattgaaa cgttgatcga aaacgcgctg 25080
   aaaaacgctg ctgaatgtgc ggcgctggat gtcacaaagc aaatggcagc agacaagaaa 25140
   gcgatggatg aactggcttc ctatgtccgc acggccatca tgatggaatg tttccccggt    25200
   ggtgttatct ggcagcagtg ccgtcgatag tatgcaattg ataattatta tcatttgcgg    25260
   gtcctttccg gcgatccgcc ttgttacggg gcggcgacct cgcgggtttt cgctatttat    25320
   gaaaattttc cggtttaagg cgtttccgtt cttcttcgtc ataacttaat gtttttattt    25380
   aaaataccct ctgaaaagaa aggaaacgac aggtgctgaa agcgagcttt ttggcctctg    25440
   tcgtttcctt tctctgtttt tgtccgtgga atgaacaatg gaagtcaaca aaaagcagac    25500
   gtatctagac acgtctgaag ctagcttcga ggaactttcg gcggctttgc tgtgcgacag    25560
   gctcacgtct aaaaggaaat aaatcatggg tcataaaaat tatcacgttg tcggcgcggc    25620
   gacggatgtt ctgtatgcgc tgttttccgt tggccgttgc tgtctggtga tctgccttct    25680
   aaatctgcac agccgaattg cgcgagcttg gttttgctga aaccgacaca cagcaactga    25740
   ataccagaaa gaaaatcact ttgcctttct gacatcagaa gggcagaaat ttgccgttga    25800
   acacctggtc aatacgcgtt ttggtgagca gcaatattgc gcttcgatga gccttggcgt    25860
   tgagattgat acctctgctg cacaaaaggc aatcgaccga gctggaccag cgcattcgtg    25920
   acaccgtctc cttcgaactt attcgcaatg gagtgtcatt catcaaggac ngcctgatcg    25980
   caaatggtgc tatccacgca gcggcaatcg aaaaccctca gccggtgacc aatatctaca    26040
   acatcagcct tggtatcctg cgtgatgagc cagcgcagaa caaggtaacc gtcagtgccg    26100
   ataagttcaa agttaaacct ggtgttgata ccaacattga aacgttgatc gaaaacgcgc    26160
   tgaaaaacgc tgctgaatgt gcggcgctgg atgtcacaaa gcaaatggca gcagacaaga    26220
   aagcgatgga tgaactggct tcctatgtcc gcacggccat catgatggaa tgtttccccg    26280
   gtggtgttat ctggcagcag tgccgtcgat agtatgcaat tgataattat tatcatttgc    26340
   gggtcctttc cggcgatccg ccttgttacg gggcggcgac ctcgcgggtt ttcgctattt    26400
   atgaaaattt tccggtttaa ggcgtttccg ttcttcttcg tcataactta atgtttttat    26460
   ttaaaatacc ctctgaaaag aaaggaaacg acaggtgctg aaagcgagct ttttggcctc    26520
   tgtcgtttcc tttctctgtt tttgtccgtg gaatgaacaa tggaagtcaa caaaaagcag    26580
   agcttatcga tgataagcgg tcaaacatga gaattcgtcg g                        26621